# EUROPEAN PATENT SPECIFICATION

(11) **EP 3 186 247 B1**
(45) Date of publication and mention of the grant of the patent: **03.03.2021**
(21) Application number: 15834916.7
(22) Date of filing: 30.07.2015
(51) Int. Cl.: A61P 35/00, C07D 403/12, C07D 403/10, C07D 237/20, C07D 239/42, C07D 498/04, A61P 11/06, A61P 19/02, A61P 29/00, A61P 35/02, A61P 43/00

(54) **PROTEIN KINASE INHIBITORS**
PROTEINKINASEHEMMER
INHIBITEURS DE PROTÉINE KINASE

(30) Priority: 26.08.2014 US 201414469156
(43) Date of publication of application: 05.07.2017
(73) Proprietor: Astar Biotech LLC, Richmond, VA 23219 (US)
(72) Inventor: YU, Chunrong, Richmond, Virginia 23219 (US); HUANG, Haihong, Beijing 100050 (CN); ZHANG, Dongfeng, Beijing 100050 (CN); LI, Peng, Beijing 100050 (CN)
(74) Representative: Grünecker Patent- und Rechtsanwälte PartG mbB
(86) International application number: PCT/CN2015/085600
(87) International publication number: WO 2016/029776

(56) References cited:
- WO-A1-01/46124
- WO-A1-2012/089106
- WO-A1-2013/184681
- WO-A1-2014/019338
- CN-A- 102 775 411

## Description

### FIELD OF THE INVENTION

The present invention describes specific alkynyl derivatives with absolute configuration S' with inhibitory activity on protein kinases and a pharmaceutical composition for preventing or treating diseases involving abnormal cell growth.

### BACKGROUND OF THE INVENTION

Protein kinases are a group of enzymes for phosphorylation of hydroxyl groups in serine, threonine, and tyrosine moieties of protein. They are essential in growth factor signal transduction inducing cell growth, differentiation, and proliferation. Human protein kinases can be divided into tyrosine protein kinases and serine/threonine protein kinases. Tyrosine protein kinases can be further divided into receptor and cytoplasma/non-receptor kinases (Manning et al., Science, 2002, 298, 1912). Receptor tyrosine kinases possess cell surface domains to interact with growth factors, and cytoplasma domains to conduct phosphorylation of tyrosine moieties. As such, a growth factor can bind to the growth factors receptor site, thereby triggering polymerization of the receptor tyrosine kinase, and autophosphorylation of the tyrosine moieties in cytoplasma. Subsequently, sequential phosphorylation of subfamily proteins proceeds as signal transduction progresses, leading to overexpression of transcription factors and eventually cancer. A mutation or an overexpression of certain protein kinases may impact the signal transduction in a normal cell resulting in the imbalance of homeostasis in body. For example, continuous signal transduction may lead to cancer, inflammation, metabolic syndromes, and CNS diseases.

The *bcr*/*abl* fusion gene, formed by rearrangement of the breakpoint cluster region (bcr) on chromosome 22 with the *c-abl* proto-oncogene on chromosome 9, is present in CML (chronic myeloid leukemia) patients. The chromosome containing *bcr*/*abl* gene is referred to as Philadelphia chromosome (Nowell, J. Natl. Cancer Inst., 1960;25:85). In the *bcr*/*abl* fusion gene, the *bcr* gene part contains oligomerization domains, and the *abl* gene part contains tyrosine domains. 3 principle forms (p190, p210 and p230 kDa) of *bcr*/*abl* gene, determined by the breakpoints of the *bcr* gene, have been reported. Gleevec (imatinib mesylate, STI-571) is the first targeted anti-cancer agent which was developed by Norvartis and released in 2002. Gleevec can selectively inhibit *bcr*/*abl* by inhibiting tyrosine kinases of *abl.* Gleevec is commonly used as standard treatment for CML. But acquired resistance to Gleevec became a serious problem. There are various mechanisms to cause the acquired Gleevec-resistance including amplification of *bcr*/*abl* gene, loss of *bcr*/*abl* gene, point mutations of *bcr*/*abl* gene. Among them, the most important factor inducing the acquired Gleevec-resistance is T315I*-bcr*/*abl* point mutation within the *abl* kinase domain. Multiple creative endeavors to overcome the acquired Gleevec-resistance were engaged. The recently released drugs such as Nilotinib and Dasatinib effectively inhibit many point-mutations of *abl* kinase domains generated by the acquired Gleevec-resistance. However, Nilotinib and Dasatinib are unable to inhibit *T3151-bcr*/*abl* mutant species. Therefore, there are a number of attempts to develop a medicine inhibiting *T315I-bcr*/*abl* mutation. Vascular endothelial growth factors (VEGFs) mediate a plethora of biological process in endothelial cells such as cell survival, proliferation, differentiation, angiogenesis, and migration. VEGFs are primarily produced by vascular endothelial, hematopoietic and stromal cells in response to hypoxia and upon stimulation of growth factors such as TGFs, PDGFs or interleukins. VEGFs bind to their high-affinity specific receptors VEGFRs (VEGFR-1, -2, and -3). Each VEGF isoform binds to a particular subset of these receptors leading to the formation of receptor homo- and heterodimers that activates discrete signaling pathways and executes its biological functions such as angiogenesis [Cébe-Suarez S, Zehnder-Fjällman A, Ballmer-Hofer K. Cell Mol Life Sci. 2006 Mar; 63(5): 601-15]. Angiogenesis provides tumors with nutrients, oxygen, and path for cancer cell spread. Therefore, it is essential for cancer cell proliferation and spread. Angiogenesis in normal body is balanced by co-regulation between angiogenic stimulators and angiogenic suppressors. However, in off-balanced cancer cells, VEGFR is activated by growth factors such as VEGF which have a great effect on vascular endothelial cells [Ann Hoeben, Bart Landuyt, Martin S. Highley, Hans Wildiers, Allan T. Van Oosterom and Ernst A. De Bruijn. Pharmacological Reviews. 2004 vol. 56(4): 549-580]. Various inhibitors (small synthetic molecules) of VEGF receptor tyrosine kinases are being developed, most of which are able to be used in solid tumors and to inhibit angiogenesis activated only in cancer cells and have a tremendous medicinal action with fairly low side effects. WO 2014/019338 relates to ptotein kinase inhibitors of similar structure having configuration R.

### SUMMARY OF THE INVENTION

The present invention relates to the following embodiments defined under items 1-8, below:
1. A compound with absolute configuration S, or a pharmaceutically acceptable salt, hydrate, or solvate thereof, wherein the compound is selected from the group consisting of:
   (S)-N-(1,1-difluoro-2,3 -dihydro-3-(4-methylpiperazin-1-yl)-1H-indan-6-yl)-3-(2-(imidazo[1,2-b]pyridazin-3- yl)ethynyl)-4-methylbenzamide;
   (S)-3-(2-(2-(Cyclopropylamino)pyrimidin-5-yl)ethynyl)-N-(1,1-difluoro-2,3-dihydro-3-(4-methylpiperazin-1-yl)-1H-indan-6-yl)-4-methylbenzamide;
   (S)-N-(1,1-difluoro-2,3-dihydro-3-(4-methylpiperazin-1-yl)-1H-indan-6-yl)-3-(2-(imidazo[1,2-b]pyridazin-3-yl)ethynyl)-4-chlorobenzamide; and
   (S)-N-(1,1-difluoro-2,3-dihydro-3-(4-methylpiperazin-1-yl)-1H-indan-6-yl)-3-(2-(imidazo[1,2-b]pyridazin-3 - yl)ethynyl)-4-fluorobenzamide.
2. A pharmaceutical composition comprising at least one compound of item 1 or a salt, hydrate, or solvate thereof, and one or more pharmaceutically acceptable carriers and/or additives.
3. A compound of item 1, or a salt, hydrate, or solvate thereof for use in the treatment of cancer or inflammation, wherein protein kinase is inhibited.
4. The compound for use according to item 3, wherein said protein kinase is selected from the group consisting of Bcr-Abl, c-Kit, c-Src, FGFR1, FLT3, LYN and PDGFR.
5. A compound of item 1, or a salt, hydrate, or solvate thereof for use in the treatment of cancer.
6. The compound for use according to item 5, wherein said cancer is selected from Chronic Myelogenous Leukemia (CML), Acute Lymphocytic Leukemia (ALL), Non-Small Cell Lung Cancer (NSCLC), Gastrointestinal stromal tumor (GIST) and Acute Myelogenous Leukemia (AML).
7. A compound of item 1, or a salt, hydrate, or solvate thereof for use in the treatment of inflammation.
8. The compound for use according to item 7, wherein said inflammation is selected from Asthma and Rheumatoid Arthritis.

Disclosed herein are compounds which have protein kinase inhibitory activity, which are valuable pharmaceutically active compounds for the therapy to treat abnormal cell growth diseases, for example tumors in cancer patients.

Disclosed herein are compounds of Formula I, or a pharmaceutically acceptable salt, isomer, hydrate, and solvate thereof: 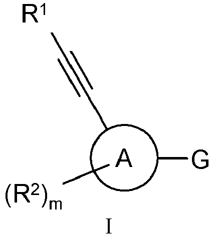 Wherein,
ring A is a 4- to 8-membered heterocyclyl ring containing 1-2 heteroatoms selected from O, N and S, a 5- to 10-membered heteroaryl ring containing 1-3 heteroatoms selected from O, N and S, or a 6- to 12-membered aryl ring, wherein the heterocyclyl ring, heteroaryl ring and aryl ring are optionally and independently substituted with (R²)ₘ;
R² is independently H, halogen, CN, CH₂CN, NO₂, CF₃, OCF₃, NH₂, C₁-C₆ alkyl, C₃-C₆ cycloalkyl, OR, SR, NR^{a}S(O)₂R^{b}, (CR₂),-S(O)ₚR^{c}, C(O)OR^{c}, NR^{a}C(O)R^{d}, NR^{a}C(O)OR^{d}, NR^{e}R^{f}, or (CR⁹₂)ₜ-S(O)₂R^{c};
R¹ is aryl, heteroaryl, heterocyclyl, alkyl, or cycloalkyl, wherein said aryl, heteroaryl, heterocyclyl, alkyl and cycloalkyl are optionally and independently substituted with: halogen, CN, CH₂CN, NO₂, CF₃, OCF₃, NH₂, C₁-C₆ alkyl, C₃-C₆ cycloalkyl, OR, SR, NR^{a}S(O)₂R^{b}, (CR₂)ᵥ-S(O)ₚR^{c}, C(O)OR^{c}, NR^{a}C(O)R^{d}, NR^{a}C(O)OR^{d}, NR^{e}R^{f}, (CR^{g}₂)ₜ-S(O)₂R^{c}, aryl, heterocyclyl, or heteroaryl;
each R^{a} is independently H, C₁-C₆ alkyl, C₃-C₁₀ cycloalkyl, heteroaryl or aryl, wherein said alkyl, cycloalkyl, heteroaryl and aryl are optionally and independently substituted with aryl, heteroaryl or heterocyclyl;
each R^{b} is independently C₁-C₆ alkyl, C₃-C₁₀ cycloalkyl or aryl, wherein said alkyl, cycloalkyl and aryl are optionally and independently substituted with 1 to 3 groups selected from C₃-C₁₀ cycloalkyl and halo substituents;
each R^{c} is independently H, C₁-C₆ alkyl, C₃-C₁₀ cycloalkyl, heteroaryl or aryl, wherein said alkyl, cycloalkyl, heteroaryl and aryl are optionally and independently substituted with aryl, heteroaryl or heterocyclyl;
each R^{d} is independently H, C₁-C₆ alkyl or aryl, wherein said alkyl and aryl are optionally substituted with 1 to 3 halo substituents;
each of R^{e} and R^{f} is independently C₁-C₆ alkyl, or a 4- to 8-membered heterocyclyl containing 1 to 2 heteroatoms each independently selected from O, S, or N, wherein said heterocyclyl and alkyl are optionally substituted with 1 to 4 groups selected from halo and alkyl groups;
each R^{g} is independently H, F, C₁-C₆ alkyl, aryl or CF₃, wherein said alkyl and aryl are optionally and independently substituted with 1 to 3 groups selected from aryl, CF₃ and halo substituents;
each R is independently H, C₁-C₆ alkyl or C₃-C₁₀ cycloalkyl, wherein said alkyl and cycloalkyl are optionally and independently substituted with 1 to 4 halo substituents;
m is 0, 1, 2, or 3;
v is 0, 1, 2, or 3;
p is 0, 1, or 2;
t is 0, 1, 2, or 3; and
G is selected from: 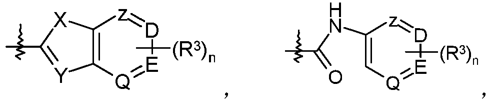 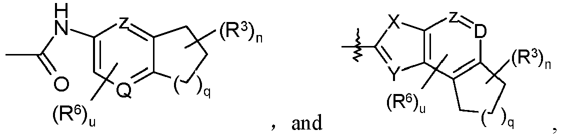
wherein,
R³ is independently H, halogen, C₁-C₆ alkyl, C₃-C₆ cycloalkyl, (CH₂)ₖ-(5- or 6-membered) heteroaryl, or (CH₂)ₖ-(5- or 6-membered) heterocyclyl, wherein said heteroaryl, heterocyclyl, alkyl, and cycloalkyl are independently and optionally substituted with: halogen, CN, CH₂CN, CF₃, OCF₃, NH₂, C₁-C₆ alkyl, C₃-C₆ cycloalkyl, OR, SR, NR^{a}S(O)₂R,^{b} (CR₂)ᵥ-S(O)ₚR^{c}, C(O)OR^{c}, NR^{e}R^{f}, (CR^{g}₂)ₜS(O)₂R^{c}, NR^{a}C(O)R^{d}, NR^{a}C(O)OR^{d}, aryl, heterocyclyl, or heteroaryl;
R⁶ is independently halogen, CN, CH₂CN, NO₂, CF₃, OCF₃, NH₂, C₁-C₆ alkyl, C₃-C₆ cycloalkyl, OR, SR, NR^{a}S(O)₂R^{b}, (CR₂),-S(O)ₚR^{c}, C(O)OR^{c}, NR^{a}C(O)R^{d}, NR^{a}C(O)OR^{d}, NR^{e}R^{f}, or (CR^{g}₂)ₜ-S(O)₂R^{c};
X is NH or O;
Y is CH or N;
Z, D, E and Q are independnetly CH or N;
k is 0, 1, 2, or 3;
n is 1, 2, or 3;
q is 1, or 2; and
u is 0, 1, 2, or 3.

Further disclosed herein are pharmaceutical compositions comprising at least one compound of Formula I, or a salt, hydrate, isomer, or solvate thereof, and one or more pharmaceutically acceptable carriers and/or additives.

Further disclosed herein is a therapeutically effective amount of a compound of Formula I, or a salt, hydrate, isomer, or solvate thereof for use in inhibiting protein kinase.

Further disclosed herein is a therapeutically effective amount of a compound of Formula I, or a salt, hydrate, isomer, or solvate thereof for use in treating cancers in a human patient.

Further disclosed herein is a therapeutically effective amount of a compound of Formula I, or a salt, hydrate, isomer, or solvate thereof for use in treating inflammation in a human patient.

Further disclosed herein is the use of a compound of Formula I, or a salt, hydrate, isomer, or solvate thereof in the manufacture of a medicament for treating cancers or inflammation.

Disclosed herein is a compound of Formula VIII having an absolute configuration S, or a pharmaceutically acceptable salt, hydrate, and solvate thereof: 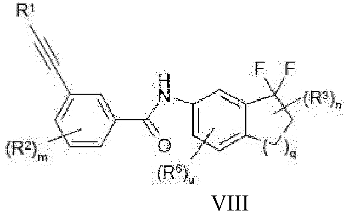 wherein,
R¹ is selected from: 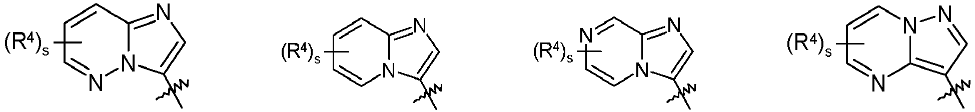 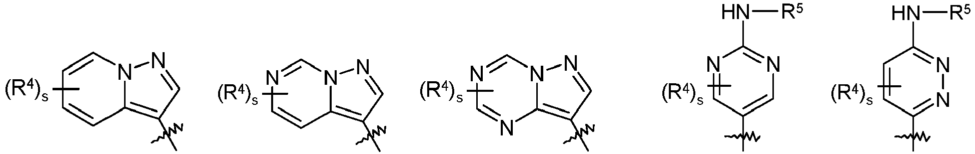 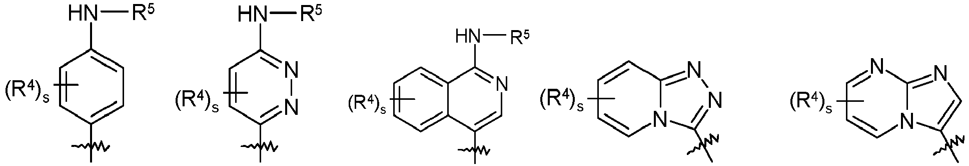 and 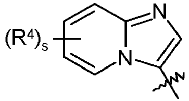
R² is independently H, halogen, CN, NO₂, CF₃, OCF₃, NH₂, NR^{e}R^{f}, C₁-C₆ alkyl, C₃-C₆ cycloalkyl, OCH₃, COOCH₃, or NHCOCH₂CH₃;
R³ is 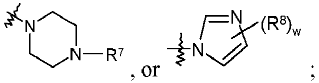
R⁴ is independently H, halogen, CN, NO₂, CF₃, OCF₃, NH₂, NR^{e}R^{f}, C₁-C₆ alkyl, C₃-C₆ cycloalkyl, OCH₃, COOCH₃, or NHCOCH₂CH₃;
R⁵ is independently H, C₁-C₆ alkyl, C₃-C₆ cycloalkyl, phenyl, or benzyl;
R⁶ is independently H, halogen, CN, NO₂, CF₃, OCF₃, NH₂, NR^{e}R^{f}, C₁-C₆ alkyl, C₃-C₆ cycloalkyl, OCH₃, COOCH₃, or NHCOCH₂CH₃;
R⁷ is independently H, C₁-C₆ alkyl, or C₃-C₆ cycloalkyl;
R⁸ is independently H, halogen, CN, NO₂, CF₃, NH₂, NR^{e}R^{f}, C₁-C₆ alkyl, or C₃-C₆ cycloalkyl;
R^{e} and R^{f} is independently C₁-C₆ alkyl;
m is 0, 1, or 2; n is 1, or 2; w is 0, 1, or 2; s is 0, 1, or 2; u is 0, 1, or 2; and q is 1 or 2.

In a further aspect, the compound having an absolute configuration S is represented by Formula VIIIa, VIIIb or VIIIc:

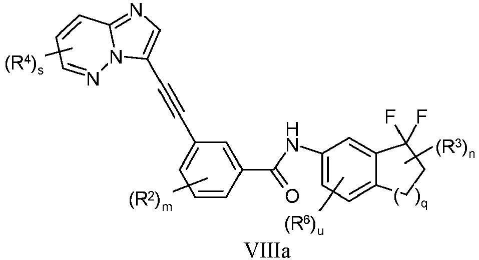

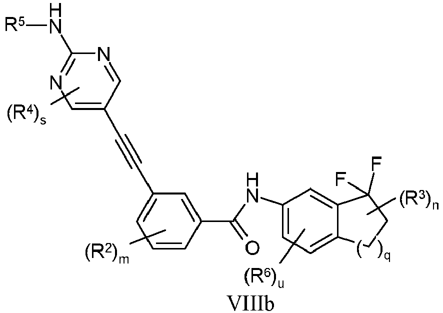

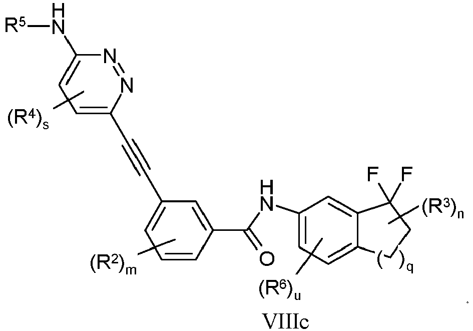

In still a further aspect, according to formula VIIIa, formula VIIIb or formular VIIIc, in which R² is H, CH₃ , F or Cl; R³ is 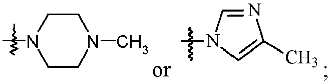 R⁵ is cyclopropyl, cyclobutyl, or isopropyl; R⁶ is H or CF₃; and s is 0, u is 0, m is 0 or 1, n is 1 and q is 1.

Particularly, the compound of formula VIII having an absolute configuration S is:
(S)-N-(1,1-difluoro-2,3-dihydro-3-(4-methylpiperazin-1-yl)-1H-indan-6-yl)-3-(2-(imidazo[1,2-b]pyridazin-3- yl)ethynyl)-4-methylbenzamide;
(S)-3-(2-(2-(Cyclopropylamino)pyrimidin-5-yl)ethynyl)-N-(1,1-difluoro-2,3-dihydro-3-(4-methylpiperazin-1-yl)-1H-indan-6-yl)-4-methylbenzamide;
   or
(S)-N-(1,1-difluoro-2,3-dihydro-3-(4-methylpiperazin-1-yl)-1H-indan-6-yl)-3-(2-(imidazo[1,2-b]pyridazin-3- yl)ethynyl)-4-chlorobenzamide.
(S)-N-(1,1-difluoro-2,3-dihydro-3-(4-methylpiperazin-1-yl)-1H-indan-6-yl)-3-(2-(imidazo[1,2-b]pyridazin-3- yl)ethynyl)-4-fluorobenzamide.

In a further aspect, the present disclosure provides pharmaceutical compositions comprising at least one compound of Formula VIII with absolute configuration S, or a salt, hydrate, or solvate thereof, and one or more pharmaceutically acceptable carriers and/or additives.

In a further aspect, the present disclosure provides a therapeutically effective amount of a compound of Formula VIII with absolute configuration S, or a salt, hydrate, or solvate thereof for use in inhibiting protein kinase.

The protein kinase may be selected from a group consisting of Bcr-Abl, c-Kit, c-Src, FGFR1, FLT3, LYN and PDGFR; particularly Bcr-Able (T315I).

In still a further aspect, the present disclosure provides a therapeutically effective amount of a compound of Formula VIII with absolute configuration S, or a salt, hydrate, or solvate thereof for use in treating cancers in a human patient.

The cancer may be selected from a group consisting of Chronic Myelogenous Leukemia (CML), Acute Lymphocytic Leukemia (ALL), Non-Small Cell Lung Cancer (NSCLC), Gastrointestinal stromal tumor (GIST) and Acute Myelogenous Leukemia (AML); particularly CML.

In still a further aspect, the present disclosure provides a therapeutically effective amount of a compound of Formula VIII with absolute configuration S, or a salt, hydrate, isomer, or solvate thereof for use in treating inflammation in a human patient.

The inflammation may be selected from a group consisting of Asthma and Rheumatoid Arthritis.

In still a further aspect, the present disclosure provides the use of a compound of Formula VIII with absolute configuration S, or a salt, hydrate or solvate thereof in the manufacture of a medicament for treating cancers or inflammation.

The cancer may be selected from a group consisting of Chronic Myelogenous Leukemia (CML), Acute Lymphocytic Leukemia (ALL), Non-Small Cell Lung Cancer (NSCLC), Gastrointestinal stromal tumor (GIST) and Acute Myelogenous Leukemia (AML). The inflammation may be selected from a group consisting of Asthma and Rheumatoid Arthritis.

### BRIEF DESCRIPTION OF THE DRAWINGS

Fig. 1: shows the spectrogram for separation of the optimal isomers of Compound 36 by SFC method with a chiral column.
Fig. 2: shows the experimental ECD spectrum of Compound HA-6-13-1 and the calculated ECD spectra of the optical isomers ZDF2a and ZDF2b.
Fig. 3: shows the ECD spectra of prepared (*S*)-N-(1,1-difluoro-2,3-dihydro-3-(4-methylpiperazin-1-yl)-1H-indan-6-yl)-3-(2-(imidazo[1,2-b]pyridazin-3-yl)ethynyl)-4-methylbenzamide (i.e. Compound 36-(S)), HAP-71-p1 and HAP-71-p2.
Fig. 4: shows the chiral column result of prepared(*S*)-N-(1,1-difluoro-2,3-dihydro-3-(4-methylpiperazin-1-yl)-1H-indan-6-yl)-3-(2-(imidazo[1,2-b]pyridazin-3-yl)ethynyl)-4-methylbenzamide (i.e. Compound 36-(S)).
Fig. 5. shows the spectrogram for separation of the optimal isomers of Compound 37 by SFC method with a chiral column.
Fig. 6: shows the spectrogram for separation of the optimal isomers of Compound 38 by SFC method with a chiral column.
Fig. 7: shows the spectrogram for separation of the optimal isomers of Compound 39 by SFC method with a chiral column.
Fig. 8: shows the tumor volume (A) and body weight (B) during the treatment of BCR-ABL inhibitors.

### DETAILED DESCRIPTION OF THE INVENTION

The present disclosure provides novel compounds according to Formula I shown and described above. Specifically, the compounds disclosed herein are protein kinase inhibitors. As a result, novel compounds according to Formula I, as well as pharmaceutically acceptable salts, hydrates, isomers or solvates thereof are disclosed. Values and particular values for the variables in Formula I are provided in the following paragraphs.

In an embodiment, R¹ is independently aryl, or heteroaryl ring selected from: 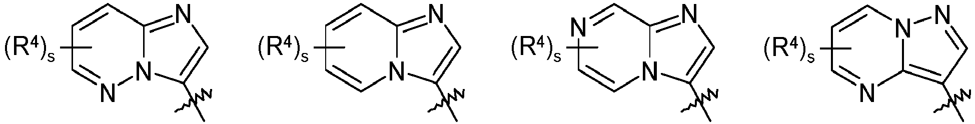 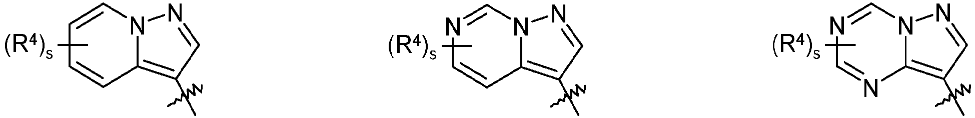 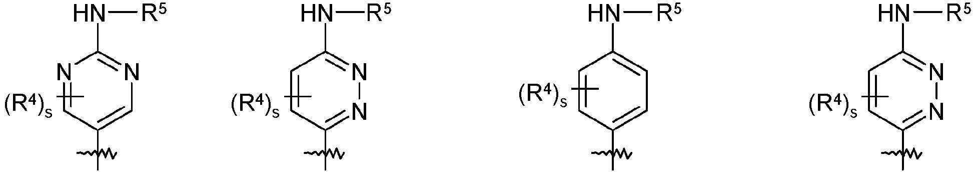 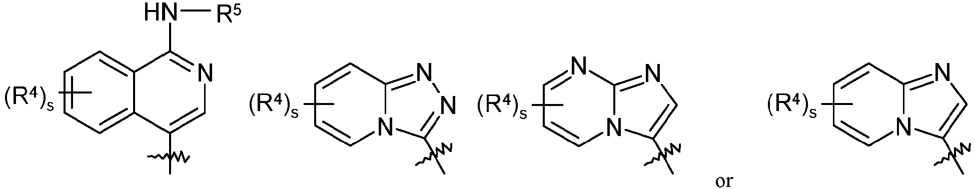 Wherein,
R⁴ is independently H, halogen, CN, CH₂CN, NO₂, CF₃, OCF₃, NH₂, C₁-C₆ alkyl, C₃-C₆ cycloalkyl, OR, SR, NR^{a}S(O)₂R^{b}, (CR₂),-S(O)ₚR^{c}, C(O)OR^{c}, NR^{a}C(O)R^{d}, NR^{a}C(O)OR^{d}, NR^{e}R^{f}, or (CR^{g}₂)ₜ-S(O)₂R^{c};
R⁵ is H, C₁-C₆ alkyl, C₃-C₆ cycloalkyl, phenyl, or benzyl; and
s is 0, 1, or 2.

Disclosed herein are the compounds of Formula I, wherein G is 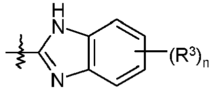 with the following structure of Formula II, and all other variables are as previously defined in Formula I.

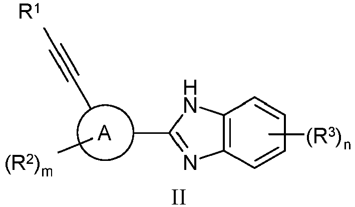

Disclosed herein are the compounds of Formula II, wherein ring A is benzene with the following structure of Formula III: 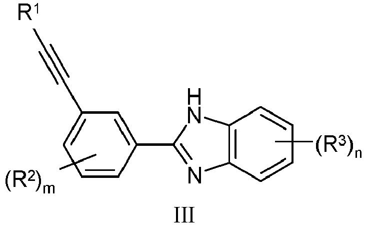 wherein,
R² is independently H, halogen, CN, NO₂, CF₃, OCF₃, NH₂, NR^{e}R^{f}, C₁-C₆ alkyl, C₃-C₆ cycloalkyl, OCH₃, COOCH₃, or NHCOCH₂CH₃;
R³ is 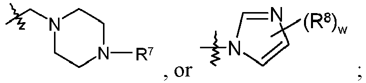
R⁴ is independently H, halogen, CN, NO₂, CF₃, OCF₃, NH₂, NR^{e}R^{f}, C₁-C₆ alkyl, C₃-C₆ cycloalkyl, OCH₃, COOCH₃, or NHCOCH₂CH₃;
R⁵ is independently H, C₁-C₆ alkyl, C₃-C₆ cycloalkyl, phenyl, or benzyl;
R⁷ is independently H, C₁-C₆ alkyl, or C₃-C₆ cycloalkyl;
R⁸ is independently H, halogen, CN, NO₂, CF₃, NH₂, NR^{e}R^{f}, C₁-C₆ alkyl, or C₃-C₆ cycloalkyl;
R^{e} and R^{f} is independently C₁-C₆ alkyl;
m is 0, 1, or 2;
n is 1, or 2;
w is 0, 1, or 2; and
s is 0, 1, or 2,
   and all other variables are as previously defined in Formula II.

Disclosed herein are the compounds of Formula III, wherein the compounds are represented by Formula IIIa, Formula IIIb, or Formula IIIc: 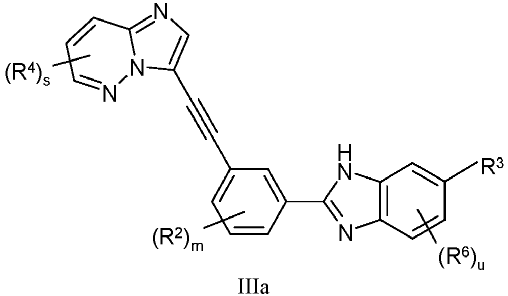 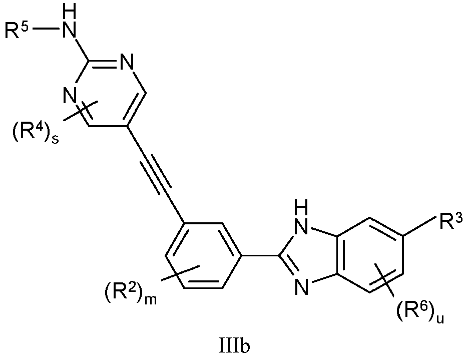 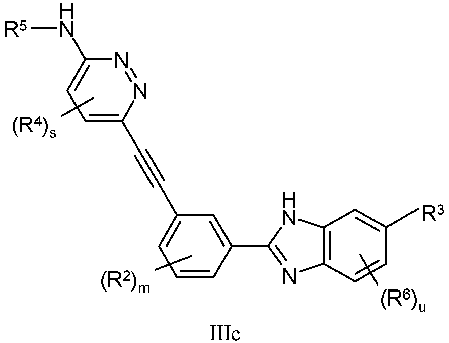 Wherein,
R² is independently H, halogen, CN, NO₂, CF₃, OCF₃, NH₂, NR^{e}R^{f}, C₁-C₆ alkyl, C₃-C₆ cycloalkyl, OCH₃, COOCH₃, or NHCOCH₂CH₃;
R³ is 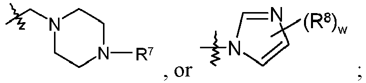
R⁴ is independently H, halogen, CN, NO₂, CF₃, OCF₃, NH₂, NR^{e}R^{f}, C₁-C₆ alkyl, C₃-C₆ cycloalkyl, OCH₃, COOCH₃, or NHCOCH₂CH₃;
R⁵ is independently H, C₁-C₆ alkyl, C₃-C₆ cycloalkyl, phenyl, or benzyl;
R⁶ is independently H, halogen, CN, NO₂, CF₃, OCF₃, NH₂, NR^{e}R^{f}, C₁-C₆ alkyl, C₃-C₆ cycloalkyl, OCH₃, COOCH₃, or NHCOCH₂CH₃;
R⁷ is independently H, C₁-C₆ alkyl, or C₃-C₆ cycloalkyl;
R⁸ is independently H, halogen, CN, NO₂, CF₃, NH₂, NR^{e}R^{f}, C₁-C₆ alkyl, or C₃-C₆ cycloalkyl;
R^{e} and R^{f} is independently C₁-C₆ alkyl;
m is 0, 1, or 2;
w is 0, 1, or 2;
s is 0, 1, or 2; and
u is 0, 1, or 2,
   and all other variables are as previously defined in Formula I.

Disclosed herein are compounds of Formulas IIIa-IIIc, wherein R² is H, CH₃, or Cl; R³ is 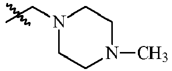 or 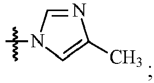 R⁵ is cyclopropyl, cyclobutyl, or isopropyl; and R⁶ is H or CF₃; and u is 0, s is 0, and m is 0 or 1.

Further disclosed herein are compounds of Formula I, wherein G is 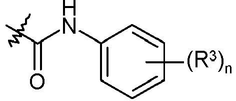 with the following structure of Formula IV, and all other variables are as previously defined in Formula I.

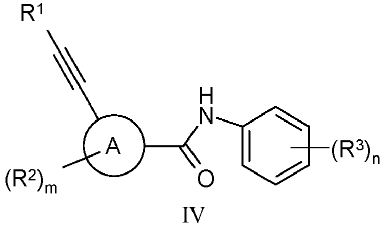

Further disclosed herein are compounds of Formula IV, wherein ring A is benzene with the following structure of Formula V: 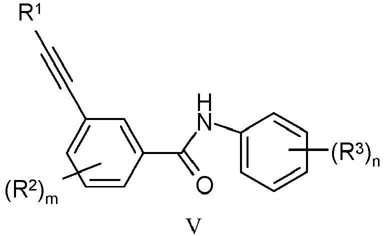 wherein,
R² is independently H, halogen, CN, NO₂, CF₃, OCF₃, NH₂, NR^{e}R^{f}, C₁-C₆ alkyl, C₃-C₆ cycloalkyl, OCH₃, COOCH₃ or NHCOCH₂CH₃;
R³ is 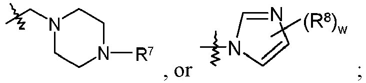
R⁴ is independently H, halogen, CN, NO₂, CF₃, OCF₃, NH₂, NR^{e}R^{f}, C₁-C₆ alkyl, C₃-C₆ cycloalkyl, OCH₃, COOCH₃, or NHCOCH₂CH₃;
R⁵ is independently H, C₁-C₆ alkyl, C₃-C₆ cycloalkyl, phenyl, or benzyl;
R⁷ is independently H, C₁-C₆ alkyl, or C₃-C₆ cycloalkyl;
R⁸ is independently H, halogen, CN, NO₂, CF₃, NH₂, NR^{e}R^{f}, C₁-C₆ alkyl, or C₃-C₆ cycloalkyl;
R^{e} and R^{f} is independently C₁-C₆ alkyl;
m is 0, 1, or 2;
n is 1, or 2;
w is 0, 1, or 2; and
s is 0, 1, or 2,
and all other variables are as previously defined in Formula IV.

Further disclosed herein are compounds of Formula V, wherein the compounds are represented by Formula Va or Formula Vb: 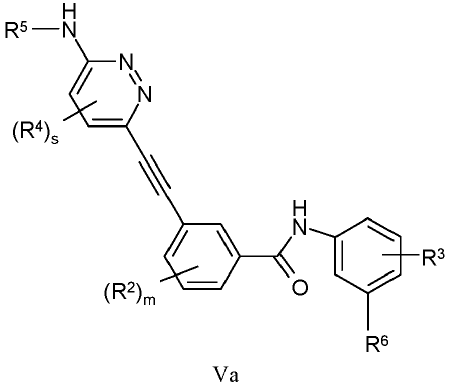 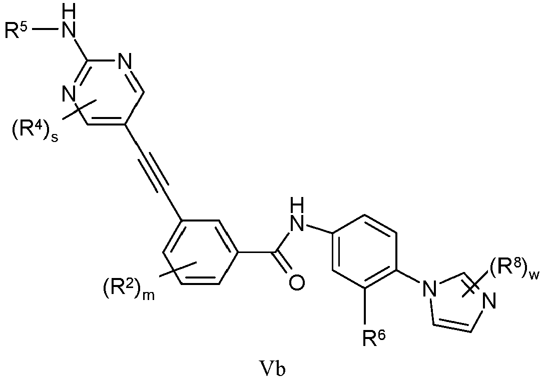 Wherein,
R² is independently H, halogen, CN, NO₂, CF₃, OCF₃, NH₂, NR^{e}R^{f}, C₁-C₆ alkyl, C₃-C₆ cycloalkyl, OCH₃, COOCH₃, or NHCOCH₂CH₃;
R³ is 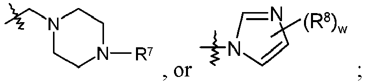
R⁴ is independently H, halogen, CN, NO₂, CF₃, OCF₃, NH₂, NR^{e}R^{f}, C₁-C₆ alkyl, C₃-C₆ cycloalkyl, OCH₃, COOCH₃, or NHCOCH₂CH₃;
R⁵ is independently H, C₁-C₆ alkyl, C₃-C₆ cycloalkyl, phenyl, or benzyl;
R⁶ is independently H, halogen, CN, NO₂, CF₃, OCF₃, NH₂, NR^{e}R^{f}, C₁-C₆ alkyl, C₃-C₆ cycloalkyl, OCH₃, COOCH₃, or NHCOCH₂CH₃;
R⁷ is independently H, C₁-C₆ alkyl, or C₃-C₆ cycloalkyl;
R⁸ is independently H, halogen, CN, NO₂, CF₃, NH₂, NR^{e}R^{f}, C₁-C₆ alkyl, or C₃-C₆ cycloalkyl;
R^{e} and R^{f} is independently C₁-C₆ alkyl;
m is 0, 1, or 2;
w is 0, 1, or 2;
s is 0, 1, or 2,
   and all other variables are as previously defined in Formula I.

Further disclosed herein are compounds of Formulas Va-Vb, wherein R² is H, CH₃, or Cl; R³ is 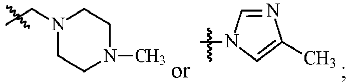 R⁵ is cyclopropyl, cyclobutyl, or isopropyl; R⁶ is H or CF₃; and s is 0, w is 1, and m is 0 or 1.

Further disclosed herein are compounds of Formula IV, wherein ring A is heterocyclyl with the following structure of Formula VI: 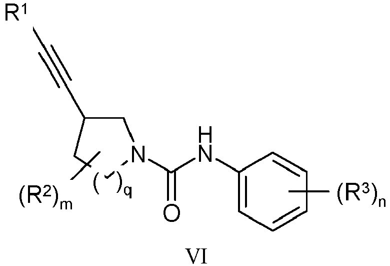 wherein,
R² is independently H, halogen, CN, NO₂, CF₃, OCF₃, NH₂, NR^{e}R^{f}, C₁-C₆ alkyl, C₃-C₆ cycloalkyl, OCH₃, COOCH₃, or NHCOCH₂CH₃;
R³ is 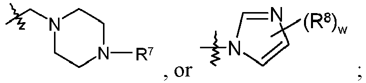
R⁴ is independently H, halogen, CN, NO₂, CF₃, OCF₃, NH₂, NR^{e}R^{f}, C₁-C₆ alkyl, C₃-C₆ cycloalkyl, OCH₃, COOCH₃, or NHCOCH₂CH₃;
R⁵ is independently H, C₁-C₆ alkyl, C₃-C₆ cycloalkyl, phenyl, or benzyl;
R⁷ is independently H, C₁-C₆ alkyl, or C₃-C₆ cycloalkyl;
R⁸ is independently H, halogen, CN, NO₂, CF₃, NH₂, NR^{e}R^{f}, C₁-C₆ alkyl, or C₃-C₆ cycloalkyl;
R^{e} and R^{f} is independently C₁-C₆ alkyl;
m is 0, 1, or 2;
n is 1, or 2;
w is 0, 1, or 2;
s is 0, 1, or 2; and
q is 1 or 2;
and all other variables are as previously defined in Formula IV.

Further disclosed herein are compounds of Formula VI, wherein the compounds are represented by Formula VIa: 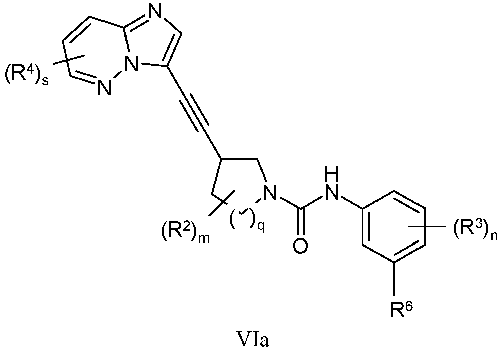 Wherein,
q is 1, or 2; n is 1 or 2; and all other variables are as previously defined in Formula IIIa-IIIc.

In another embodiment of the compounds of Formulas VIa, R² is H; R³ is 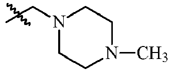 or 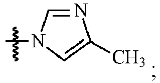 R⁶ is H or CF₃; and s is 0, w is 1, m is 0, and n is 1.

Further disclosed herein are compounds of Formula I, wherein G is 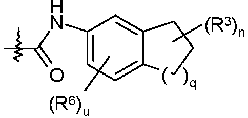 with the following structure of Formula VII: 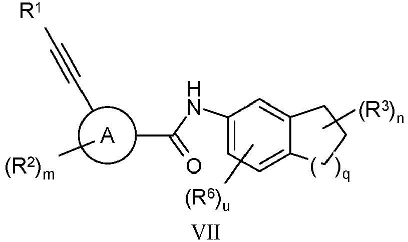 Wherein,
R⁶ is independently halogen, CN, CH₂CN, NO₂, CF₃, OCF₃, NH₂, C₁-C₆ alkyl, C₃-C₆ cycloalkyl, OR, SR, NR^{a}S(O)₂R^{b}, (CR₂)ᵥ-S(O)ₚR^{c}, C(O)OR^{c}, NR^{a}C(O)R^{d}, NR^{a}C(O)OR^{d}, NR^{e}R^{f}, or (CR^{g}₂)ₜ-S(O)₂R^{c};
u is 0, 1, 2, or 3;
q is 1 or 2;
   and all other variables are as previously defined in Formula I.

In another embodiment of the compounds of Formula VII, ring A is benzene with the following structure of Formula VIII: 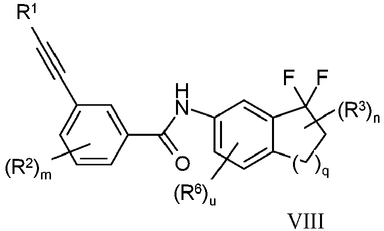 wherein,
R² is independently H, halogen, CN, NO₂, CF₃, OCF₃, NH₂, NR^{e}R^{f}, C₁-C₆ alkyl, C₃-C₆ cycloalkyl, OCH₃, COOCH₃, or NHCOCH₂CH₃;
R³ is 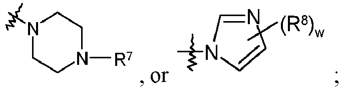
R⁴ is independently H, halogen, CN, NO₂, CF₃, OCF₃, NH₂, NR^{e}R^{f}, C₁-C₆ alkyl, C₃-C₆ cycloalkyl, OCH₃, COOCH₃, or NHCOCH₂CH₃;
R⁵ is independently H, C₁-C₆ alkyl, C₃-C₆ cycloalkyl, phenyl, or benzyl;
R⁶ is independently H, halogen, CN, NO₂, CF₃, OCF₃, NH₂, NR^{e}R^{f}, C₁-C₆ alkyl, C₃-C₆ cycloalkyl, OCH₃, COOCH₃, or NHCOCH₂CH₃;
R⁷ is independently H, C₁-C₆ alkyl, or C₃-C₆ cycloalkyl;
R⁸ is independently H, halogen, CN, NO₂, CF₃, NH₂, NR^{e}R^{f}, C₁-C₆ alkyl, or C₃-C₆ cycloalkyl;
R^{e} and R^{f} is independently C₁-C₆ alkyl;
m is 0, 1, or 2;
n is 1, or 2;
w is 0, 1, or 2;
s is 0, 1, or 2;
u is 0, 1, or 2; and
q is 1 or 2,
and all other variables are as previously defined in Formula VII.

Preferably, the compounds of formula VIII have an absolute configuration S, in which the chiral carbon is on the indanyl that connects R³. More preferably, the compounds of formula VIII have an absolute configuration S.

In another embodiment of the compounds of Formula VIII, the compounds are represented by Formula VIIIa, Formula VIIIb, or Formula VIIIc: 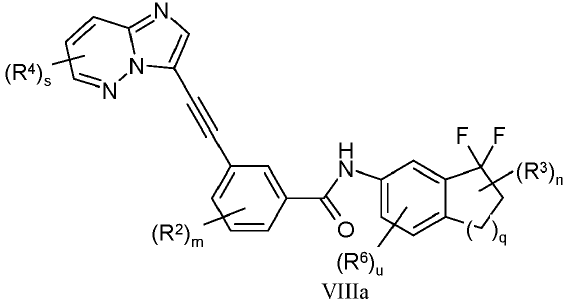 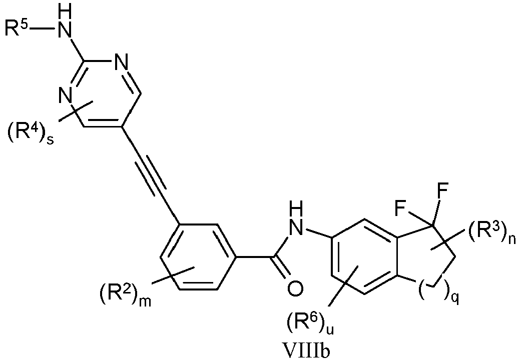 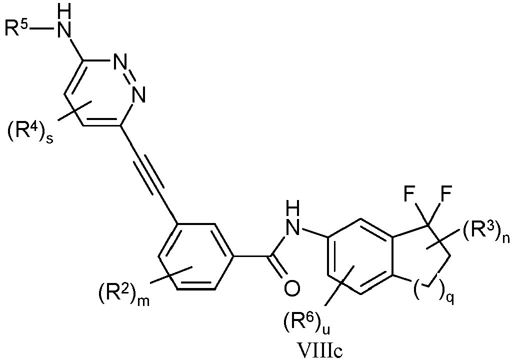 Wherein,
all variables are as previously defined in Formulas VIII.

In another embodiment of the compounds of Formulas VIIIa-VIIIc, R² is H, CH₃, or Cl; R³ is 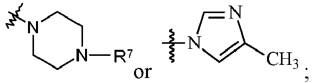 R⁵ is cyclopropyl, cyclobutyl, or isopropyl; R⁶ is H or CF₃ and s is 0, q is 1, u is 0, m is 0 or 1 and n is 1.

In another embodiment of the compounds of Formula I, G is 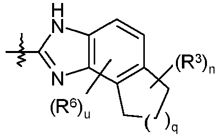 with the following structure of Formula IX, wherein u = 0, 1, or 2; and all other variables are as previously defined in Formula VII.

Disclosed herein are compounds of formula VIIIa, formula VIIIb or formula VIIIc having an absolute configuration S, in which the chiral carbon is on the indanyl that connects R³. Further disclosed herein is the compound of formula VIII having an absolute configuration S.

According to a preferred embodiment, in formula VIIIa, formula VIIIb or formula VIIIc having an absolute configuration S, R² is H, CH₃, F or Cl; R³ is 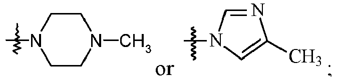 R⁵ is cyclopropyl, cyclobutyl, or isopropyl; R⁶ is H or CF₃; and s is 0, u is 0, m is 0 or 1, n is 1 and q is 1.

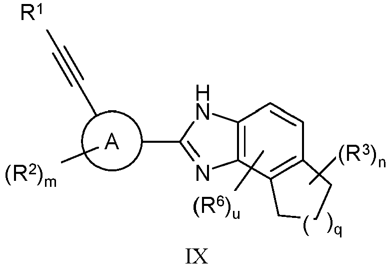

In another embodiment of the compounds of Formula IX, ring A is benzene with the following structure of Formula X: 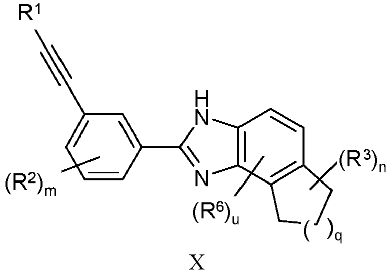 wherein,
R² is independently H, halogen, CN, NO₂, CF₃, OCF₃, NH₂, NR^{e}R^{f}, C₁-C₆ alkyl, C₃-C₆ cycloalkyl, OCH₃, COOCH₃, or NHCOCH₂CH₃;
R³ is 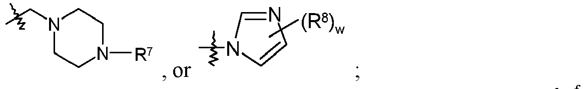
R⁴ is independently H, halogen, CN, NO₂, CF₃, OCF₃, NH₂, NR^{e}R^{f}, C₁-C₆ alkyl, C₃-C₆ cycloalkyl, OCH₃, COOCH₃, or NHCOCH₂CH₃;
R⁵ is independently H, C₁-C₆ alkyl, C₃-C₆ cycloalkyl, phenyl, or benzyl;
R⁶ is independently H, halogen, CN, NO₂, CF₃, OCF₃, NH₂, NR^{e}R^{f}, C₁-C₆ alkyl, C₃-C₆ cycloalkyl, OCH₃, COOCH₃, or NHCOCH₂CH₃;
R⁷ is independently H, C₁-C₆ alkyl, or C₃-C₆ cycloalkyl;
R⁸ is independently H, halogen, CN, NO₂, CF₃, NH₂, NR^{e}R^{f}, C₁-C₆ alkyl, or C₃-C₆ cycloalkyl;
R^{e} and R^{f} is independently C₁-C₆ alkyl;
m is 0, 1, or 2;
n is 1, or 2;
w is 0, 1, or 2;
s is 0, 1, or 2;
u is 0, 1, or 2; and
q is 1 or 2;
and all other variables are as previously defined in Formula VII.

In another embodiment of the compounds of Formula X, the compounds are represented by Formula Xa, Formula Xb, or Formula Xc: 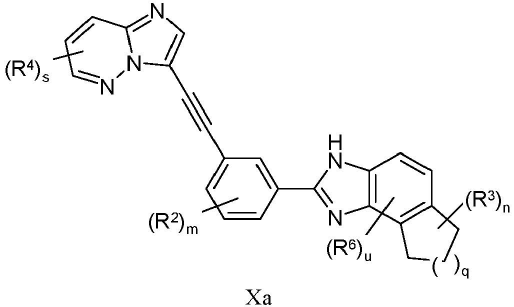 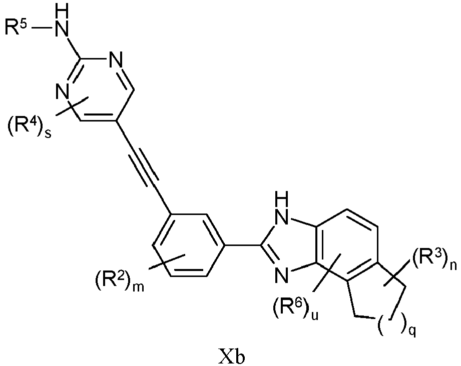 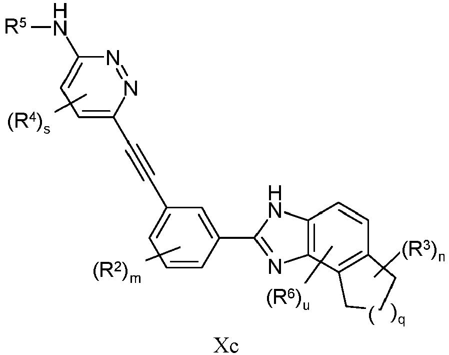 Wherein,
u is 0, 1, or 2;
q is 1, or 2;
n is 1, or 2;
and all other variables are as previously defined in Formulas IIIa-IIIc.

Some specific examples of the compounds of the invention include:

| Compound | Structure | HR/MS[M+H]⁺ |
|---|---|---|
| Reference compound 1 | 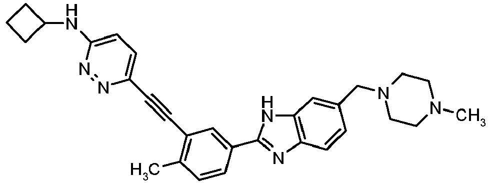 | 492.2856 |
| Reference compound 2 | 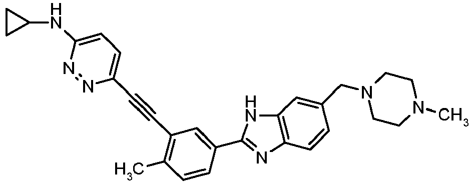 | 478.2708 |
| Reference compound 3 | 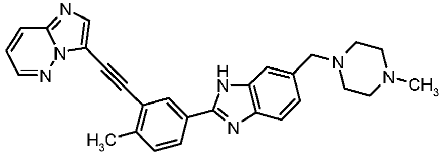 | 462.2413 |
| Reference compound 4 | 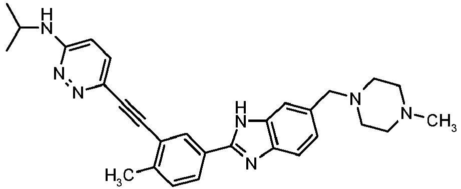 | 480.2847 |
| Reference compound 5 | 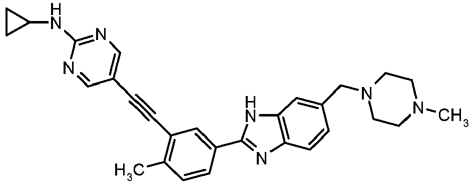 | 478.2718 |
| Reference compound 6 | 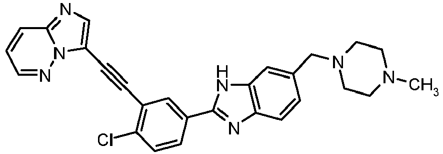 | 482.1841 |
| Reference compound 7 | 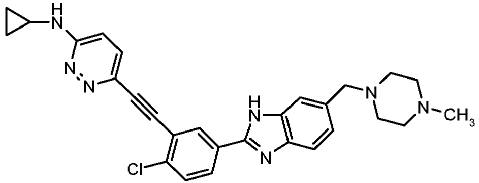 | 498.2150 |
| Reference compound 8 | 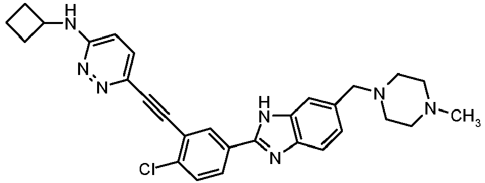 | 512.2303 |
| Reference compound 9 | 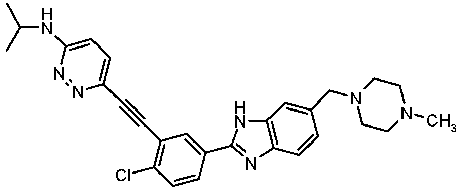 | 500.23 13 |
| Reference compound 10 | 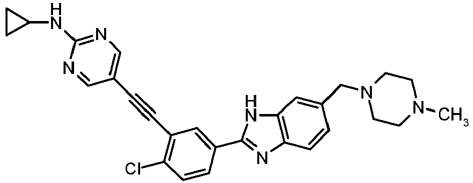 | 498.2163 |
| Reference compound 11 | 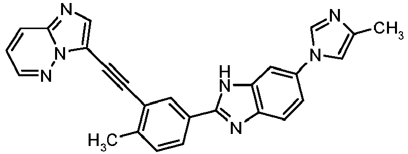 | 430.1778 |
| Reference compound 12 | 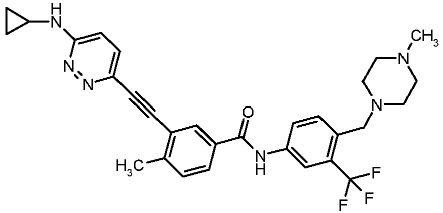 | 549.2568 |
| Reference compound 13 | 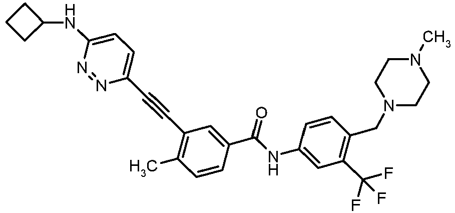 | 563.2768 |
| Reference compound 14 | 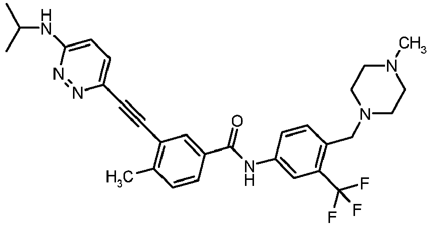 | 551.2734 |
| Reference compound 15 | 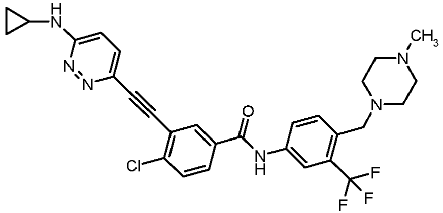 | 569.2000 |
| Reference compound 16 | 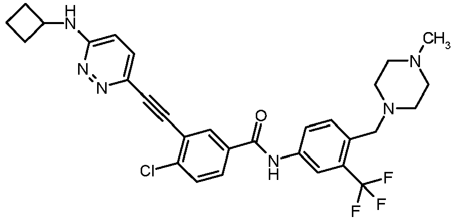 | 583.2174 |
| Reference compound 17 | 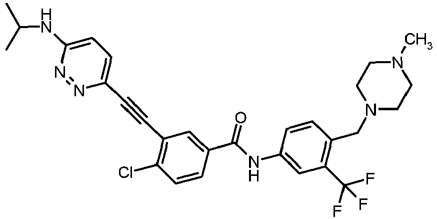 | 571.2209 |
| Reference compound 18 | 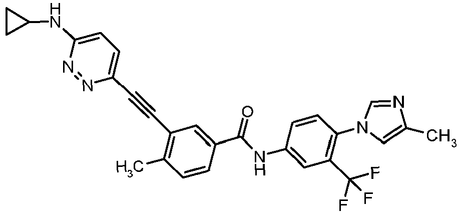 | 259.1023* [M+H]⁺² |
| Reference compound 19 | 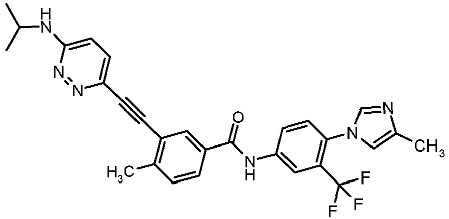 | 519.2116 |
| Reference compound 20 | 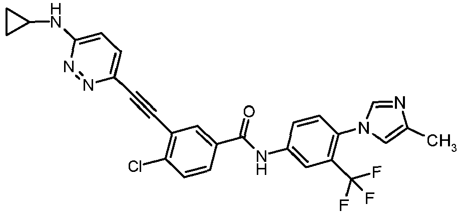 | 269.0735* [M+H]⁺² |
| Reference compound 21 | 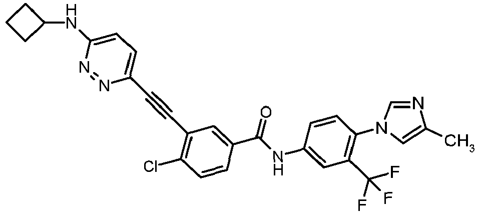 | 276.0817* [M+H]⁺² |
| Reference compound 22 | 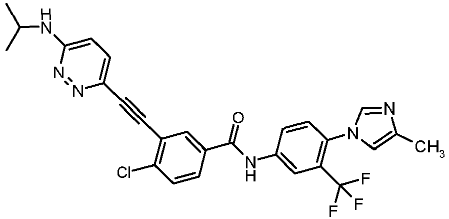 | 539.1592 |
| Reference compound 23 | 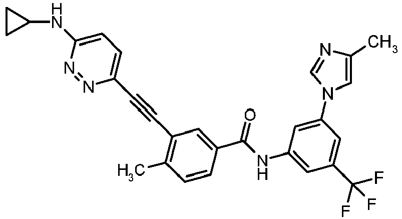 | 517.1950 |
| Reference compound 24 | 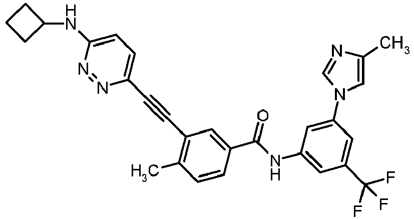 | 531.2113 |
| Reference compound 25 | 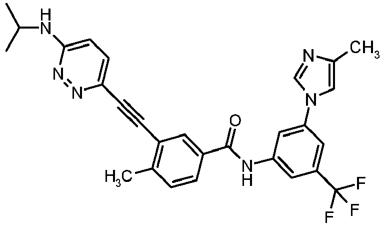 | 519.2121 |
| Reference compound 26 | 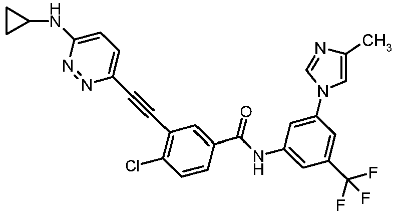 | 537.1413 |
| Reference compound 27 | 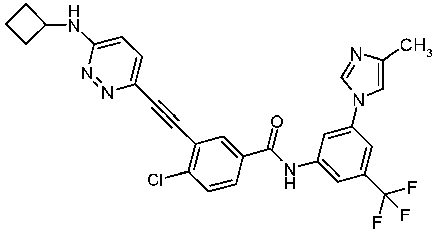 | 276.0819* [M+H]⁺² |
| Reference compound 28 | 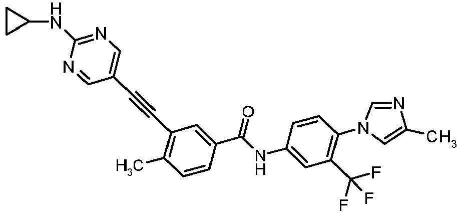 | 517.1960 |
| Reference compound 29 | 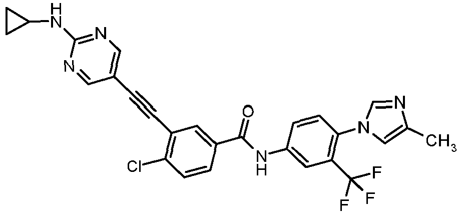 | 537.1417 |
| Reference compound 30 | 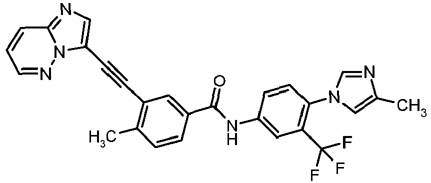 | 501.1626 |
| Reference compound 31 | 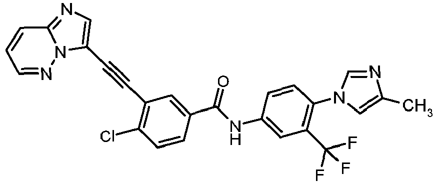 | 521.1099 |
| Reference compound 32 | 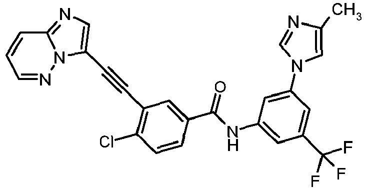 | 261.0584* [M+H]⁺² |
| Reference compound 33 | 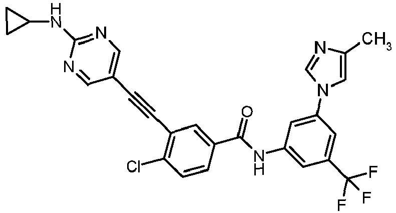 | 537.1399 |
| Reference compound 34 | 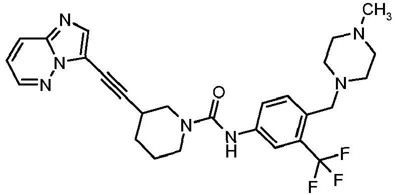 | 526.2536 |
| Reference compound 35 | 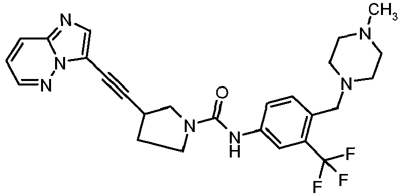 | 512.2375 |
| Reference compound 36 | 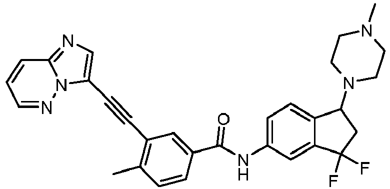 | 264.1212* [M+2H]⁺² |
| 36-(S) | 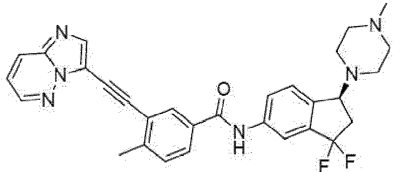 | 264.1218* [M+2H]⁺² |
| Reference compound 36-(R) | 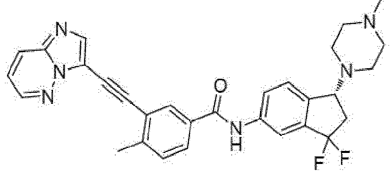 | 264.1217* [M+2H]⁺² |
| Reference compound 37 | 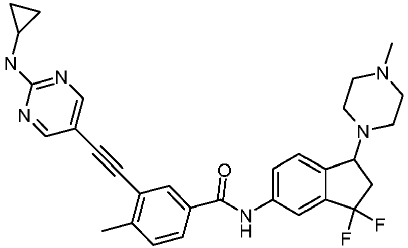 | 272.1370* [M+2H]⁺² |
| 37-(S) | 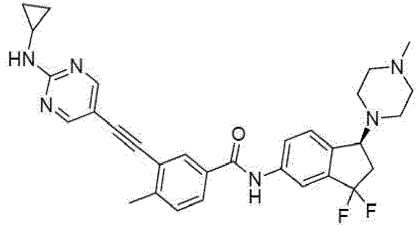 | 272.1374* [M+2H]⁺²* |
| Reference compound 37-(R) | 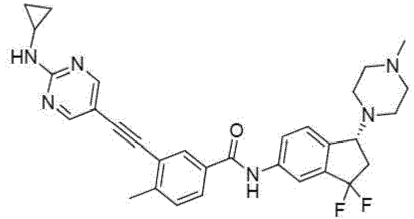 | 272.1373* [M+2H]⁺²* |
| Reference compound 38 | 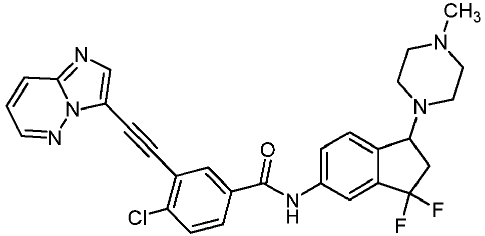 | 274.0962 [M+2H]⁺² |
| 38-(S) | 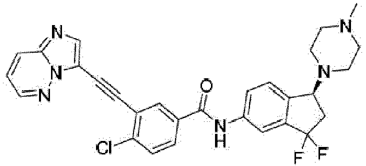 | 274.0949 [M+2H]⁺²* |
| Reference compound 38-(R) | 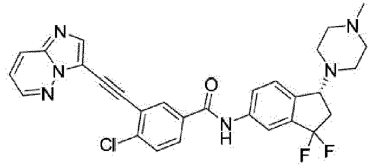 | 274.0944 [M+2H]⁺²* |
| Reference compound 39 | 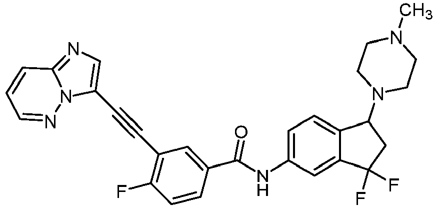 | 531.2120 [M+H]⁺¹ |
| 39-(S) | 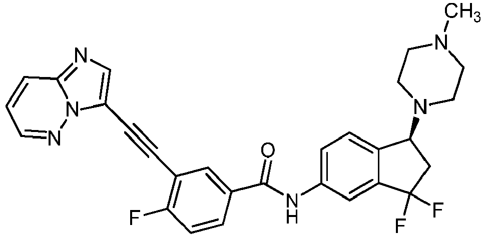 | 531.2122 [M+H]⁺¹ |
| Reference compound 39-(R) | 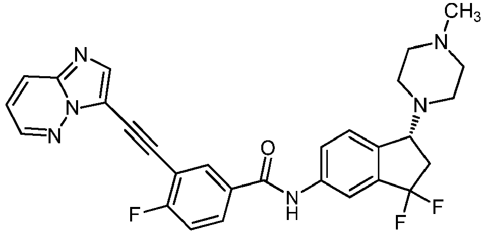 | 531.2125 [M+H]⁺¹ |

As used herein unless otherwise indicated, "alkyl" includes branched- and straight-chain saturated aliphatic hydrocarbon groups having the specified carbon atom numbers. Commonly used abbreviations for alkyl groups are used throughout the application, e.g. methyl may be represented by conventional abbreviations including "Me" or CH₃ or a symbol that is an extended bond without defined terminal group, e.g. " 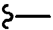 " ethyl is represented by "Et" or CH₂CH₃, propyl is represented by "Pr" or CH₂CH₂CH₃, butyl can be represented by "Bu" or CH₂CH₂CH₂CH_{3,}, etc. "C₁₋₆ alkyl" (or "C₁-C₆ alkyl") means branched or linear chain alkyl groups, including all isomers, having the specified number of carbon atoms. C₁₋₆ alkyl includes all of the hexyl alkyl and pentyl alkyl isomers as well as n-, iso-, sec- and t-butyl, n- and isopropyl, ethyl and methyl. If no number is provided, 1-10 carbon atoms are intended for linear or branched alkyl groups. C₁₋₆ alkyl may be unsubstituted or substituted with 1-3 fluorine or 1-3 chlorine atoms.

"Cycloalkyl" means C₃₋₁₀ carbocycles not containing heteroatoms. For example, cycloalkyl includes cyclopropyl, cyclobutyl, cyclopentyl, cyclohexyl, cycloheptyl, decahydronaphthyl, and the like.

"Aryl" means mono- and bicyclic aromatic rings containing 6-12 carbon atoms. Examples of aryl include, but are not limited to, phenyl, naphthyl, indenyl and so on. Aryl also includes monocyclic rings fused to an aryl group. Examples include tetrahydronaphthyl, indanyl and the like.

"Heterocyclyl", unless otherwise indicated, means a 4-, 5-, 6-, 7- or 8-membered monocyclic saturated ring containing 1-2 heteroatoms selected from N, O and S, in which the point of attachment may be carbon or nitrogen. Examples of "heterocyclyl" include, but are not limited to, piperidinyl, piperazinyl, morpholinyl, azetidinyl, pyrrolidinyl, oxazolidinyl, imidazolidinyl, 2,3-dihydrofuro(2,3-b)pyridyl, benzoxazinyl, and so on. The term also includes partially unsaturated monocyclic rings that are not aromatic, such as 2- or 4-pyridones attached through the nitrogen or *N*-substituted-(1*H*, 3*H*)-pyrimidine-2, 4-diones (*N*-substituted uracils). Heterocyclyl may also include such moieties in charged form, e.g., piperidinium.

"Heteroaryl" means a mono- or bicyclic aromatic ring or ring system having 5 to 10 atoms and containing 1-3 heteroatoms selected from N, O, and S. Examples include, but are not limited to, oxadiazolyl, thiadiazolyl, pyrrolyl, furanyl, triazinyl, thienyl, pyrimidyl, pyrimidinyl, pyridazinyl, pyrazinyl, isoxazolyl, triazolyl, isothiazolyl, pyrazolyl, imidazolyl, pyridyl, pyridinyl,oxazolyl, thiazolyl, tetrazolyl, and the like. Heteroaryl also includes aromatic heterocyclic groups fused to heterocycles that are non-aromatic or partially aromatic, and aromatic heterocyclic groups fused to cycloalkyl rings. Additional examples of heteroaryls include, but are not limited to, imidazopyridinyl, imidazopyridazinyl, pyrazolopyrazolyl, indazolyl, thienopyrazolyl, pyrazolopyridinyl, and imidazothiazolyl. Heteroaryl also includes such groups in charged form, such as pyridinium. In an embodiment, heteroaryl is imidazolyl, oxadiazolyl, pyrazolyl, oxazolyl, and pyridinyl.

"Heterocyclic alkyl", unless otherwise indicated, includes both branched- and straight-chain saturated aliphatic hydrocarbon groups which is bonded to a carbon or nitrogen atom of a heterocyclyl, as described above.

"Halogen (or halo)" includes fluorine (fluoro), chlorine (chloro), bromine (bromo) and iodine (iodo). In one embodiment, halogen is chlorine or fluorine.

Substitution by a named substituent is permitted on any atom in a ring (e.g., aryl, a heteroaryl ring, or a saturated heterocyclic ring) provided such ring substitution is chemically allowed and results in a stable compound. A "stable" compound can be prepared and isolated, and whose structure and properties remain or can be caused to remain essentially unchanged for a period of time that allows use of the compound for the described purposes.

Under standard nomenclature used throughout this disclosure, the terminal portion of the designated side chain is described first, followed by the adjacent functionality toward the point of attachment. For example, a C₁₋₅ alkyl COOR is equivalent to

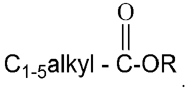

When a variable (e.g., R, R^{x}, etc.) occurs more than once in any constituent or formula, its definition on each occurrence is independent of its definition at every other occurrence. In addition, combinations of substituents and/or variables are allowed only if such combinations lead to stable compounds.

In choosing compounds of the present disclosure, one of ordinary skill in the art will recognize that the various substituents, i.e. R¹, R², R, etc., are to be chosen in conformity with common principles of chemical structure connectivity and stability.

The term "substituted" is used to include multiple degrees of substitution by a named substituent. Where multiple substituents are claimed, the substituted compound can be independently substituted by one or more of the disclosed substituents. By independently substituted, it is meant that the (two or more) substituents can be the identical or different.

Where a substituent or variable has multiple definitions, the substituent or variable is defined as being selected from the group consisting of the indicated definitions.

### Isomers: (Optical isomers, diastereoisomers, tautomers, atropisomers, geometric isomers):

Compounds of structural Formula I may contain one or more chiral centers and can occur as racemic mixtures, a single enantiomer, diastereoisomeric mixtures and a single diastereoisomer. The disclosure contains all such isomeric forms of the compounds covered by Formula I when applicable.

Compounds of structural Formula I may be separated into their individual enantiomers or diastereoisomers by fractional crystallization from a suitable solvent, or via chiral chromatography using an optically active immobile phase. Absolute configuration may be determined by X-ray crystallography of crystalline products or intermediates, or the chirality from vendors who provided chiral material.

Stereoisomers or isomers of a compound of Formula I may be obtained by stereoselective synthesis using optically pure starting materials or reagents of known absolute configuration.

Racemic mixtures of the compounds may be separated by methods well known in the art, such as chrial chromatography, fractional crystallization, the coupling of a racemic mixture of compounds to an enantiomerically pure compound to form a diastereoisomeric mixture, followed by separation of the individual diastereoisomers by chromatography. The coupling reaction may be the formation of salts using an enantiomerically pure acid or base. The diasteromeric derivatives may then be converted to the pure enantiomers by removal of the chiral additives.

Some of the compounds presented in this disclosure may exist as tautomers with different points of attachment of hydrogen accompanied by one or more double bond shifts. For example, a ketone and its enol form are keto-enol tautomers. The individual tautomers as well as mixtures thereof are both covered with compounds of the present invention.

For compounds described herein which contain olefinic double bonds, unless otherwise specified, they include both E and Z olefin isomers.

All atropisomers of compounds covered by Formula I, if applicable, are included in the present application. Atropisomers are stereoisomers resulting from hindered rotation of single bonds where the steric strain barrier to rotation is significant enough to allow for the isolation of the conformers. Separation of atropisomers is possibly by chiral resolution methods such as chiral chromatography or selective crystallization.

In the compounds of Formula I, the atoms may exhibit their natural isotopic abundances. Alternatively, one or more of the atoms may be artificially enriched in a particular isotope having the same atomic number, but an atomic mass different from the atomic mass predominately found in nature. The application includes all suitable isotopic variations of the compounds of Formula I. For example, different isotopic forms of hydrogen (H) include protium (¹H) and deuterium (²H, also denoted as D). Enriching for deuterium may afford certain therapeutic advantages, such as increasing metabolic stability, *in vivo* half-life or reducing dosage requirements, or may provide a compound useful as a standard for characterization of biological samples. Isotopically-enriched compounds within structural Formula I, can be prepared by conventional techniques.

The present disclosure includes all stereoisomeric forms (as described above), in all ratios, of the compounds of the Formula I.

### Salts:

Compounds of structural Formula I also cover the pharmaceutically acceptable salts, and salts that are not pharmaceutically acceptable when they are used as precursors to the free compounds or their pharmaceutically acceptable salts or in salt conversions.

The compounds disclosed herein may be administered in the form of a pharmaceutically acceptable salt. The term "pharmaceutically acceptable salt" means salts prepared from pharmaceutically acceptable bases or acids including inorganic or organic bases or acids. Pharmaceutically acceptable salts of basic compounds refer to non-toxic salts of the compounds disclosed herein which are generally prepared by mixing the free base with a suitable organic or inorganic acid. Representative salts of basic compounds disclosed herein include, but are not limited to, the following: acetate, ascorbate, benzenesulfonate, benzoate, bicarbonate, bisulfate, bitartrate, borate, bromide, butyrate, camphorate, camphorsulfonate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, methanesulfonate, mucate, napsylate, nitrate, N-methylglucamine ammonium salt, oleate, oxalate, pamoate (embonate), palmitate, pantothenate, phosphate/diphosphate, polygalacturonate, propionate, salicylate, stearate, sulfate, subacetate, succinate, tannate, tartrate, teoclate, thiocyanate, tosylate, triethiodide, valerate and the like. Suitable pharmaceutically acceptable salts of acids covered by Formula I include, but are not limited to, salts generated from inorganic bases including aluminum, ammonium, calcium, copper, ferric, ferrous, lithium, magnesium, manganic, mangamous, potassium, sodium, zinc, and the like. Salts derived from pharmaceutically acceptable organic non-toxic bases include salts of primary, secondary, and tertiary amines, cyclic amines, dicyclohexyl amines and basic ion-exchange resins, such as arginine, betaine, caffeine, choline, N,N-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and so on.

If a carboxylic acid (-COOH) or an alcohol is present in the compounds disclosed herein, pharmaceutically acceptable esters of carboxylic acid derivatives, such as methyl, ethyl, or pivaloyloxymethyl, or acyl derivatives of alcohols, such as *O*-acetyl, *O*-pivaloyl, *O*-benzoyl, and O-aminoacyl, can be utilized. Included are esters and acyl groups known in the art for modifying the solubility or hydrolysis characteristics for use as controlled-release or prodrug formulations.

If the compounds of Formula I contain both acidic and basic groups in the disclosure, inner salts or betaines (zwitterions) can be obtained via customary methods known to the person skilled in the art. For example, one can combine an organic or inorganic acid or base in a solvent or dispersant, or by anion exchange or cation exchange from other salts. The present disclosure also covers all salts of the compounds of Formula I which may not suitable as pharmaceuticals but can be used for the preparation of physiologically acceptable salts.

Solvates and hydrates of the compounds of Formula I are also included in the present disclosure.

The present disclosure also discloses processes to synthesize the compounds of Formula I which are described in the following.

One aspect of the disclosure that is of interest relates to a compound in accordance with Formula I or a pharmaceutically acceptable salt, hydrate, isomer, or solvate thereof for use in a method of treatment of the human by therapy.

The disclosure relates to a compound in accordance with Formula I or a pharmaceutically acceptable salt, hydrate, isomer, or solvate thereof for use as an anti-cancer agent in a human, wherein said cancer includes, but is not limited to, chronic myeloid leukemia (CML) and acute lymphoblastic leukemia (ALL).

Further disclosed herein is a therapeutically effective amount of a compound of Formula I or a pharmaceutically acceptable salt, hydrate, isomer, or solvate thereof for use in treating inflammations in a human patient.

Further disclosed herein are pharmaceutical preparations or pharmaceutical compositions which comprise as active component an effective dose of at least one compound of the Formula I, and/or a physiologically acceptable salt, hydrate, isomer, or solvate thereof, and one or more pharmaceutically acceptable carrier substances and/or additives.

The pharmaceutical compositions disclosed herein can be administered orally (e.g. in the form of pills, tablets, granules, hard and soft gelatin capsules, lacquered tablets, sugar-coated tablets, aqueous, syrups, alcoholic or oily solutions, emulsions, suspensions, etc), rectally (e.g. in the form of suppositories), parenterally, subcutaneously, intramuscularly or intravenously (in the form of solutions for injection or infusion), percutaneously or topically (e.g. for example in the form of ointments, tinctures, sprays or transdermal therapeutic systems), or inhalatively (e.g. in the form of nasal sprays or aerosol mixtures, microcapsules, implants or rods). The preferred administration form depends on the progress and severity of disease to be treated.

The compounds of the Formula I and their physiologically acceptable salts, hydrates, isomers, or solvates can be administered to animals, in particular to humans, as pharmaceuticals by themselves, in mixtures with one another or in the form of pharmaceutical preparations. A therapeutically effective amount means that amount of a drug or pharmaceutical agent that will elicit the biological or medical response of a tissue, a system, an animal or a human.

The amount of active compound of the Formula I and/or its physiologically acceptable salts, hydrates, isomers, or solvates in the pharmaceutical preparations normally is from 1 to 2000 mg, preferably from 1 to 500 mg, per dose, but may be higher depending on the type of the pharmaceutical composition. The pharmaceutical preparations typically comprise 0.5 to 90 percent by weight of the compounds of the Formula I and/or their physiologically acceptable salts. One or more compounds of the Formula I and/or their physiologically acceptable salts, together with one or more solid or liquid pharmaceutical carrier substances and/or additives (or auxiliary substances) and, if desired, in combination with other pharmaceutically active compounds having therapeutic action, are formulated in a suitable form which can then be used as a pharmaceutical in human or animal health.

To produce pills, tablets, sugar-coated tablets and hard gelatin capsules, it is possible to use lactose, starch, for example maize starch, or starch derivatives, talc, stearic acid or its salts, etc. Carriers for soft gelatin capsules and suppositories include fats, waxes, semisolid, liquid polyols, natural or hardened oils, etc. Suitable carriers for the preparation of solutions, for example of solutions for injection, or of emulsions or syrups include, for example, water, physiologically sodium chloride solution, alcohols such as ethanol, glycerol, polyols, sucrose, mannitol, vegetable oils, invert sugar, glucose, etc. The compounds of the Formula I and their physiologically acceptable salts may be used to generate lyophilisates, which may be used for preparing preparations for injection or infusion. Suitable carriers for microcapsules, implants or rods are, for example, copolymers of lactic acid and glycolic acid.

Besides active compounds and carriers, the pharmaceutical preparations can also contain customary additives, for example, aromatizers, buffer substances, fillers, diluents, disintegrants, dispersants, binders, colorants, flavorings, emulsifiers, lubricants, preservatives, stabilizers, thickeners,sweeteners, wetting agents, solvents, solubilizers, agents for achieving a depot effect, salts for altering the osmotic pressure, coating agents or antioxidants.

The dosage of the active compound of the Formula I to be administered and/or of a physiologically acceptable salt thereof depends on the individual case, such as the nature and the severity of the disorder to be treated, sex, age, weight and individual responsiveness of the human or animal to be treated, the efficacy and duration of action of the compounds used, whether the therapy is acute or chronic or prophylactic, or whether other active compounds are administered in addition to compounds of the Formula I. In general, a daily dose of approximately 0.01 to 100 mg/kg, preferably 0.3 to 10 mg/kg (in each case mg per kg of bodyweight) is appropriate for administration to an adult to obtain the desired results. The daily dose can be administered in a single dose or multiple doses.

The aforementioned compounds are also of use in combination with other pharmacologically active compounds. Additional active compounds that may be used in combination with the compounds disclosed herein, either co-administered or in a fixed combination, include, but are not limited to anticancer alkylating or intercalating agents, antimetabolites, purine antagonists or pyrimidine antagonists, spindle poisons, podophyllotoxins, antibiotics, nitrosoureas, inorganic ions, enzymes, hormones, mTOR inhibitors, protease inhibitors, NF-kB inhibitor, other inhibitors of kinases (e.g. Src, Brc/Abl, kdr, Flt3, Aurora, GSK-3, EGFR, VEGFR, FGFR, JNK, PKC, CDKs, Syk, JAK, PDGFR, cMET, MEK, AKT, PI3K, c-kit, fit-3, IGFR, ErbB2, etc), antibodies, soluble receptor or other receptor antagonists against a receptor or hormone implicated in a cancer, etc.

Examples of other active ingredients that may be administered in combination with a compound of Formula I, and either administered separately or in the same pharmaceutical composition, include, but are not limited to:
Mechlorethamine, Chlorambucil, Cyclophosphamide, Melphalan, Ifosfamide, Methotrexate, 6-Mercaptopurine, 5-Fluorouracil, Cytarabile, Gemcitabine, Vinblastine, Vincristine, Vinorelbine, Paclitaxel, Etoposide, Irinotecan, Topotecan, Doxorubicin, Bleomycin, Mitomycin, Carmustine, Lomustine, Cisplatin, Carboplatin, Oxaliplatin, Oxiplatin, Asparaginase, Tamoxifen, Leuprolide, Flutamide, Megestrol, Sirolimus, Temsirolimus, Everolimus, AP23573, Velcade, Iressa, Tarceva, Herceptin, Avastin, Erbitux, Zyloprim, Alemtuzmab, Altretamine, Amifostine, Nastrozole, MLN-591, MLN591RL, MLN2704, Arsenic trioxide, Bexarotene, Busulfan, Capecitabine, Gliadel Wafer, Celecoxib, Chloramubucil, Cisplatin-epinephrine gel, Cladribine, Cytarabine liposomal, Daunorubicin liposomal, Daunorubicin, Daunomycin, Dexrazoxane, Docetaxel, Doxorubicin, Elliott's B solution, Epirubicin, Estramustine, Etoposide Phosphate, Etoposide, Exemestane, Fludarabine, 5-FU, Fulvestrant, Gemcitabine, Gemtuzumab-ozogamicin, Goserelin acetate, Hydroxyurea, Idarubicin, Idamycin, Imatinib mesylate, irinotecan, MLN576, Letrozole, Leucovorin, Leucovorin levamisole, melphalan, L-PAM, Mesna, Mitomycin C, Mitoxantrone, Methoxsalen, MLN518, MLN608, Itoxantrone, Rituximab, Talc, Temozolamide, Teniposide, VM-26, Topotecan, Pegademase, Pentostatin, Porfimer sodium, 2C4, Tretinoin, ATRA, Valrubicin, Vinorelbine, Pamidronate, Zoledronate.

Furthermore, the disclosure that is of interest is a method for inhibiting protein kinase comprising administering a therapeutically effective amount of a compound of Formula I, or a salt, hydrate, isomer, or solvate thereof, or a pharmaceutical composition of above described. The protein kinase above mentioned includes, but is not limited to, Bcr-Abl.

The compounds of Formula I can be synthesized in accordance with the general schemes provided below, taking into account the specific examples that are provided. Preferred methods include, but are not limited to those described below. Throughout the synthetic schemes and examples, abbreviations are used with the following meanings unless otherwise indicated:
Ac is acetate, or acetyl;
aq. is aqueous;
AIBN is 2,2'-azobis(2-methylpropionitrile);
Ar is Aryl;
Bn is benzyl;
Boc is *tert* butylcarbamoyl;
br is broad;
Bu is butyl;
*^{t}*Bu is tert-butyl;
celite is Celite® diatomaceous earth;
*^{c}*Pr is cyclopropyl;
DCM is dichloromethane;
DIPEA is N,N-diisopropylethylamine;
DMAP is 4-dimethylaminopyridine
DMF is *N,N*-dimethylformamide;
DMSO is dimethyl sulfoxide;
EDCI is *N*-(3-dimethylaminopropyl)-*N*'-ethylcarbodiimide hydrochloride;
EDTA is ethylendiamine tetraacetic acid;
ES-MS is electrospray ion-mass spectroscopy;
Et is ethyl;
Et₃N is triethylaimne;
Et₂O is diethyl ether;
EtOH is ethanol,
EtOAc is ethyl acetate;
halo is a halogen (preferably fluorine or chlorine);
HetAr or HAR is Heteroaryl;
¹HNMR is proton nuclear magnetic resonance;
HOBt is 1-hydroxybenzotriazole;
HPLC is high performance liquid chromatography;
Hz is hertz;
*i* is Iso;
kg is kilogram;
LC/MS is Liquid chromatography / Mass Spectroscopy;
M is molar;
Me is methyl;
µg is microgram;
MeCN is acetonitrile;
MeOH is methanol;
MHz is megahertz;
mm is millimeter;
µL is microliter;
mM is milimolar;
µM is micromolar;
mmol is milimoles;
MS is mass spectrum, and a mass spectrum obtained by ES-MS may be denoted herein by "ES";
mw is microwave;
*m*/*z* is mass to charge ratio;
*n* is normal;
NBS is N-bromosuccinimide;
nm is nanometer;
nPr is *n*-propyl;
*p* is para;
PE is petroleum;
Ph is phenyl;
Pr is propyl;
rt is room temperature;
*sec* is secondary;
*^{t}*Bu is *tert-butyl;*
*^{t}*BuOH is tert-butanol;
*tert* is tertiary;
TBAF is tetrabutylammonium fluoride;
TFA is trifluoroacetic acid;
THF is tetrahydrofuran;
TLC is thin layer chromatography;
TMSA is trimethylsilylacetylene;
TMSOTf is trimethylsilyl trifluoromethanesulfonate;
U is units;
UV is ultraviolet;

### SCHEMES

Reaction schemes 1-12 illustrate the methods employed in the synthesis of the compounds of Formula I. All abbreviations are as defined above unless indicated otherwise. In the Schemes, all substituents are as defined above in Formula I unless indicated otherwise.

Synthetic methods for preparing the compounds of the present invention are illustrated in the following Schemes and Examples. Starting materials are commercially available or may be made according to procedures known in the art or as illustrated herein.

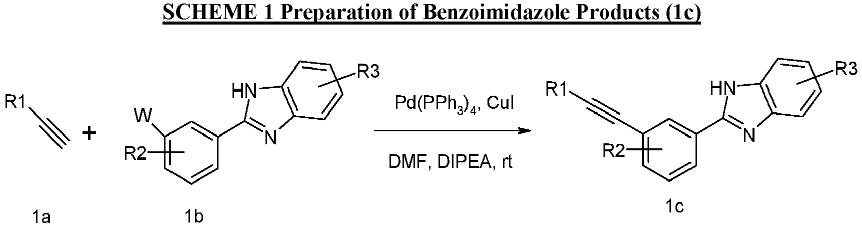

As shown in SCHEME 1, a palladium catalyzed Sonogashira coupling reaction is used to afford the final product 1c. In Scheme 1, the Sonogashira coupling reaction is performed with an acetylenic moiety 1a and a benzimidazole moiety 1b which has been activated by the presence of a reactive group, W, which is an I, a Br or another reactive group permitting the desired coupling reaction.

Several illustrative overall synthetic approaches to the preparation of the acetylenic moieties, based on known transformations, are described below in SCHEME 2 to 4:

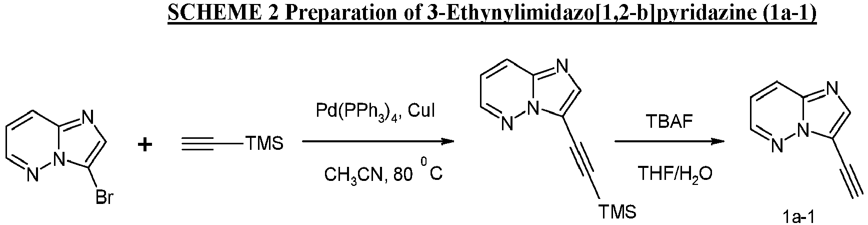

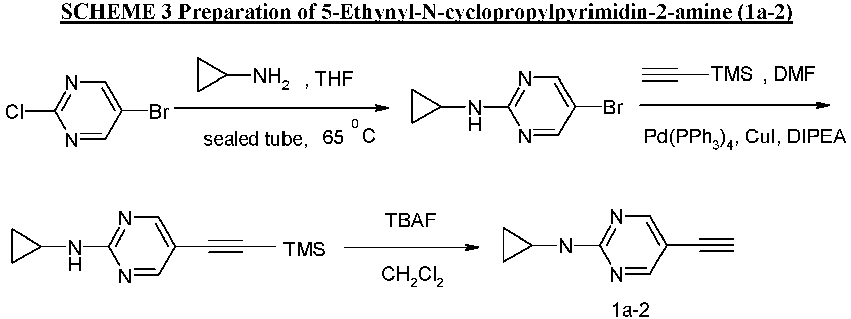

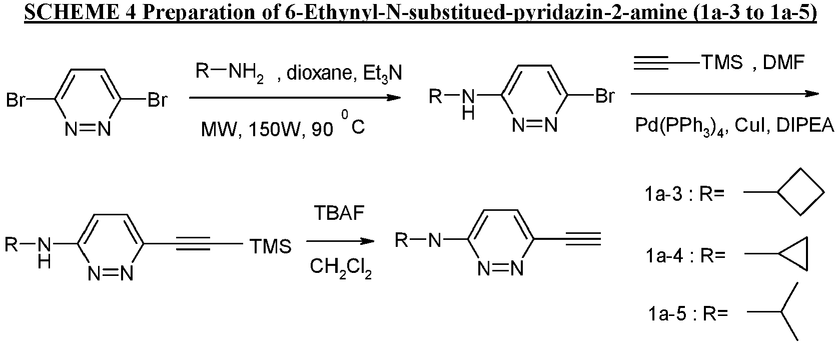

SCHEME 5 to 6 below depict the synthesis of some representative benzoimidazole compounds of the formula 1b which are useful as intermediates in the coupling reaction described in SCHEME 1.

SCHEME 5 describes an illustrative synthesis of the formula lb-1 and lb-2 in which R3 is (4-methylpiperazin-1-yl)methyl, and R2 is methyl and chloro respectively.

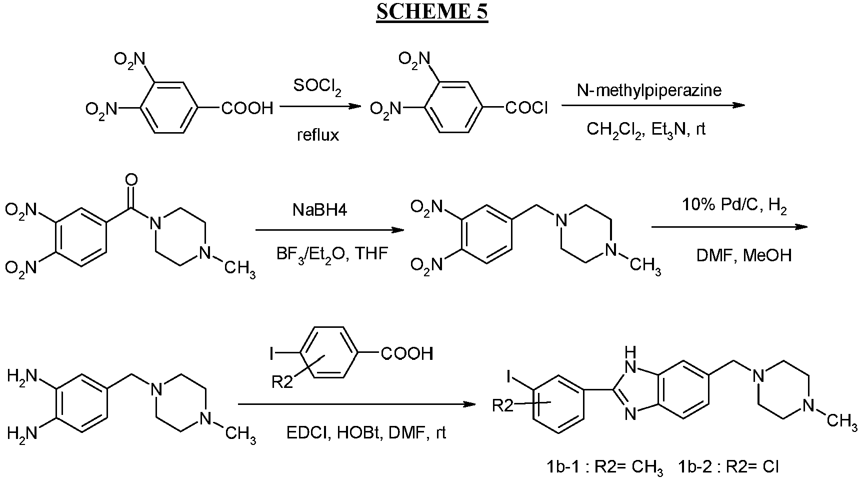

SCHEME 6 describes an illustrative synthesis of the formula lb-3 in which R3 is 4-methyl-1H-imidazol-1-yl, and R2 is methyl.

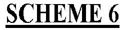

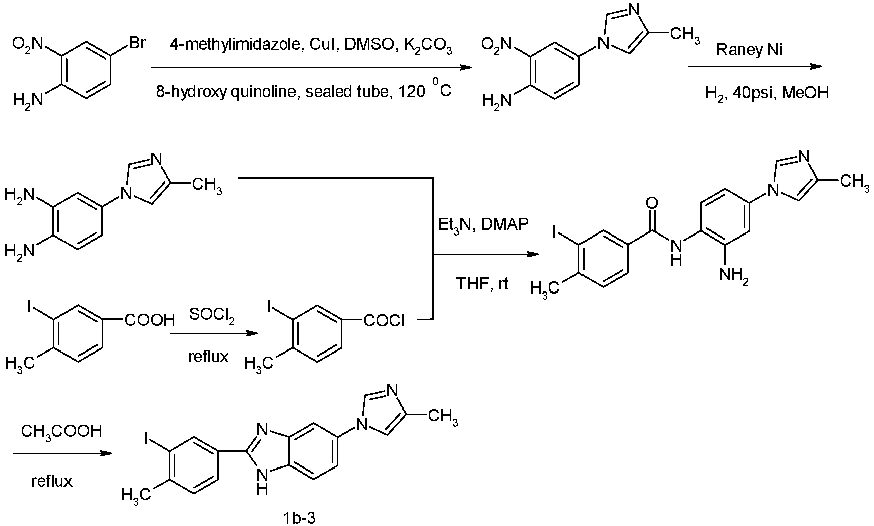

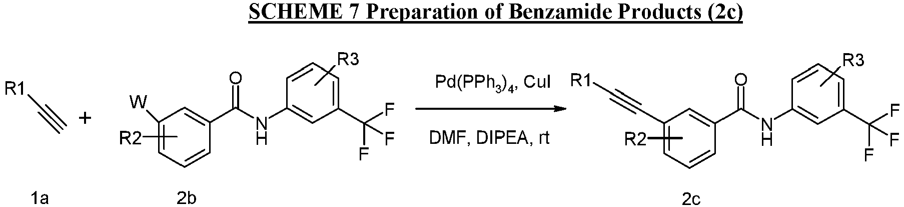

Like SCHEME 1, in SCHEME 7, an acetylenic moiety **1a** condensed with a benzamide moiety **2b** through the Sonogashira coupling reaction to afford benzamide analogs **2c.**

SCHEME 8 to 10 below depict the synthesis of some representative benzamide compounds of the formula **2b** which are useful as intermediates in the coupling reaction described in SCHEME 1.

SCHEME 8 and 9 depict the synthesis of **2b** in which R3 is 4-methyl-1H-imidazol-1-yl, R2 is methyl or chloro group respectively. Some representative intermediates include following structures:

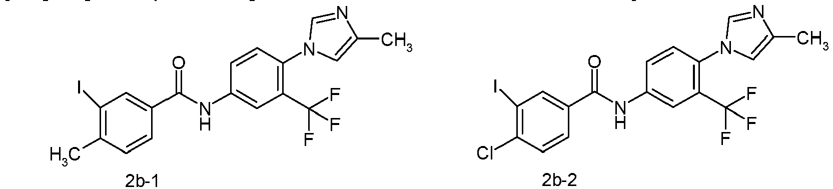

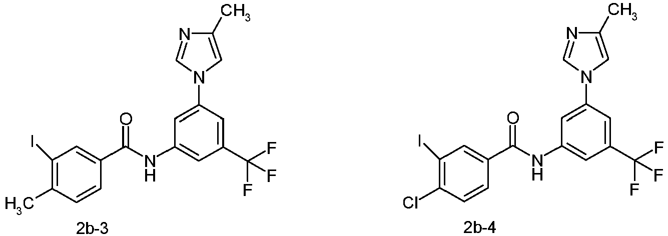

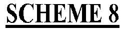

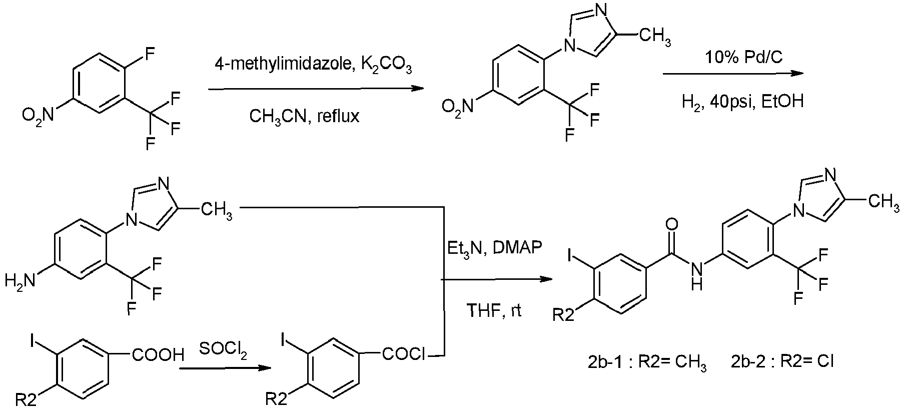

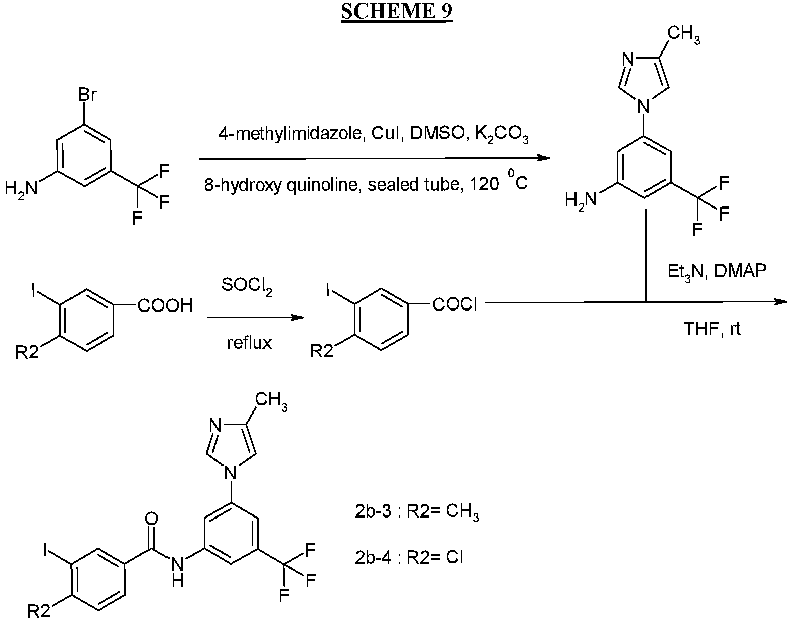

SCHEME 10 decribes the synthesis of **2b** in which R3 is (4-methylpiperazin-1-yl)methyl, R2 is methyl or chloro group respectively.

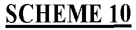

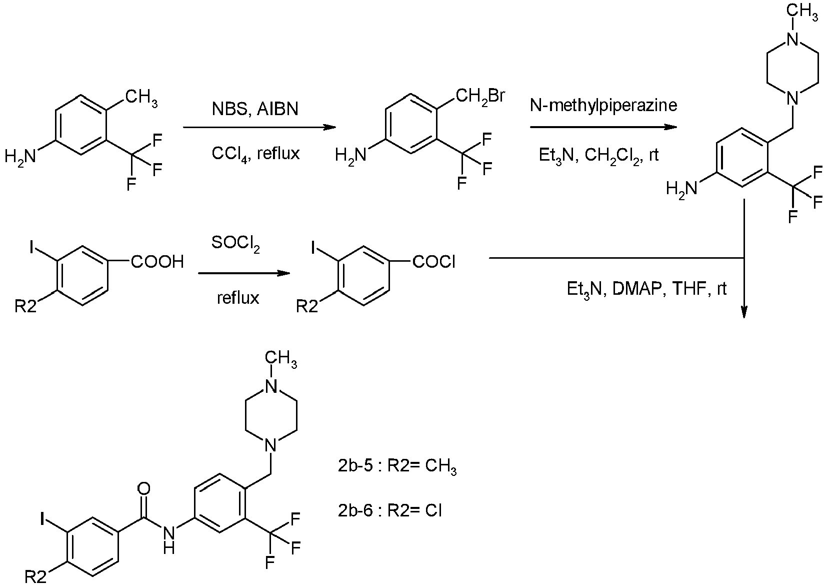

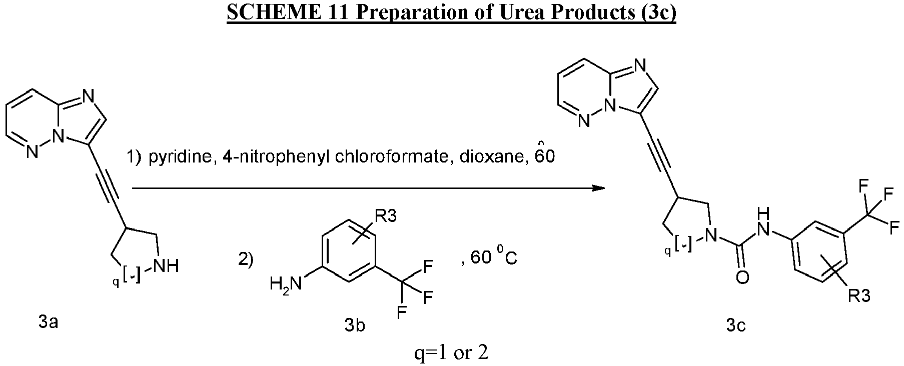

As shown in SCHEME 11, an amine containing acetyl moiety **3a** condensed with a substituted aromatic amine moiety 3b to afford urea analogs **3c.**

SCHEME 12 below depicts the synthesis of some representative compounds of the formula **3a** which are useful as intermediates in the condensation reaction described in SCHEME 11. The synthesis of aromatic amine compounds of the formula **3b** has been described in SCHEME 8 to 10 for the synthesis of **2b-1** to **2b-6.**

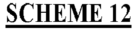

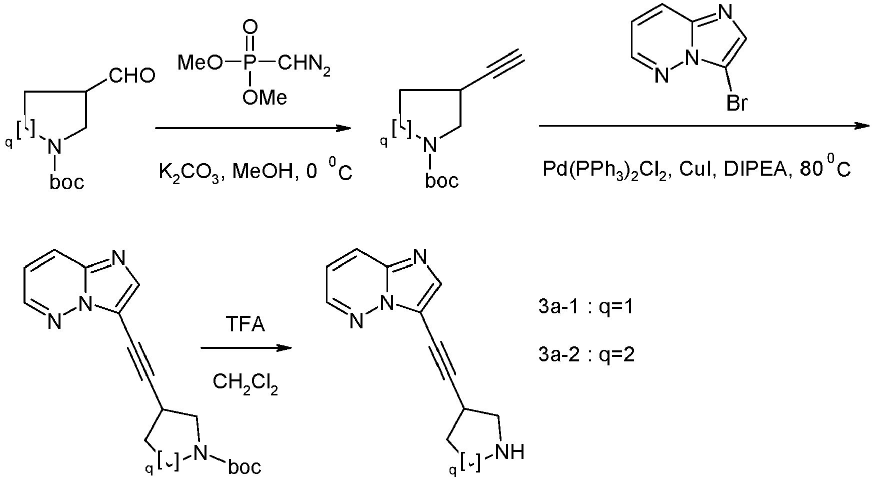

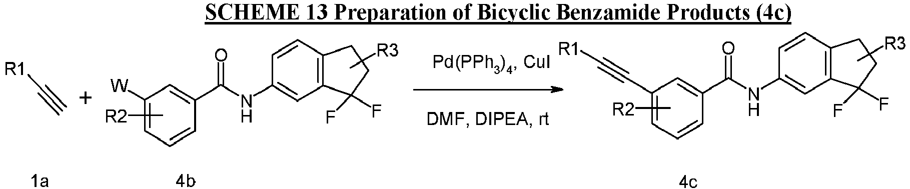

Like SCHEME 1, in SCHEME 13, an acetylenic moiety **1a** condensed with a benzamide moiety 4b through the Sonogashira coupling reaction to afford benzamide analogs **4c.**

SCHEME 14 decribes the synthesis of **4b** in which R3 is (4-methylpiperazin-1-yl)methyl, and R2 is methyl.

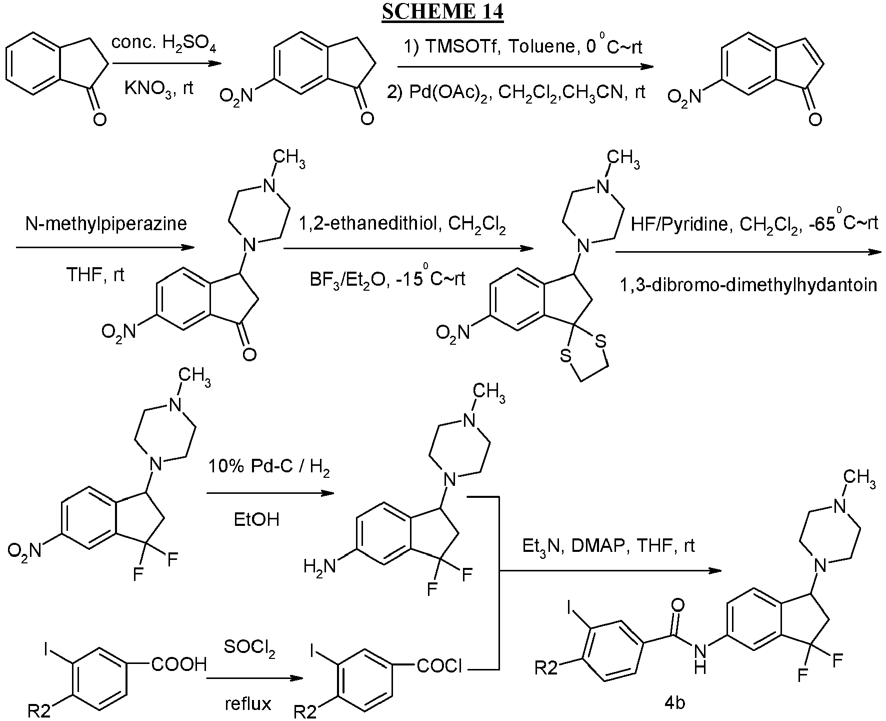

### Examples

In the following examples, only compounds 36(S) to 39(S) are according to the present invention.

### Example 1

### N-Cyclobutyl-6-(2-(2-methyl-5-(6-((4-methylpiperazin-1-yl)methyl)-1H-benzo[d]imidazol-2-yl)phenyl)ethynyl)pyridazin-3-amine

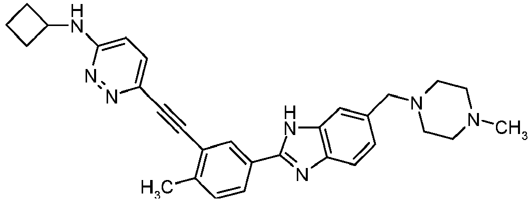

### Step 1. 6-Bromo-N-cyclobutylpyridazin-3-amine

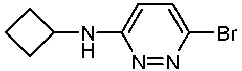

Into a solution of 3,6-dibromopyridazine (1.19 g, 5 mmol) in dioxane (5 mL) was added cyclobutylamine (0.39 g, 5.5 mmol), and Et₃N (0.60 g, 6 mmol). The reaction was microwave at 90°C, 150W for 0.45 h. The reaction was monitored by TLC and the crude product was purified by silica gel chromatography (eluent: 30% Ethyl acetate in n-hexane), to give the desired product (0.63 g, 55.3%). ¹H NMR (300 MHz, CDCl₃) δ: 7.25-7.28 (1 H, d, *J* = 9.0 Hz), 6.49-6.52 (1 H, d, *J* = 9.0 Hz), 5.27 (1 H, s), 4.16-4.24 (1 H, m), 2.40-4.49 (2 H, m), 1.87-1.95 (2 H, m), 1.75-1.84 (2 H, m).

### Step 2. N-Cyclobutyl-6-(2-(trimethylsilyl)ethynyl)pyridazin-3-amine

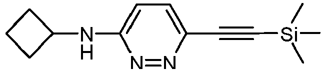

6-Bromo-N-cyclobutylpyridazin-3-amine (0.60 g, 2.63 mmol), trimethylsilylacetylene (TMSA) (1.29 g, 13.1 mmol), Pd(PPh₃)₄ (0.15 g), and CuI (0.04 mg) were placed in a vial with a rubber septum. After the mixture underwent 3 cycles of vacuum/filling with Ar₂, DMF (3.0 mL) and N,N-diisopropylethylamine (DIPEA) (0.41 g, 3.2 mmol) were added. The mixture was then stirred at 80°C for 16 hrs. The reaction mixture was concentrated and the resulting residue was purified by silica gel chromatography (eluent: 30% ethyl acetate in n-hexane, ethyl acetate was added 0.5% Et₃N) to give an off-white solid (0.48 g, 74.5%). ¹H NMR (300 MHz, CDCl₃) δ: 7.26-7.29 (1 H, d, *J* = 9.0 Hz), 6.50-6.53 (1 H, d, *J* = 9.0 Hz), 5.55 (1 H, s), 4.17-4.24 (1 H, m), 2.40-2.46 (2 H, m), 1.89-1.98 (2 H, m), 1.77-1.85 (2 H, m), 0.28(9 H, s).

### Step 3. N-Cyclobutyl-6-ethynylpyridazin-3-amine

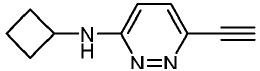

Into a solution of N-cyclobutyl-6-(2-(trimethylsilyl)ethynyl)pyridazin-3-amine (0.49 g, 2 mmol) in CH₂Cl₂, TBAF was added. The reaction was monitored by TLC. The reaction mixture was concentrated and the residue obtained was purified by silica gel chromatography (eluent: 30% ethyl acetate in n-hexane, ethyl acetate was added 0.5% Et₃N) to give an off-white solid (0.26 g, 75.1%).¹H NMR (300 MHz, CDCl₃) δ: 7.26-7.29 (1 H, d, *J* = 9.0 Hz), 6.51-6.54 (1 H, d, *J* = 9.0 Hz), 5.57 (1 H, s), 4.17-4.24 (1 H, m), 3.21 (1 H, s), 2.40-2.46 (2 H, m), 1.89-1.98 (2 H, m), 1.77-1.85 (2 H, m).

### Step 4. (3,4-Dinitrophenyl)-(4-methylpiperazin-1-yl)-methanone

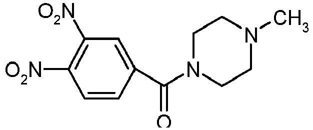

A mixture of 3,4-dinitrobenzoic acid (10.6 g, 50 mmol) and SOCl₂ (50 mL) was heated at reflux for 6 hrs. Then the mixture was evaporated to dryness in vacuo. The residue was dissolved in CH₂Cl₂ (50mL) and cooled to 5°C. To this solution, N-methylpiperazine (5.5 g, 55 mmol) and Et₃N (5.5 g, 55 mmol) were added dropwise as a solution in CH₂Cl₂ (50 mL). After stirring overnight at rt, the combined organic phase was washed with water (100 mL), dried over Na₂SO₄, filtered and concentrated, the resulting residue was purified by silica gel chromatography (eluent: 5% MeOH in CH₂Cl₂) to give a yellow solid (14.0 g, 95.2%). ¹H NMR (300 MHz, DMSO-d₆) δ: 8.29 (1 H, s), 8.25-8.27 (1 H, d, *J =* 9.0 Hz), 7.95-7.98 (1 H, d, *J =* 9.0 Hz), 3.62 (2 H, m), 3.28 (2 H, m), 2.38 (2 H, m), 2.26 (2 H, m), 2.19 (3 H, s).

### Step 5. 1-(3,4-Dinitrobenzyl)-4-methylpiperazine

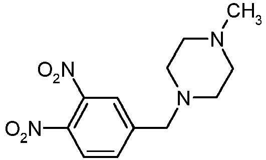

To a cooled solution (-5°C) of (3,4-dinitrophenyl)-(4-methylpiperazin-1-yl)methanone (5.88 g, 20 mmol) in THF, was added powdered NaBH4 (1.89 g, 50 mmol), followed by the dropwise addition of BF₃.OEt₂ (6.4 mL, 50 mmol), while keeping the temperature below 5°C. The mixture was allowed to come to room temperature over 2 hrs, and then stirred for a further 3 hrs at room temperature. MeOH was then added cautiously to the mixture, the stirring was continued for 10 minutes and then the mixture was concentrated. The residue was partitioned between EtOAc (150 mL) and saturated aqueous NaHCO₃ (150 mL). The organic layer was washed with water (100 mL), brine (100 mL) and then dried by Na₂SO₄, filtered and concentrated. The residue was purified by silica gel chromatography (eluent: 2% MeOH in CH₂Cl₂) to give a yellow solid (4.5 g, 80.3%). ¹H NMR (300 MHz, CDCl₃) δ: 7.90-7.92 (1 H, d, *J =* 6.0 Hz), 7.90 (1 H, s), 7.69-7.71 (1 H, d, *J =* 6.0 Hz), 3.73 (2 H, s), 3.07-3.10 (2 H, m), 2.94-2.99 (2 H, m), 2.76-2.81 (2 H, m), 2.67 (3 H, s), 2.53-2.56 (2 H, m).

### Step 6. 1-(3,4-Diaminobenzyl)-4-methylpiperazine

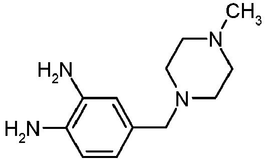

1-(3,4-Dinitrobenzyl)-4-methylpiperazine (2.8 g, 10 mmol), was dissolved in DMF:MeOH (1:1, 20 mL) and agitated with 10% Pd/C (280 mg) under an atmosphere of H₂ for 12 hrs. The reaction was monitored by TLC. The mixture was then filtered and evaporated to give a dark solid which was used immediately without any further purification.

### Step7. 2-(3-Iodo-4-methylphenyl)-6-((4-methylpiperazin-1-yl)methyl)-1H-benzo[d]imidazole

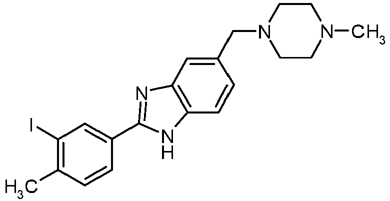

A mixture of 1-(3,4-diaminobenzyl)-4-methylpiperazine (10 mmol), 3-iodo-4- methylbenzoic acid (2.6 g, 10 mmol), EDCI (11 mmol), and HOBt (11 mmol) in dry DMF (25 mL) was stirred at ambient temperature for 24 hrs. The mixture was then evaporated in vacuo and the crude residue was dissolved in CH₂Cl₂ (100 mL) was washed with water (100 mL), brine (100 mL) and then dried by Na₂SO₄, filtered and concentrated. The residue obtained was dissolved in AcOH (30 mL) and heated at reflux for 3 hrs. The reaction mixture was eavporated in vacuo and the residue was purified by silica gel chromatography (eluent: 10% MeOH in CH₂Cl₂, MeOH was added 0.5% Et₃N) to give a yellow solid (1.35g, 30.3%). ¹H NMR (300 MHz, CDCl₃) δ: 8.50 (1 H, s), 7.94-7.97 (1 H, *d, J=* 9.0 Hz), 7.53-7.56 (1 H, d, *J* = 9.0 Hz), 7.52-7.55 (1 H, d, *J* = 9.0 Hz), 7.30 (1 H, s), 7.20-7.23 (1 H, d, *J* = 9.0 Hz), 3.60 (2 H, s), 2.54 (8 H, brs), 2.45 (3 H, s), 2.32 (3 H, s).

### Step 8.

### N-Cyclobutyl-6-(2-(2-methyl-5-(6-((4-methylpiperazin-1-yl)methyl)-1H-benzo[d]imidazol-2-yl)phenyl)ethynyl)pyridazin-3-amine

N-Cyclobutyl-6-ethynylpyridazin-3-amine (62 mg, 0.36 mmol), 2-(3-iodo-4-methylphenyl)-6-((4-methylpiperazin-1-yl)methyl)-1H-benzo[d]imidazole (134 mg, 0.3 mmol), Pd(PPh₃)₄ (18 mg, 0.015 mmol) and CuI (4.3 mg, 0.023 mmol) were placed in a two neck flask with a rubber plug. The mixture underwent 3 cycles of vacuum and filling with Ar₂, a solution of DIPEA (58 mg, 0.45 mmol) and DMF (2 mL) was injected to the flask. The mixture was stirred at rt for 20 hrs, and then was poured into 25 mL water, extracted with CH₂Cl₂ (20 mL×3), organic layer was washed with brine, dried with Na₂SO₄, filtered, and the filtrate was evaporated in vacuo. The residue was purified by chromatography on silica gel (CH₂Cl₂/CH₃OH 97:3 to 97:6) to give 0.12 g crude product, continue to purify via preparation TLC (CH₂Cl₂/CH₃OH 120:8) to give 87 mg product as a pale yellow solid. Mp:148-150°C; ¹H NMR (300 MHz, CDCl₃) *δ:* 8.07-8.10 (1 H, d, *J=* 9.0 Hz), 7.90 (1 H, s), 7.63-7.66 (1 H, d, *J =* 9.0 Hz), 7.63 (1 H, s), 7.26-7.24 (1 H, d, *J =* 6.0 Hz), 7.20 (1 H, m), 7.15 (1 H, m), 6.60-6.63 (1 H, d, *J =* 9.0 Hz), 5.63 (1 H, s), 4.26-4.33 (1 H, m), 3.62 (2 H, s), 2.58 (8 H, brs), 2.35 (3 H, s), 2.31 (3 H, s), 1.94-2.03 (2 H, m), 1.82-1.85 (2 H, m). HRMS (ESI-TOF⁺): m/z [M+H]⁺ calcd for C₃₀H₃₄N₇: 492.2870; found: 492.2856.

### Example 2

### N-Cyclopropyl-6-(2-(2-methyl-5-(6-((4-methylpiperazin-1-yl)methyl)-1H-benzo[d]imidazol-2-yl)phenyl)ethynyl)pyridazin-3-amine

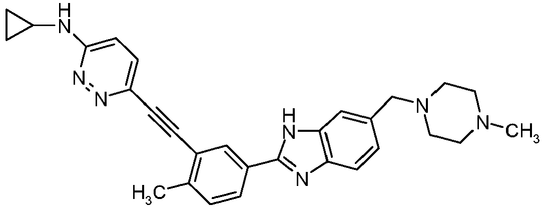

The title compound was synthesized from N-cyclopropyl-6-ethynylpyridazin-3-amine and 2-(3-iodo-4-methylphenyl)-6-((4-methylpiperazin-1-yl)methyl)-1H-benzo[d]imidazole (as prepared in Example 1) in a manner similar to that described for in Example 1. The intermediate compound N-cyclopropyl-6-ethynylpyridazin-3-amine was made as for Example 1 (Step 1 to 3) with the spectra below: ¹H NMR (300 MHz, CDCl₃) δ: 7.38-7.41 (1 H, d, *J* = 9.0 Hz), 6.96-6.99 (1 H, d, *J =* 9.0 Hz), 5.99 (1 H, s), 3.25-3.27 (1 H, m), 2.57 (1 H, s), 0.87-0.89 (2 H, s), 0.61-0.63 (2 H, s).

The title compound was obtained as as a khaki solid. Mp:145-146°C; ¹H NMR (300 MHz, CDCl₃) *δ:* 8.14 (1 H, s), 8.11-8.13 (1 H, d, *J =* 6.0 Hz), 7.64 (2 H, m), 7.37-7.40 (1 H, d, *J* = 9.0 Hz), 7.21-7.24 (1 H, d, *J* = 9.0 Hz), 7.14-7.17 (1 H, d, *J =* 7.0 Hz), 7.01-7.04 (1 H, d, *J =* 9.0 Hz), 6.19 (1 H, s), 3.67 (2 H, s), 2.74 (8 H, brs), 2.58 (1 H, m), 2.50 (3 H, s), 2.34 (3 H, s), 0.84-0.87 (2 H, m), 0.64 (2 H, m); HRMS (ESI-TOF⁺): m/z [M+H]⁺ calcd for C₂₉H₃₂N₇: 478.2714; found: 478.2708.

### Example 3

### 3-(2-(2-Methyl-5-(6-((4-methylpiperazin-1-yl)methyl)-1H-benzo[d]imidazol-2-yl)phenyl)ethynyl)imidazo[1,2-b]pyridazine

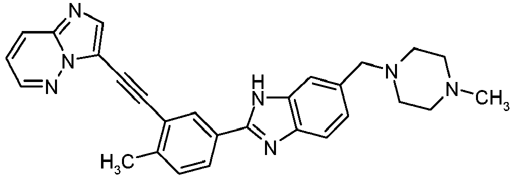

The title compound was synthesized from 3-ethynylimidazo[1,2-b]pyridazine and 2-(3-iodo-4-methylphenyl)-6-((4-methylpiperazin-1-yl)methyl)-1H-benzo[d]imidazole (as prepared in **Example 1**) in a manner similar to that described for in **Example 1.** The intermediate compound 3-ethynylimidazo[1,2-b]pyridazin was made as for **Example 1 (Step 1** to **3)** with the spectra below: ¹H NMR (300 MHz, DMSO-*d*6) δ: 8.63-8.64 (1 H, m), 8.18-8.21 (1 H, m), 8.10 (1 H, s), 7.32-7.35 (1 H, m), 4.94 (1 H, s).

The title compound was obtained as a pale yellow solid. Mp:114-115°C; ¹H NMR (300 MHz, CDCl₃) *δ:* 8.46-8.48 (1 H, d, *J* = 6.0 Hz), 8.25 (1 H, s), 8.15 (1 H, s), 8.09-8.12 (1 H, d, *J =* 9.0 Hz), 7.96-7.99 (1 H, d, *J =* 9.0 Hz), 7.59 (1 H, s), 7.35-7.37 (1 H, d, *J =* 9.0 Hz), 7.26 (1 H, s), 7.22 (1 H, s), 7.10-7.15 (1 H, m), 3.64 (2 H, s), 2.60 (8 H, brs), 2.58 (3 H, s), 2.34 (3 H, s). HRMS (ESI-TOF⁺): m/z [M+H]⁺ calcd for C₂₈H₂₈N₇: 462.2401; found: 462.2413.

### Example 4

### N-Isopropyl-6-(2-(2-methyl-5-(6-((4-methylpiperazin-1-yl)methyl)-1H-benzo[d]imidazol-2-yl)phenyl)ethynyl)pyridazin-3-amine

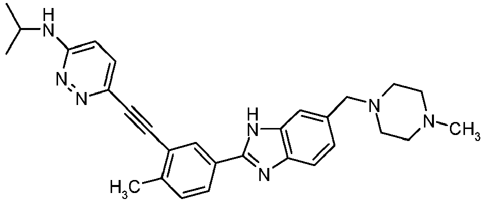

The title compound was synthesized from N-isopropyl-6-ethynylpyridazin-3-amine and 2-(3-iodo-4-methylphenyl)-6-((4-methylpiperazin-1-yl)methyl)-1H-benzo [d] - imidazole (as prepared in **Example 1**) in a manner similar to that described for in **Example 1.** The intermediate compound N-isopropyl-6-ethynylpyridazin-3-amine was made as for **Example 1 (Step 1** to **3)** with the spectra below: ¹H NMR (300 MHz, CDCl₃) δ: 7.26-7.29 (1 H, d, *J =* 9.0 Hz), 6.54-6.57 (1 H, d, *J =* 9.0 Hz), 5.00 (1 H, s), 3.97-4.04 (1 H, m), 3.25 (1 H, s), 1.29 (3 H, s), 1.27 (3 H, s).

The title compound was obtained as a pale yellow solid. Mp:129-130°C; ¹H NMR (300 MHz, CDCl₃) *δ:* 8.06-8.08 (1 H, d, *J =* 6.0 Hz), 7.87 (1 H, s), 7.65 (2 H, m), 7.27 (1 H, s), 7.25 (1 H, s), 7.19-7.21 (1 H, d, *J =* 9.0 Hz), 6.61-6.64 (1 H, d, *J* = 9.0 Hz), 5.01-5.04 (1 H, d*, J =* 9.0 Hz), 4.11-4.15 (1 H, m), 3.61 (2 H, s), 2.43 (8 H, brs), 2.32 (3 H, s), 2.27 (3 H, s), 1.25-1.32 (6 H, m). HRMS (ESI-TOF⁺): m/z [M+H]⁺ calcd for C₂₉H₃₄N₇: 480.2870; found: 480.2847.

### Example 5

### N-Cyclopropyl-5-(2-(2-methyl-5-(6-((4-methylpiperazin-1-yl)methyl)-1H-benzo[d]imidazol-2-yl)phenyl)ethynyl)pyrimidin-2-amine

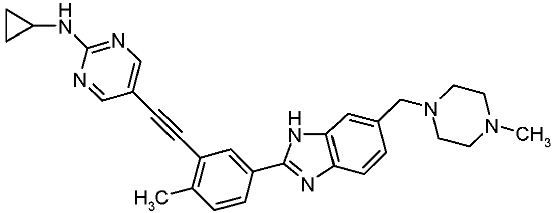

### Step 1. 5-Bromo-N-cyclopropylpyrimidin-2-amine

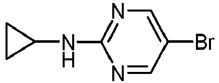

A solution of 5-bromo-2-chloropyrimidine (3.87 g, 20 mmol) and cyclopropylamine (5.7 g, 0.1 mol) in 20 mL THF was heated at 65°C for 5 hrs in a sealed tube. The mixture was evaporated in vacuo, to the residue ethanol was added, after filtration, the cake was washed with ethanol to give 4.07 g product as a colorless solid (95.5%). ¹H NMR (300 MHz, CDCl₃) δ: 8.32 (2 H, s), 5.58 (1 H, brs), 2.72 (1 H, brs), 0.82-0.84 (2 H, m), 0.54 (2 H, brs). LCMS: m/z [M+H]⁺ 214.0011.

### Step 2. N-Cyclopropyl-5-ethynylpyrimidin-2-amine

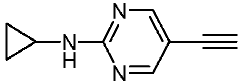

5-Bromo-N-cyclopropylpyrimidin-2-amine (1.06 g, 5 mmol), trimethylsilylacetylene (2.5 g, 25 mmol), Pd(PPh₃)₄ (289 mg, 0.25 mmol) and CuI (71 mg, 0.375 mmol) were placed in a two neck flask with a rubber plug. The mixture underwent 3 cycles of vacuum and filling with Ar₂, a solution of DIPEA (968 mg, 0.45 mmol) and DMF (10 mL) was injected to the flask. The mixture was stirred at 80°C for 15 hrs, and then was poured into 50 mL water, extracted with EtOAc (30 mL×3), organic layer was washed with brine, dried with Na₂SO₄, filtered, and the filtrate was evaporated in vacuo. The residue was purified by chromatography on silica gel (PE/ EtOAc 82:18 to 64:36) to give 1.1g N-cyclopropyl-5-(2-(trimethylsilyl)ethynyl)pyrimidin-2-amine. This compound was dissolved in 20 mL CH₂Cl₂, a solution of TBAF (1.3 g, 5 mmol) in 10 mL CH₂Cl₂ was added into the above solution. The mixture was stirred at rt for 1 h, evaporated in vacuo. The residue was purified by chromatography on silica gel (PE/ EtOAc 82:18 to 64:36) to give 0.55 g product as a pale yellow solid (72.8%). ¹H NMR (300 MHz, CDCl₃) δ: 8.43 (2 H, s), 5.77 (1 H, brs), 3.18 (1 H, s), 2.76-2.81 (1 H, m), 0.82-0.87 (2 H, m), 0.54-0.59 (2 H, m). LCMS: m/z [M+H]⁺ 160.0863.

### Step 3.

### N-Cyclopropyl-5-(2-(2-methyl-5-(6-((4-methylpiperazin-1-yl)methyl)-1H-benzo[d]imidazol-2-yl)phenyl)ethynyl)pyrimidin-2-amine

The title compound was synthesized from N-cyclopropyl-5-ethynylpyrimidin-2-amine (as prepared above) and 2-(3-iodo-4-methylphenyl)-6-((4-methylpiperazin-1-yl)methyl)-1H-benzo[d]imidazole (as prepared in **Example 1**) in a manner similar to that described for in **Example 1.** The title compound was obtained as a brown solid. Mp: 136-137°C; ¹H NMR (300 MHz, CDCl₃) *δ*: 8.40 (2 H, s), 8.21 (1 H, s), 7.96-7.99 (1 H, d, *J* = 9.0 Hz), 7.52-7.55 (1 H, d, *J* = 9.0 Hz), 7.49-7.52 (1 H, d, *J* = 9.0 Hz), 7.23-7.26 (1 H, d, *J* = 9.0 Hz), 7.15-7.18 (1 H, d, *J* = 9.0 Hz), 5.81 (1 H, s), 5.28 (1 H, s), 3.57 (2 H, s), 2.78 (1 H, s), 2.55 (8 H, brs), 2.46 (3 H, s), 2.32 (3 H, s), 0.83-0.87 (2 H, m), 0.56 (2 H, brs). HRMS (ESI-TOF⁺): m/z [M+H]⁺ calcd for C₂₉H₃₂N₇: 478.2714; found: 478.2718.

### Example 6

### 3-(2-(2-Chloro-5-(6-((4-methylpiperazin-1-yl)methyl)-1H-benzo[d]imidazol-2-yl) phenyl)ethynyl)imidazo[1,2-b]pyridazine

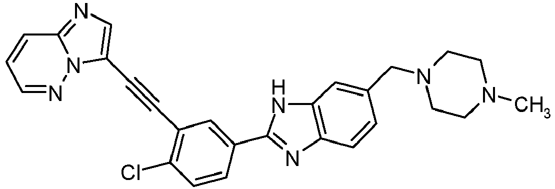

The title compound was synthesized from 3-ethynylimidazo[1,2-b]pyridazine (as prepared in **Example 3**) and 2-(4-chloro-3-iodophenyl)-6-((4-methylpiperazin-1-yl)methyl)-1H-benzoimidazole in a manner similar to that described for in **Example 1.** The intermediate compound 2-(4-chloro-3-iodophenyl)-6-((4-methylpiperazin-1-yl)methyl)-1H-benzo[d] imidazole was made as for **Example 1 (Step 4** to **7)** with the spectra below: ¹H NMR (300 MHz, CDCl₃) δ: 8.52 (1 H, s), 7.95-7.98 (1 H, d, *J* = 9.0 Hz), 7.45-7.48 (1 H, d, *J* = 9.0 Hz), 7.43-7.46 (1 H, d, *J* = 9.0 Hz), 7.23 (1 H, s), 7.21-7.24 (1 H, d, *J* = 9.0 Hz), 3.59 (2 H, s), 2.51 (8 H, brs), 2.29 (3 H, s).

The title compound was obtained as a khaki solid. Mp: 156-157°C; ¹H NMR (300 MHz, CDCl₃) *δ*: 8.43 (1 H, s), 8.36 (1 H, s), 8.11-8.14 (1 H, d, *J* = 9.0 Hz), 8.11 (1 H, s), 7.92-7.95 (1 H, d, *J* = 9.0 Hz), 7.54-7.57 (2 H, m), 7.45-7.45 (1 H, d, *J* = 9.0 Hz), 7.18-7.21 (1 H, d, *J* = 9.0 Hz), 7.10-7.11 (1 H, m), 3.60 (2 H, s), 2.61 (8 H, brs), 2.31 (3 H, s). HRMS (ESI-TOF⁺): m/z [M+H]⁺ calcd for C₂₇H₂₅ClN₇: 482.1854; found: 482.1841.

### Example 7

### 6-(2-(2-Chloro-5-(6-((4-methylpiperazin-1-yl)methyl)-1H-benzo[d]imidazol-2-yl)phenyl)ethynyl)-N-cyclopropylpyridazin-3-amine

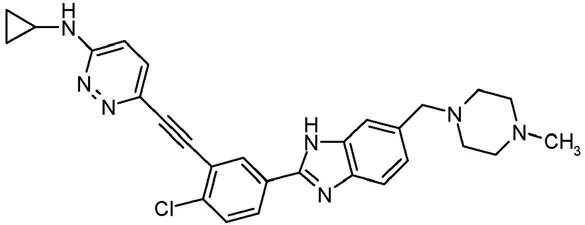

The title compound was synthesized from N-cyclopropyl-6-ethynylpyridazin-3-amine and 2-(4-chloro-3-iodophenyl)-6-((4-methylpiperazin-1-yl)methyl)-1H-benzo[d]imidazole in a manner similar to that described for in **Example 1.** The title compound was obtained as a khaki solid. Mp: 130-131°C; ¹H NMR (300 MHz, CDCl₃) *δ*: 8.11-8.13 (1 H, d, *J* = 6.0 Hz), 7.86 (1 H, s), 7.64 (2 H, m), 7.42-7.45 (1 H, d, *J* = 9.0 Hz), 7.39-7.42 (1 H, d, *J* = 9.0 Hz), 7.22 (1 H, s), 7.01-7.04 (1 H, d, *J* = 9.0 Hz), 6.00 (1 H, s), 3.61 (2 H, s), 2.60 (1 H, m), 2.44 (8 H, brs), 2.27 (3 H, s), 0.87-0.89 (2 H, m), 0.65 (2 H, m). HRMS (ESI-TOF⁺): m/z [M+H]⁺ calcd for C₂₈H₂₉ClN₇: 498.2167; found: 498.2150.

### Example 8

### 6-(2-(2-Chloro-5-(6-((4-methylpiperazin-1-yl)methy|)-1H-benzo[d]imidazol-2-yl)phenyl)ethynyl)-N-cyclobutylpyridazin-3-amine

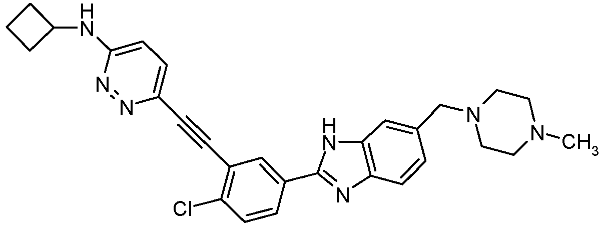

The title compound was synthesized from N-cyclobutyl-6-ethynylpyridazin-3-amine and 2-(4-chloro-3-iodophenyl)-6-((4-methylpiperazin-1-yl)methyl)-1H-benzo[d]imidazole in a manner similar to that described for in **Example 1.** The title compound was obtained as a pale yellow solid. Mp:161-163°C; ¹H NMR (300 MHz, CDCl₃) *δ*: 8.13-8.15 (1 H, d, *J* = 6.0 Hz), 7.95 (1 H, s), 7.62-7.65 (1 H, d, *J* = 9.0 Hz), 7.65 (1 H, s), 7.36-7.39 (1 H, d, *J* = 9.0 Hz), 7.29-7.32 (1 H, d, *J* = 9.0 Hz), 7.18-7.21 (1 H, d, *J* = 9.0 Hz), 6.63-6.66 (1 H, d, *J* = 9.0 Hz), 5.80 (1 H, s), 4.28-4.30 (1 H, m), 3.63 (2 H, s), 2.63 (8 H, brs), 2.40-2.44 (2 H, m)2.40 (3 H, s), 1.96-2.02 (2 H, m), 1.79-1.81 (2 H, m). HRMS (ESI-TOF⁺): m/z [M+H]⁺ calcd for C₂₉H₃₁ClN₇: 512.2324; found: 512.2303.

### Example 9

### 6-(2-(2-Chloro-5-(6-((4-methylpiperazin-1-yl)methyl)-1H-benzo[d]imidazol-2-yl)phenyl)ethynyl)-N-isopropylpyridazin-3-amine

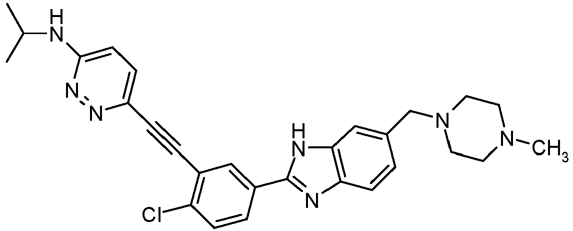

The title compound was synthesized from N-isopropyl-6-ethynylpyridazin-3-amine and 2-(4-chloro-3-iodophenyl)-6-((4-methylpiperazin-1-yl)methyl)-1H-benzo[d]imidazole in a manner similar to that described for in **Example 1.** The title compound was obtained as a khaki solid. Mp:127-128°C; ¹H NMR (300 MHz, CDCl₃) *δ*: 8.11-8.13 (1 H, d, *J* = 6.0 Hz), 7.82 (1 H, s), 7.64 (2 H, m), 7.53 (1 H, s), 7.35-7.38 (1 H, d, *J* = 9.0 Hz), 7.29-7.32 (1 H, d, *J* = 9.0 Hz), 6.66-6.69 (1 H, d, *J* = 9.0 Hz), 5.22 (1 H, s), 4.12-4.14 (1 H, m), 3.62 (2 H, s), 2.51 (8 H, brs), 2.31 (3 H, s), 1.25-1.31 (6 H, m). HRMS (ESI-TOF⁺): m/z [M+H]⁺ calcd for C₂₈H₃₁ClN₇: 500.2324; found: 500.2313.

### Example 10

### 5-(2-(2-Chloro-5-(6-((4-methylpiperazin-1-yl)methyl)-1H-benzo[d]imidazol-2-yl)phenyl)ethynyl)-N-cyclopropylpyrimidin-2-amine

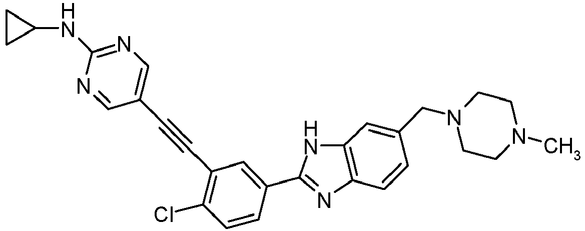

The title compound was synthesized from N-cyclopropyl-5-ethynylpyrimidin-2-amine and 2-(4-chloro-3-iodophenyl)-6-((4-methylpiperazin-1-yl)methyl)-1H-benzo[d]imidazole in a manner similar to that described for in **Example 1.** The title compound was obtained as a brown solid. Mp:160-162°C; ¹H NMR (300 MHz, CDCl₃) *δ*: 8.43-8.45 (2 H, d, *J* = 6.0 Hz), 8.12-8.15 (1 H, d, *J* = 6.0 Hz), 7.58-7.60 (1 H, d, *J* = 6.0 Hz), 7.52 (1 H, s), 7.45-7.47 (1 H, d, *J* = 6.0 Hz), 7.38-7.40 (1 H, d, *J* = 6.0 Hz), 7.15-7.17 (1 H, d, *J* = 6.0 Hz), 5.80 (1 H, s), 3.60 (2 H, s), 2.80 (8 H, brs), 2.59 (3 H, s), 1.35-1.39 (1 H, m), 0.82-0.88(2 H, m), 0.58-0.60 (2 H, m). HRMS (ESI-TOF⁺): m/z [M+H]⁺ calcd for C₂₈H₂₉ClN₇: 498.2167; found: 498.2163.

### Example 11

### 3-(2-(2-Methyl-5-(6-(4-methyl-1H-imidazol-1-yl)-1H-benzo[d]imidazol-2-yl)phenyl)ethynyl)imidazo[1,2-b]pyridazine

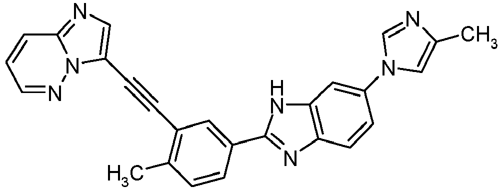

### Step 1. 4-(4-Methyl-1H-imidazol-1-yl)-2-nitroaniline

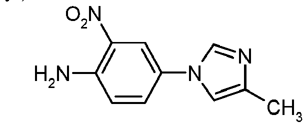

A suspension of 4-bromo-2-nitroaniline (4.34 g, 20 mmol), 4-methylimidazole (1.97 g, 24 mmol), K₂CO₃ (3.04 g, 22mmol), CuI (0.57 g, 3 mmol) and 8-hydroxyquinoline (0.44 g, 3 mmol) in 20 mL DMSO was stirred at 120°C in a sealed tube under Ar₂ for 29 hrs. The mixture was cooled down to rt and 28% aqueous ammonia (10 mL) was added and then H₂O and EtOAc were added. The aqueous layer was extracted with EtOAc (80 mL×3) and the organic layer was washed with brine, dried with Na₂SO₄, after filtration, the filtrate was evaporated in vacuo and the residue was washed with PE/EtOAc to give 2.47 g product as a red solid (56.6%). ¹H NMR (300 MHz, DMSO-*d*₆) δ: 8.03 (1 H, d, *J* = 2.1 Hz), 7.64-7.68 (1 H, dd, *J* = 2.1 and 9.0 Hz), 7.53 (2 H, brs), 7.10-7.13 (1 H, d, *J* = 9.0 Hz), 2.12 (3 H, s). LCMS: m/z [M+H]⁺ 219.0895.

### Step 2. 2-Amino-4-(4-methyl-1H-imidazol-1-yl)aniline

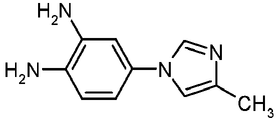

4-(4-Methyl-1H-imidazol-1-yl)-2-nitroaniline (0.22 g, 1 mmol) was suspended in 20 mL of anhydrous methanol. The mixture was reduced with 0.11 g of Raney Ni at 40 psi for 7 hrs. Then the Raney Ni was removed by filtration. The filtrate was evaporated to give the title compound 0.18 g as a yellow solid (95.7%).

### Step 3. N-(2-Amino-4-(4-methyl-1H-imidazol-1-yl)phenyl)-3-iodo-4-methylbenzamide

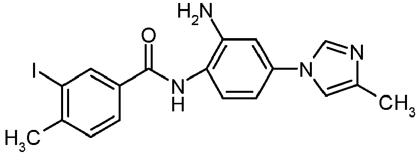

A solution of 3-iodo-4-methylbenzoic acid (0.26 g, 1 mmol) in SOCl₂ (5 mL) was refluxed for 2 h, and then evaporated in vacuo to remove excess SOCl₂. The residue was dissolved in 5 mL anhydrous THF and added to a solution of triethylamine (0.12 g, 1.2 mmol), 2-amino-4-(4-methyl-1H-imidazol-1-yl)aniline (0.18 g, 1 mmol) and DMAP (24 mg) in 5 mL anhydrous THF in dropwise. The result mixture was stirred at rt for 20 hrs, and evaporated in vacuo. The residue was purified by chromatography on silica gel (CH₂Cl₂/CH₃OH 97:3) to give 0.16 g product as a pale yellow solid (37.0%). LC/MS: m/z [M+H]⁺ 433.0520.

### Step4. 2-(3-Iodo-4-methylphenyl)-6-(4-methyl-1H-imidazol-1-yl)-1H-benzo[d] imidazole

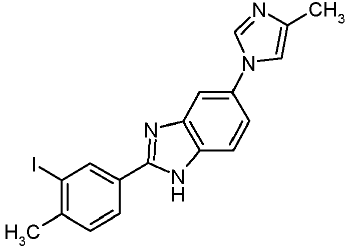

A solution of N-(2-amino-4-(4-methyl-1H-imidazol-1-yl)phenyl)-3-iodo-4-methylbenzamide (0.16 g, 0.37 mmol) in 5 mL glacial acetic acid was refluxed for 8 hrs, and then the mixture was evaporated in vacuo, the residue was purified by chromatography on silica gel (CH₂Cl₂/CH₃OH 97:3 to 94:6) to give 0.1 g product as pale yellow solid (65.3%). LC/MS: m/z [M+H]⁺ 415.0415.

### Step 5.

### 3-(2-(2-Methyl-5-(5-(4-methyl-1H-imidazol-1-yl)-1H-benzo[d]imidazol-2-yl)phenyl)ethynyl)imidazo[1,2-b]pyridazine

The title compound was synthesized from 3-ethynylimidazo[1,2-b]pyridazine and 2-(3-iodo-4-methylphenyl)-6-(4-methyl-1H-imidazol-1-yl)-1H-benzo[d]imidazole (as prepared above) in a manner similar to that described for in **Example 1.** The title compound was obtained a pale yellow solid. Mp: 182-184°C; ¹H NMR (400 MHz, CD₃OD) *δ*: 8.57 (2 H, brs), 8.26 (1 H, s), 7.98-8.05 (3 H, m), 7.74 (1 H, s), 7.68-7.70 (1 H, d, *J =* 8.4 Hz), 7.46-7.48 (2 H, m), 7.42-7.44 (1 H, d, *J* = 8.4 Hz), 7.29-7.33 (1 H, dd, *J* = 4.4 and 9.2 Hz), 2.61 (3 H, s), 2.31 (3 H, s). HRMS (ESI-TOF⁺): m/z [M+H]⁺ calcd for C₂₆H₂₀N₇: 430.1775; found: 430.1778.

### Example 12

### 3-(2-(6-(Cyclopropylamino)pyridazin-3-yl)ethynyl)-4-methyl-N-(4-((4-methylpiperazin-1-yl)methyl)-3-(trifluoromethyl)phenyl)benzamide

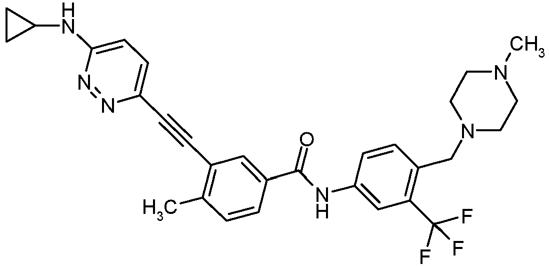

### Step 1. 1-(Bromomethyl)-2-(trifluoromethyl)-4-nitrobenzene

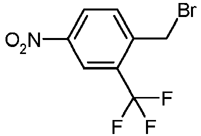

Into a solution of 1-methyl-4-nitro-2-trifluormethylbenzene (4.1 g, 20 mmol) in carbon tetrachloride (30 mL) were added NBS (5.4 g, 30 mmol) and AIBN (493 mg, 3 mmol). The reaction was refluxed overnight and then partitioned with water. The organic layer was separated and the aqueous layer was extracted with CH₂Cl₂. The combined organic extracts were washed with water, dried over Na₂SO₄, filtered and concentrated to afford the material that was not purified but used directly in the next step.

### Step 2. 4-Methyl-1-(4-nitro-2-(trifluoromethyl)benzyl)piperazine

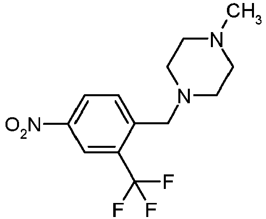

To a solution of crude 1-(bromomethyl)-2-(trifluoromethyl)-4-nitrobenzene (13.7 mmol, 60% pure) in CH₂Cl₂ (20 mL) was added Et₃N (1.5 g, 15 mmol) and 4-methylpiperazine (1.5 g, 15 mmol). After stirring for 5 hrs at rt, 50mL H₂O was added, and the mixture was extracted with 50mL CH₂Cl₂. The combined organic layer was dried over Na₂SO₄, filtered, concentrated, and the resulting residue was purified by silica gel chromatography (eluent: 10% MeOH in CH₂Cl₂) to give the product (67.4%, 2.8 g). ¹H NMR (300 MHz, CDCl₃) δ: 8.52 (1 H, s), 8.37-8.40 (1 H, d, *J* = 9.0 Hz), 8.00-8.03 (1 H, d, *J* = 9.0 Hz), 3.85 (2 H, s), 3.09-3.15 (2 H, m), 2.90-2.93 (2 H, m), 2.80-2.83 (2 H, m), 2.67 (3 H, s), 2.53-2.59 (2 H, m).

### Step 3. 4-((4-Methylpiperazin-1-yl)methyl)-3-(trifluoromethyl)aniline

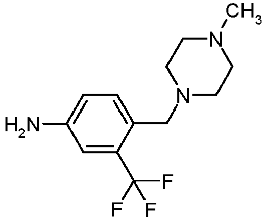

Into a solution 4-methyl-1-(4-nitro-2-(trifluoromethyl)benzyl)piperazine (1.5 g, 5 mmol) in MeOH (250 mL) was added Raney Nickel (0.15 g, 10 wt %). The suspension was stirred under hydrogen atmosphere (50 psi) for 24 hrs and monitored by TLC. The reaction mixture was filtered through celite and the filtrate was concentrated under reduced pressure to yield the desired product (1.36 g, 100%). ¹H NMR (300 MHz, CDCl₃) δ: 7.43-7.46 (1 H, d, *J* = 9.0 Hz), 6.91 (1 H, s), 6.77-6.80 (1 H, d *J* = 9.0 Hz), 3.77 (2 H, s), 3.54 (2 H, s), 2.53 (8 H, brs), 2.34 (3 H, s).

### Step4. 3-Iodo-4-methyl-N-(4-((4-methylpiperazin-1-yl)methyl)-3-(trifluoromethyl) phenyl)benzamide

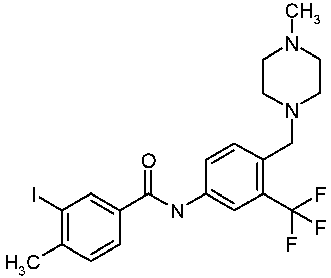

3-Iodo-4-methylbenzoyl chloride (1.06 g, 3.8 mmol), prepared from the reaction of 3-iodo-4-methylbenzoic acid and SOCl₂, was added to a solution of 4-((4-methylpiperazin-1-yl)methyl)-3-(trifluoromethyl)aniline (1.00 g, 3.6 mmol), Et₃N (0.36 g 3.6 mmol), and a catalytic amount of DMAP in THF (20mL). After stirring at rt for 2 hrs, the reaction was quenched with water. EtOAc was added and the layers separated. The combined organic layers were concentrated to dryness and purified by silica gel chromatography (eluent: 5% MeOH in CH₂Cl₂, MeOH was added 0.5% Et₃N) to provide the desired product as an off-white solid (67.2%, 1.25 g). ¹H NMR (300 MHz, CDCl₃) δ: 8.29 (1 H, s), 8.00 (1 H, s), 7.85 ((1 H, m), 7.73-7.76 ((2 H, m), 7.31-7.34 ((1 H, d, *J* = 9.0 Hz), 3.64 (2 H, s), 2.53 (8 H, brs), 2.49 (3 H, s), 2.33 (3 H, s).

### Step 5.

### 3-(2-(6-(Cyclopropylamino)pyridazin-3-yl)ethynyl)-4-methyl-N-(4-((4-methylpiperazin-1-yl)methyl)-3-(trifluoromethyl)phenyl)benzamide

The title compound was synthesized from N-cyclopropyl-6-ethynylpyridazin-3-amine and 3-iodo-4-methyl-N-(4-((4-methylpiperazin-1-yl)methyl)-3-(trifluoromethyl)phenyl) benzamide (as prepared above) in a manner similar to that described for in **Example 1.** The product was obtained as a pale yellow solid. Mp: 68-69°C; ¹H NMR (300 MHz, CDCl₃) *δ*: 8.84 (1 H, s), 7.99-8.01 (2 H, d, *J =* 6.0 Hz), 7.95-7.98 (1 H, d, *J* = 9.0 Hz), 7.82-7.85 (1 H, d, *J* = 9.0 Hz), 7.71-7.73 (1 H, d, *J* = 6.0 Hz), 7.40-7.43 (1 H, d, *J* = 9.0 Hz), 7.29-7.32 (1 H, d, *J* = 9.0 Hz), 6.99-7.02 (1 H, d, *J* = 9.0 Hz), 5.85 (1 H, s), 3.63 (2 H, s), 2.53 (8 H, brs), 2.34 (3 H, s), 2.03 (3 H, s), 1.43 (1 H, m), 0.86-0.88 (2 H, m), 0.61-0.63 (2 H, m). HRMS (ESI-TOF⁺): m/z [M+H]⁺ calcd for C₃₀H₃₂F₃N₆O: 549.2584; found: 549.2568.

### Example 13

### 3-(2-(6-(Cyclobutylamino)pyridazin-3-yl)ethynyl)-4-methyl-N-(4-((4-methylpiperazin-1-yl)methyl)-3-(trifluoromethyl)phenyl)benzamide

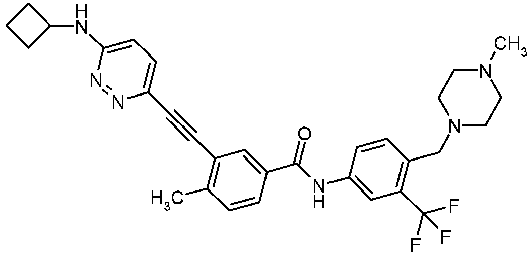

The title compound was synthesized from N-cyclobutyl-6-ethynylpyridazin-3-amine and 3-iodo-4-methyl-N-(4-((4-methylpiperazin-1-yl)methyl)-3-(trifluoromethyl)phenyl)benzamide in a manner similar to that described for in **Example 1.** The product was obtained as a pale yellow solid. Mp:120-121°C; ¹H NMR (300 MHz, CDCl₃) *δ*: 9.10 (1 H, s), 8.05 (1 H, s), 7.97-7.80 (1 H, d, *J* = 9.0 Hz), 7.94 (1 H, s), 7.81-7.83 (1 H, d, *J* = 6.0 Hz), 7.64-7.66 (1 H, d, *J* = 6.0 Hz), 7.28-7.31 (1 H, d, *J* = 9.0 Hz), 7.26-7.28 (1 H, d, *J* = 6.0 Hz), 6.57-5.59 (1 H, d, *J* = 2.0 Hz), 5.53 (1 H, s), 4.22-4.27 (1 H, m), 3.64 (2 H, s), 2.65 (8 H, brs), 2.49 (3 H, s), 2.45 (3 H, s), 2.44 (2H, m), 1.93-1.99 (2 H, m), 1.83-1.89 (2H, m). HRMS (ESI-TOF⁺): m/z [M+H]⁺ calcd for C₃₁H₃₄F₃N₆O: 563.2741; found: 563.2768.

### Example 14

### 3-(2-(6-(Isopropylamino)pyridazin-3-yl)ethynyl)-4-methyl-N-(4-((4-methylpiperazin-1-yl)methyl)-3-(trifluoromethyl)phenyl)benzamide

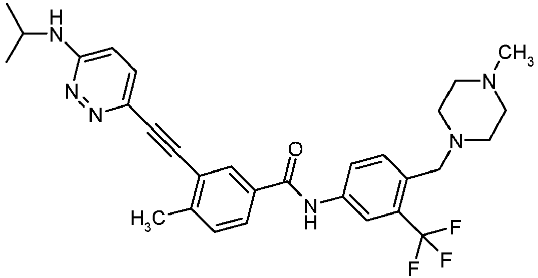

The title compound was synthesized from N-isopropyl-6-ethynylpyridazin-3-amine and 3-iodo-4-methyl-N-(4-((4-methylpiperazin-1-yl)methyl)-3-(trifluoromethyl)phenyl)benzamide in a manner similar to that described for in **Example 1.** The product was obtained as a pale yellow solid. Mp:67-68°C; ¹H NMR (300 MHz, CDCl₃) *δ*: 8.97 (1 H, s), 8.06 (1 H, s), 7.98-8.01 (1 H, d, *J* = 9.0 Hz), 7.97 (1 H, s), 7.83-7.85 (1 H, d, *J* = 6.0 Hz), 7.58-7.60 (1 H, d, *J* =6.0 Hz), 7.30-7.32 (2 H, m), 6.61-6.64 (1 H, d, J =9.0 Hz), 5.05 (1 H, s), 4.02-4.05 (1 H, m), 3.70 (2 H, s), 2.79-2.99 (8 H, brs), 2.66 (3 H, s), 2.52 (3 H, s), 1.29 (6 H, m). HRMS (ESI-TOF⁺): m/z [M+H]⁺ calcd for C₃₀H₃₄F₃N₆O: 551.2741; found: 551.2734.

### Example 15

### 4-Chloro-3-(2-(6-(cyclopropylamino)pyridazin-3-yl)ethynyl)-N-(4-((4-methylpiperazin-1-yl)methyl)-3-(trifluoromethyl)phenyl)benzamide

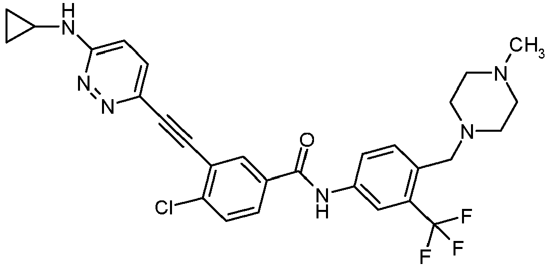

The title compound was synthesized from N-cyclopropyl-6-ethynylpyridazin-3-amine and 4-chloro-3-iodo-N-(4-((4-methylpiperazin-1-yl)methyl)-3-(trifluoromethyl)phenyl) benzamide in a manner similar to that described for in **Example 1.** The intermediate compound 4-chloro-3-iodo-N-(4-((4-methylpiperazin-1-yl)methyl)-3-(trifluoromethyl)phenyl)benzamide was made as for **Example 12 (Step 1** to **4)** with the spectra below: ¹H NMR (300 MHz, DMSO-*d*₆) *δ*: 10.66 (1 H, s), 8.53 (1 H, s), 8.19 (1 H, s), 8.02-8.05 (1 H, d, *J* = 6.0 Hz), 7.98-8.01 (1 H, d, *J* = 6.0 Hz), 7.77 (1 H, s), 7.70-7.73 (1 H, d, *J* = 6.0 Hz), 3.62 (2 H, s), 3.33 (2 H, m), 2.82 (2 H, m), 2.54 (4 H, m), 2.50 (3 H, s).

The title compound was obtained as a pale yellow solid. Mp:133-134°C; ¹H NMR (300 MHz, CDCl₃) *δ*: 9.57 (1 H, s), 8.05-8.08 (1 H, d, *J* = 9.0 Hz), 7.85-7.88 (1 H, *J* = 9.0 Hz), 7.67-7.70 (1 H, d, *J* = 9.0 Hz), 7.42-7.45 (2 H, m), 7.00-70.3 (1 H, d, *J* = 9.0 Hz), 6.00 (1 H, s), 3.61 (2 H, s), 2.53 (8 H, brs), 2.35 (3 H, s), 1.25 (1 H, s), 0.87 (2 H, m), 0.63 (2 H, m). HRMS (ESI-TOF⁺): m/z [M+H]⁺ calcd for C₂₉H₂₉ClF₃N₆O: 569.2038; found: 569.2000.

### Example 16

### 4-Chloro-3-(2-(6-(cyclobutylamino)pyridazin-3-yl)ethynyl)-N-(4-((4-methylpiperazin-1-yl)methyl)-3-(trifluoromethyl)phenyl)benzamide

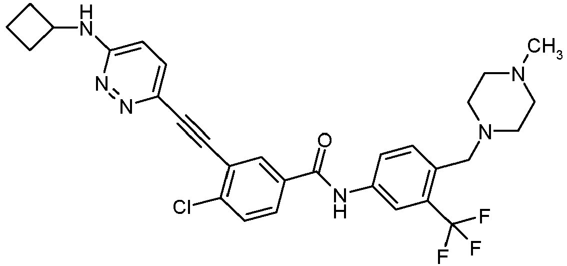

The title compound was synthesized from N-cyclobutyl-6-ethynylpyridazin-3-amine and 4-chloro-3-iodo-N-(4-((4-methylpiperazin-1-yl)methyl)-3-(trifluoromethyl)phenyl)benzamide in a manner similar to that described for in **Example 1.** The product was obtained as a pale yellow solid. Mp: 132-133°C; ¹H NMR (300 MHz, CDCl₃) *δ*: 9.53 (1 H, s), 8.10 (1 H, s), 8.00 (2 H, m), 7.85-7.87 (1 H, d, *J* = 6.0 Hz), 7.66-7.69 (1 H, d, *J* = 9.0 Hz), 7.43-7.45 (1 H, d, *J* = 6.0 Hz), 7.33-7.36 (1 H, d, *J* = 9.0 Hz), 6.58-6.61 (1 H, d, *J* = 9.0 Hz), 5.56 (1 H, s), 4.23-4.25 (1 H, m), 3.64 (2 H, s), 2.61 (8 H, brs),2.44 (3 H, s), 2.43 (2 H, m), 1.91-1.97 (2 H, m), 1.81-1.86 (1 H, brs). HRMS (ESI-TOF⁺): m/z [M+H]⁺ calcd for C₃₀H₃₁ClF₃N₆O: 583.2194; found: 583.2174.

### Example 17

### 4-Chloro-3-(2-(6-(isopropylamino)pyridazin-3-yl)ethynyl)-N-(4-((4-methylpiperazin-1-yl)methyl)-3-(trifluoromethyl)phenyl)benzamide

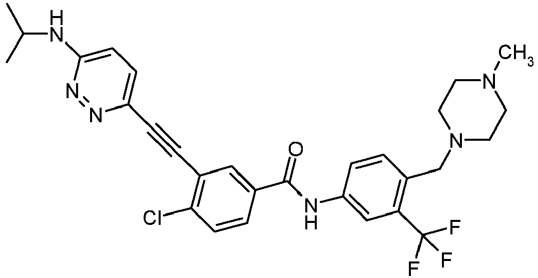

The title compound was synthesized from N-isopropyl-6-ethynylpyridazin-3-amine and 4-chloro-3-iodo-N-(4-((4-methylpiperazin-1-yl)methyl)-3-(trifluoromethyl)phenyl) benzamide in a manner similar to that described for in **Example 1.** The product was obtained as a pale yellow solid. Mp:133-134°C; ¹H NMR (300 MHz, CDCl₃) *δ*: 9.62 (1 H, s), 8.11 (1 H, s), 8.05 (1 H, s), 8.01-8.03 (1 H, d, *J* = 9.0 Hz), 7.86-7.88 (1 H, d, *J* = 6.0 Hz), 7.60-7.63 (1 H, d, *J* = 6.0 Hz), 7.44-7.47 (1 H, d, *J* = 9.0 Hz), 7.33-7.36 (1 H, d, *J* = 9.0 Hz), 6.63-6.66 (1 H, d, *J* = 9.0 Hz), 3.99-4.07 (1 H, m), 3.67 (2 H, s), 2.84 (8 H, brs), 2.56 (3 H, s), 1.25-1.30 (6 H, m). HRMS (ESI-TOF⁺): m/z [M+H]⁺ calcd for C₂₉H₃₀ClF₃N₆O: 571.2194; found: 571.2209.

### Example 18

### 3-(2-(6-(Cyclopropylamino)pyridazin-3-yl)ethynyl)-4-methyl-N-(4-(4-methyl-1H-imidazol-1-yl)-3-(trifluoromethyl)phenyl)benzamide

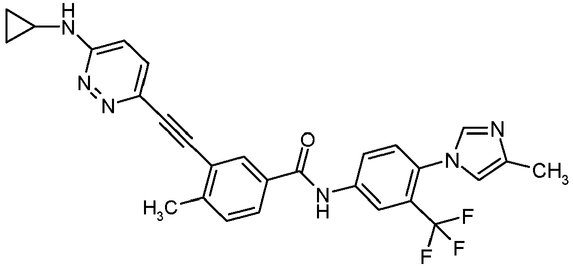

### Step 1. 4-Methyl-1-(2-(trifluoromethyl)-4-nitrophenyl)-1H-imidazole

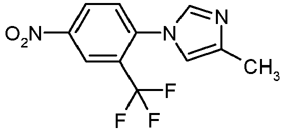

A mixture of 4-methylimidazole (0.63 g, 7.67 mmol), 1-fluoro-2-(trifluoromethyl)-4-nitrobenzene (1.06 g, 5.1 mmol), and K₂CO₃ (1.06 g, 7.67 mmol) in acetonitrile (20 mL) was refluxed for 6 hs. The mixture was filtered through Celite and filtrate was evaporated in vacuo and the residue was purified by chromatography on silica gel (PE/EtOAc 1:1) to give 1.13 g product as light green oil (81.8%). ¹H NMR (300 MHz, CDCl₃) δ: 8.71 (1 H, s), 8.52-8.55 (1 H, d, *J* = 8.4 Hz), 7.59-7.62 (2 H, m), 6.90 (1 H, s), 2.32 (3 H, s). LCMS: m/z [M+H]⁺ 272.0660.

### Step 2. 4-(4-Methyl-1H-imidazol-1-yl)-3-(trifluoromethyl)aniline

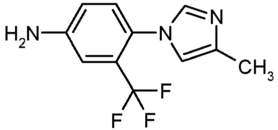

4-Methyl-1-(2-(trifluoromethyl)-4-nitrophenyl)-1H-imidazole (1.1 g, 4.1 mmol) was suspended in 50 mL of anhydrous ethanol. The mixture was hydrogenated with 0.11 g of 10% Pd-C at 40 psi for 3 hrs. Then Pd-C was removed by filtration. The filtrate was evaporated to give the title compound 1.0 g as a yellow solid (102.0%). ¹H NMR (300 MHz, CDCl₃) δ: 7.42 (1 H, s), 7.08-7.11 (1 H, d, *J* = 8.4 Hz), 6.99 (1 H, d, *J* = 2.4 Hz), 6.80-6.84 (1 H, dd, *J* = 2.1 and 8.4 Hz), 6.74 (1 H, s), 4.14 (2 H, brs), 2.27 (3 H, s). LCMS: m/z [M+H]⁺ 242.0957.

### Step 3. 3-Iodo-4-methyl-N-(4-(4-methyl-1H-imidazol-1-yl)-3-(trifluoromethyl)phenyl) benzamide

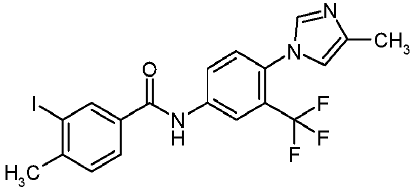

A solution of 3-iodo-4-methylbenzoic acid (0.26 g, 1 mmol) in SOCl₂ (5 mL) was refluxed for 3 hrs, then evaporated in vacuo to remove residual SOCl₂. The residue was dissolved in 5 mL anhydrous THF and added to a solution of triethylamine (0.12 g, 1 mmol), 4-(4-methyl-1H-imidazol-1-yl)-3-(trifluoromethyl)aniline (0.24 g, 1 mmol) and DMAP (12 mg) in 5 mL anhydrous THF in dropwise. The result mixture was stirred at rt for 40 hrs, and evaporated in vacuo. To the residue water was added and extracted with EtOAc (20 mL×3), the organic layer was washed with brine, dried with Na₂SO₄, after filtration, the filtrate was purified by chromatography on silica gel (CH₂Cl₂/CH₃OH 97:3) to give 0.4 g crude product. The crude product was triturated with PE/EtOAc to give the title compound 0.35 g as pale yellow solid (72.2%). ¹H NMR (300 MHz, CDCl₃) δ: 8.91 (1 H, s), 8.37 (1 H, s), 8.08-8.12 (2 H, m), 7.86-7.83 (1 H, d, *J* = 8.4 Hz), 7.54 (1 H, s), 7.34-7.36 (2 H, m), 6.84 (1 H, s), 2.50 (3 H, s), 2.29 (3 H, s). LCMS: m/z [M+H]⁺ 486.0493.

### Step 4.

### 3-(2-(6-(Cyclopropylamino)pyridazin-3-yl)ethynyl)-4-methyl-N-(4-(4-methyl-1H-imidazol-1-yl)-3-(trifluoromethyl)phenyl)benzamide

The title compound was synthesized from N-cyclopropyl-6-ethynylpyridazin-3-amine and 3-iodo-4-methyl-N-(4-(4-methyl-1H-imidazol-1-yl)-3-(trifluoromethyl)phenyl) benzamide (as prepared above) in a manner similar to that described for in **Example 1.** The product was obtained as a pale yellow solid. Mp:125-126°C; ¹H NMR (300 MHz, CDCl₃) δ: 9.94 (1 H, s), 8.34 (1 H, s), 8.20-8.23 (1 H, d, *J* = 8.7 Hz), 8.02 (1 H, s), 7.86-7.89 (1 H, d, *J* = 8.1 Hz), 7.53 (1 H, s), 7.39-7.42 (1 H, d, *J* = 9.0 Hz), 7.30-7.32 (2 H, m), 7.02-7.05 (1 H, d, *J* = 9.2 Hz), 6.80 (1 H, s), 6.02 (1 H, s), 2.58 (1 H, m), 2.48 (3 H, s), 2.29 (3 H, s), 0.87 (2 H, m), 0.64 (2 H, brs). HRMS (ESI-TOF⁺): m/z [M+2H]⁺² calcd for C₂₈H₂₅F₃N₆O: 259.1015; found: 259.1023.

### Example 19

### 3-(2-(6-(Isopropylamino)pyridazin-3-yl)ethynyl)-4-methyl-N-(4-(4-methyl-1H-imidazol-1-yl)-3-(trifluoromethyl)phenyl)benzamide

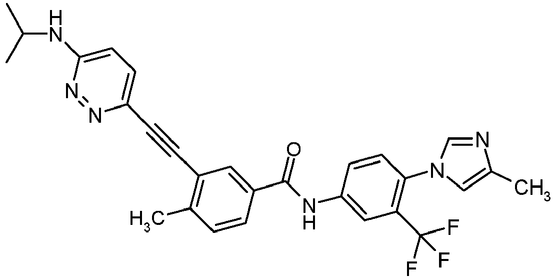

The title compound was synthesized from N-isopropyl-6-ethynylpyridazin-3-amine and 3-iodo-4-methyl-N-(4-(4-methyl-1H-imidazol-1-yl)-3-(trifluoromethyl)phenyl) benzamide in a manner similar to that described for in **Example 1.** The product was obtained as a pale yellow solid. Mp:141-143°C; ¹H NMR (300 MHz, CDCl₃) δ: 9.82 (1 H, s), 8.34 (1 H, s), 8.20-8.23 (1 H, d, *J* = 7.8 Hz), 7.96 (1 H, s), 7.84-7.86 (1 H, d, *J* = 8.1 Hz), 7.54 (1 H, s), 7.29-7.31 (3 H, m), 6.81 (1 H, s), 6.63-6.66 (1 H, d, *J* = 8.4 Hz), 5.11 (1 H, brs), 4.08 (1 H, m), 2.46 (3 H, s), 2.29 (3 H, s), 1.28-1.31 (6 H, d, *J* = 6.3 Hz). HRMS (ESI-TOF⁺): m/z [M+H]⁺ calcd for C₂₈H₂₆F₃N₆O: 519.2115; found: 519.2116.

### Example 20

### 4-Chloro-3-(2-(6-(cyclopropylamino)pyridazin-3-yl)ethynyl)-N-(4-(4-methyl-1H-imidazol-1-yl)-3-(trifluoromethyl)phenyl)benzamide

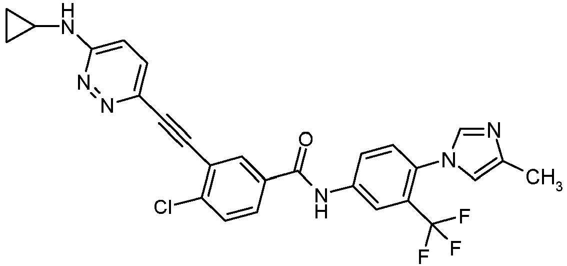

The title compound was synthesized from N-cyclopropyl-6-ethynylpyridazin-3-amine and 4-chloro-3-iodo-N-(4-(4-methyl-1H-imidazol-1-yl)-3-(trifluoromethyl)phenyl)benzamide in a manner similar to that described for in **Example 1.** The intermediate compound 4-chloro-3-iodo-N-(4-(4-methyl-1H-imidazol-1-yl)-3-(trifluoromethyl)phenyl)benzamide was made as for **Example 18 (Step 1** to **3)** with the spectra below: ¹H NMR (300 MHz, DMSO-*d*₆) δ: 10.78 (1 H, s), 8.55 (1 H, d, *J* = 2.1 Hz), 8.26 (1 H, s), 8.22 (1 H, s), 8.11 (1 H, s), 7.99-8.03 (1 H, dd, *J* = 2.1 and 8.1 Hz), 7.76-7.80 (2 H, m), 7.49 (1 H, s), 2.19 (3 H, s). LCMS: m/z [M+H]⁺ 505.9663.

The title compound was obtained as a pale yellow solid. Mp: 150-152°C; ¹H NMR (300 MHz, CDCl₃) δ: 10.45 (1 H, s), 8.38 (1 H, s), 8.24-8.27 (1 H, d, *J* = 8.1 Hz), 8.10 (1 H, s), 7.85-7.87 (1 H, d, *J* = 6.9 Hz), 7.42-7.52 (3 H, m), 7.29-7.32 (1 H, d, *J* = 9.0 Hz), 7.07 (1 H, s), 6.80 (1 H, s), 6.09 (1 H, s), 2.59 (1 H, brs), 2.28 (3 H, s), 0.88-0.89 (2 H, m), 0.65 (2 H, brs). HRMS (ESI-TOF⁺): m/z [M+2H]⁺² calcd for C₂₇H₂₂ClF₃N₆O: 269.0742; found: 269.0735.

### Example 21

### 4-Chloro-3-(2-(6-(cyclobutylamino)pyridazin-3-yl)ethynyl)-N-(4-(4-methyl-1H-imidazol-1-yl)-3-(trifluoromethyl)phenyl)benzamide

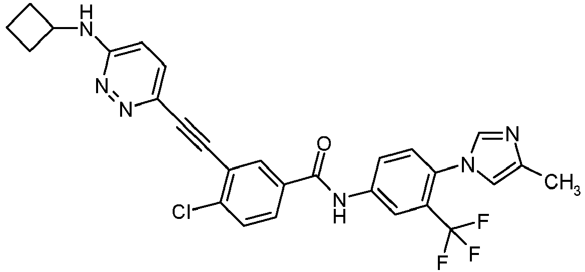

The title compound was synthesized from N-cyclobutyl-6-ethynylpyridazin-3-amine and 4-chloro-3-iodo-N-(4-(4-methyl-1H-imidazol-1-yl)-3-(trifluoromethyl)phenyl)benzamide in a manner similar to that described for in **Example 1.** The product was obtained as a pale yellow solid. Mp:153-155°C; ¹H NMR (300 MHz, CDCl₃) δ: 10.34 (1 H, s), 8.36 (1 H, s), 8.24-8.27 (1 H, d, *J* = 8.1 Hz), 8.03 (1 H, s), 7.82-7.85 (1 H, d, *J* = 8.1 Hz), 7.48 (1 H, s), 7.41-7.44 (1 H, d, *J* = 8.7 Hz), 7.34-7.37 (2 H, t, *J* = 9.3 and 8.7 Hz), 6.80 (1 H, s), 6.61-6.64 (1 H, d, *J* = 9.6 Hz), 5.57-5.59 (1 H, d, *J* = 6.0 Hz), 4.22-4.29 (1 H, m), 2.45-2.48 (2 H, m), 2.28 (3 H, s), 1.76-2.01 (4 H, m). HRMS (ESI-TOF⁺): m/z [M+2H]⁺² calcd for C₂₈H₂₄ClF₃N₆O: 276.0821; found: 276.0817.

### Example 22

### 4-Chloro-3-(2-(6-(isopropylamino)pyridazin-3-yl)ethynyl)-N-(4-(4-methyl-1H-imidazol-1-yl)-3-(trifluoromethyl)phenyl)benzamide

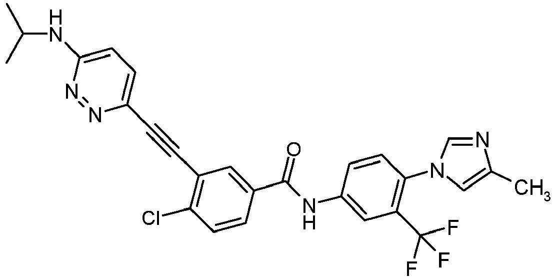

The title compound was synthesized from N-isopropyl-6-ethynylpyridazin-3-amine and 4-chloro-3-iodo-N-(4-(4-methyl-1H-imidazol-1-yl)-3-(trifluoromethyl)phenyl)benzamide in a manner similar to that described for in **Example 1.** The product was obtained as a pale yellow solid. Mp:133-135°C; ¹H NMR (300 MHz, CDCl₃) δ: 10.46 (1 H, s), 8.40 (1 H, s), 8.28-8.31 (1 H, d, *J* = 8.1 Hz), 8.04 (1 H, s), 7.82-7.84 (1 H, d, *J* = 8.1 Hz), 7.53 (1 H, s), 7.41-7.43 (1 H, d, *J* = 8.4 Hz), 7.34-7.36 (2 H, m), 6.81 (1 H, s), 6.66-6.69 (1 H, d, *J* = 9.3 Hz), 5.13-5.15 (1 H, d, *J* = 6.3 Hz), 4.08 (1 H, m), 2.29 (3 H, s), 1.30-1.32 (6 H, d, *J* = 6.0 Hz). HRMS (ESI-TOF⁺): m/z [M+H]⁺ calcd for C₂₇H₂₃ClF₃N₆O: 539.1568; found: 539.1592.

### Example 23

### 3-(2-(6-(Cyclopropylamino)pyridazin-3-yl)ethynyl)-4-methyl-N-(3-(4-methyl-1H-imidazol-1-yl)-5-(trifluoromethyl)phenyl)benzamide

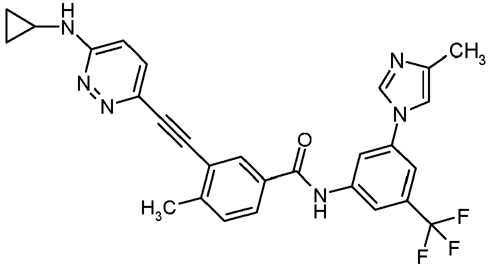

The title compound was synthesized from N-cyclopropyl-6-ethynylpyridazin-3-amine and 4-chloro-3-iodo-N-(4-(4-methyl-1H-imidazol-1-yl)-3-(trifluoromethyl)phenyl)benzamide in a manner similar to that described for in **Example 1.** The product was obtained as a colorless solid. Mp: 226-228°C; ¹H NMR (300 MHz, DMSO-*d*₆) δ: 10.72 (1 H, s), 8.30 (1 H, s), 8.18-8.24 (2 H, m), 7.94-7.98 (1 H, dd, *J* = 1.5 and 8.1 Hz), 7.72-7.74 (2 H, m), 7.55-7.59 (2 H, m), 7.50 (1 H, s), 6.93-6.96 (1 H, d, *J* = 9.6 Hz), 2.66 (1 H, m), 2.57 (3 H, s), 2.23 (3 H, s), 0.75-0.81 (2 H, m), 0.47-0.52 (2 H, m). HRMS (ESI-TOF⁺): m/z [M+H]⁺ calcd for C₂₈H₂₄F₃N₆O: 517.1958; found: 517.1950.

### Example 24

### 3-(2-(6-(Cyclobutylamino)pyridazin-3-yl)ethynyl)-4-methyl-N-(3-(4-methyl-1H-imidazol-1-yl)-5-(trifluoromethyl)phenyl)benzamide

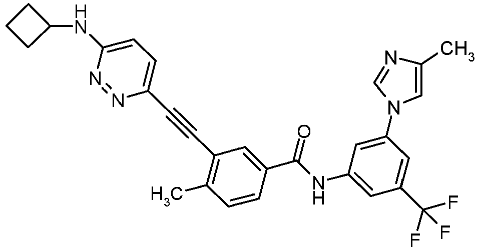

### Step 1. 3-(4-Methyl-1H-imidazol-1-yl)-5-(trifluoromethyl)aniline

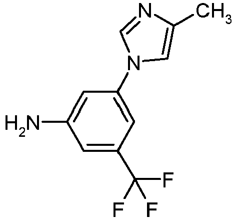

A suspension of 3-bromo-5-(trifluoromethyl)aniline (4.8 g, 20 mmol), 4-methylimidazole (1.97 g, 24 mmol), K₂CO₃ (3.04 g, 22mmol), CuI (0.57 g, 3 mmol) and 8-hydroxyquinoline (0.44 g, 3 mmol) in 20 mL DMSO was stirred at 120°C in a sealed tube under Ar₂ for 16 hrs. The mixture was cooled down to 50 °C and 28% aq ammonia (10 mL) was added. The mixture was maintained at this temperature for 1 h. After cooling to rt, H₂O and EtOAc were added. The aqueous layer was extracted with EtOAc (60 mL×3) and the organic layer was washed with brine, dried with Na₂SO₄, after filtration, the filtrate was concentrated under reduced pressure and purified by chromatography on silica gel (CH₂Cl₂/CH₃OH 97:3) to give 2.85 g product as pale yellow solid (59.1%). ¹H NMR (300 MHz, CDCl₃) δ: 7.76 (1 H, s), 7.01 (1 H, s), 6.94 (1 H, s), 6.84 (1 H, s), 6.78 (1 H, s), 4.11 (2 H, brs), 2.29 (3 H, s). LCMS: m/z [M+H]⁺ 242.0966.

### Step2. 3-Iodo-4-methyl-N-(3-(4-methyl-1H-imidazol-1-yl)-5-(trifluoromethyl)phenyl)benzamide

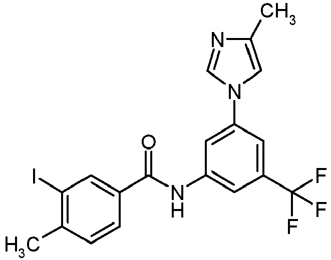

A solution of 3-iodo-4-methylbenzoic acid (1.31 g, 5 mmol) in SOCl₂ (10 mL) was refluxed for 2 hrs, then evaporated in vacuo to remove residual SOCl₂. The residue was dissolved in 5 mL anhydrous THF and added to a solution of DIPEA (0.77 g, 6 mmol), 3-(4-methyl-1H-imidazol-1-yl)-5-(trifluoromethyl)aniline (1.21 g, 5 mmol) and DMAP (24 mg) in 10 mL anhydrous THF in dropwise. The result mixture was stirred at rt for 20 hrs, and evaporated in vacuo. To the residue water was added and extracted with EtOAc (50 mL×3) then CH₂Cl₂. The combined organic layers were evaporated in vacuo to give crude product. The crude product was triturated with CH₂Cl₂/EtOAc to give the title compound 2.04 g as a colorless solid (84.3%). ¹H NMR (300 MHz, DMSO-*d*₆) δ: 10.67 (1 H, s), 8.46 (1 H, d, *J* = 1.5 Hz), 8.27 (1 H, s), 8.21 (1 H, s), 8.14 (1 H, s), 7.93-7.96 (1 H, dd, *J* = 1.5 and 7.5 Hz), 7.74 (1 H, s), 7.50-7.55 (2 H, m), 2.46 (3 H, s), 2.19 (3 H, s). LCMS: m/z [M+H]⁺ 486.0211.

### Step 3.

### 3-(2-(6-(Cyclobutylamino)pyridazin-3-yl)ethynyl)-4-methyl-N-(3-(4-methyl-1H-imidazol-1-yl)-5-(trifluoromethyl)phenyl) benzamide

The title compound was synthesized from N-cyclobutyl-6-ethynylpyridazin-3-amine and 3-iodo-4-methyl-N-(3-(4-methyl-1H-imidazol-1-yl)-5-(trifluoromethyl)phenyl)benzamide (as prepared above) in a manner similar to that described for in **Example 1.** The product was obtained as a pale yellow solid. Mp: 223-225°C; ¹H NMR (300 MHz, DMSO-*d*₆) δ: 10.72 (1 H, s), 8.31 (1 H, s), 8.17-8.22 (2 H, m), 7.94-7.96 (1 H, dd, *J* = 1.5 and 8.1 Hz), 7.75 (1 H, s), 7.47-7.61 (4 H, m), 6.77-6.80 (1 H, d, *J =* 9.0 Hz), 4.42 (1 H, m), 2.56 (3 H, s), 2.33-2.35 (2 H, m), 2.19 (3 H, brs), 1.88-1.99 (2 H, m), 1.72-1.78 (2 H, m). HRMS (ESI-TOF⁺): m/z [M+H]⁺ calcd for C₂₉H₂₆F₃N₆O: 531.2115; found: 531.2113.

### Example 25

### 3-(2-(6-(Isopropylamino)pyridazin-3-yl)ethynyl)-4-methyl-N-(3-(4-methyl-1H-imidazol-1-yl)-5-(trifluoromethyl)phenyl)benzamide

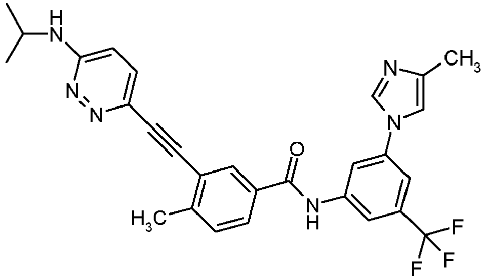

The title compound was synthesized from N-isopropyl-6-ethynylpyridazin-3-amine and 3-iodo-4-methyl-N-(3-(4-methyl-1H-imidazol-1-yl)-5-(trifluoromethyl)phenyl)benzamide in a manner similar to that described for in **Example 1.** The product was obtained as a pale yellow solid. Mp:192-194°C; ¹H NMR (300 MHz, DMSO-*d*₆) δ: 10.73 (1 H, s), 8.33 (1 H, s), 8.17-8.22 (2 H, m), 7.94-7.96 (1 H, d, *J* = 7.2 Hz), 7.76 (1 H, s), 7.54-7.57 (1 H, d, *J* = 7.8 Hz), 7.45-7.48 (1 H, d, *J* = 8.4 Hz), 7.15-7.18 (1 H, d, *J* = 7.5 Hz), 6.79-6.82 (1 H, d, *J* = 9.0 Hz), 4.14-4.20 (1 H, m), 2.56 (3 H, s), 2.19 (3 H, brs), 1.20-1.22 (6 H, d, *J* = 6.3 Hz). HRMS (ESI-TOF⁺): m/z [M+H]⁺ calcd for C₂₈H₂₆F₃N₆O: 519.2115; found: 519.2121.

### Example 26

### 4-Chloro-3-(2-(6-(cyclopropylamino)pyridazin-3-yl)ethynyl)-N-(3-(4-methyl-1H-imidazol-1-yl)-5-(trifluoromethyl)phenyl)benzamide

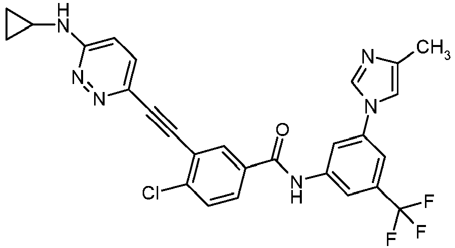

The title compound was synthesized from N-cyclopropyl-6-ethynylpyridazin-3-amine and 4-chloro-3-iodo-N-(3-(4-methyl-1H-imidazol-1-yl)-5-(trifluoromethyl)phenyl) benzamide in a manner similar to that described for in **Example 1.** The intermediate compound 4-chloro-3-iodo-N-(3-(4-methyl-1H-imidazol-1-yl)-5-(trifluoromethyl)phenyl)benzamide was made as for **Example 24 (Step 1** to **2)** with the spectra below: ¹H NMR (300 MHz, DMSO-*d*₆) δ: 10.78 (1 H, s), 8.55 (1 H, d, *J* = 2.1 Hz), 8.26 (1 H, s), 8.22 (1 H, s), 8.11 (1 H, s), 7.99-8.03 (1 H, dd, *J* = 2.1 and 8.1 Hz), 7.76-7.80 (2 H, m), 7.49 (1 H, s), 2.19 (3 H, s). LCMS: m/z [M+H]⁺ 505.9663.

The title compound was obtained as a colorless solid. Mp:213-215°C; ¹H NMR (300 MHz, DMSO-*d*₆) δ: 10.83 (1 H, s), 8.37 (1 H, d, *J* = 2.1 Hz), 8.28 (1 H, s), 8.15 (1 H, s), 8.01-8.05 (1 H, dd, *J* = 2.1 and 8.4 Hz), 7.76-7.86 (3 H, m), 7.52-7.63 (2 H, m), 6.94-6.97 (1 H, d, *J* = 9.3 Hz), 2.67 (1 H, m), 2.19 (3 H, s), 0.75-0.81 (2 H, m), 0.48-0.53 (2 H, m). HRMS (ESI-TOF⁺): m/z [M+H]⁺ calcd for C₂₇H₂₁ClF₃N₆O: 537.1412; found: 537.1413.

### Example 27

### 4-Chloro-3-(2-(6-(cyclobutylamino)pyridazin-3-yl)ethynyl)-N-(3-(4-methyl-1H-imidazol-1-yl)-5-(trifluoromethyl)phenyl)benzamide

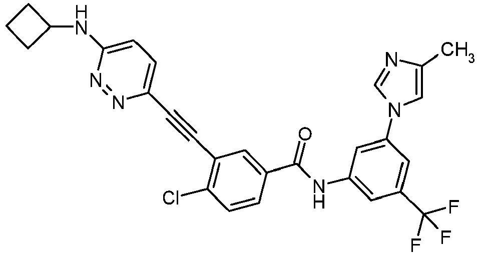

The title compound was synthesized from N-cyclobutyl-6-ethynylpyridazin-3-amine and 4-chloro-3-iodo-N-(3-(4-methyl-1H-imidazol-1-yl)-5-(trifluoromethyl)phenyl)benzamide in a manner similar to that described for in **Example 1.** The product was obtained as a pale yellow solid. Mp: 225-227°C; ¹H NMR (300 MHz, DMSO-*d*₆) δ: 10.82 (1 H, s), 8.35 (1 H, d, *J* = 2.1 Hz), 8.27 (1 H, s), 8.13 (1 H, s), 7.99-8.03 (1 H, dd, *J* = 2.1 and 8.1 Hz), 7.81-7.84 (1 H, d, *J* = 8.7 Hz), 7.76 (1 H, s), 7.66-7.68 (2 H, m), 7.46-7.49 (1 H, d, *J* = 9.0 Hz), 6.76-6.80 (1 H, d, *J* = 9.3 Hz), 4.40 (1 H, m), 2.32-2.34 (2 H, m), 2.17 (3 H, brs), 1.87-1.97 (2 H, m), 1.71-1.76 (2 H, m). HRMS (ESI-TOF⁺): m/z [M+2H]⁺² calcd for C₂₈H₂₄ClF₃N₆O: 276.0821; found: 276.0819.

### Example 28

### 3-(2-(2-(Cyclopropylamino)pyrimidin-5-yl)ethynyl)-4-methyl-N-(4-(4-methyl-1H-imidazol-1-yl)-3-(trifluoromethyl)phenyl)benzamide

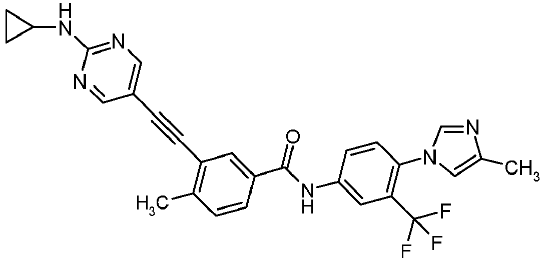

The title compound was synthesized from N-cyclopropyl-5-ethynylpyrimidin-3-amine and 3-iodo-4-methyl-N-(4-(4-methyl-1H-imidazol-1-yl)-3-(trifluoromethyl)phenyl) benzamide in a manner similar to that described for in **Example 1.** The product was obtained as a pale yellow solid. Mp:131-133°C; ¹H NMR (300 MHz, CDCl₃) δ: 8.49 (2 H, brs), 8.34 (1 H, s), 8.10 (2 H, m), 7.99 (1 H, s), 7.78-7.81 (1 H, d, *J* = 8.4 Hz), 7.61 (1 H, brs), 7.37-7.40 (2 H, m), 6.83 (1 H, brs), 5.58 (1 H, s), 2.82 (1 H, m), 2.57 (3 H, s), 2.31 (3 H, s), 0.87-0.89 (2 H, m), 0.60 (2 H, brs).
HRMS (ESI-TOF⁺): m/z [M+H]⁺ calcd for C₂₈H₂₄F₃N₆O: 517.1958; found: 517.1960.

### Example 29

### 4-Chloro-3-(2-(2-(cyclopropylamino)pyrimidin-5-yl)ethynyl)-N-(4-(4-methyl-1H-imidazol-1-yl)-3-(trifluoromethyl)phenyl)benzamide

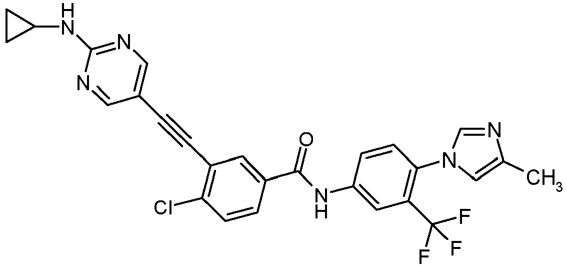

The title compound was synthesized from N-cyclopropyl-5-ethynylpyrimidin-3-amine and 4-chloro-3-iodo-N-(4-(4-methyl-1H-imidazol-1-yl)-3-(trifluoromethyl)phenyl)benzamide in a manner similar to that described for in **Example 1.** The product was obtained as a colorless solid. Mp:141-143°C; ¹H NMR (300 MHz, CDCl₃) δ: 8.72 (1 H, s), 8.50 (1 H, s), 8.06-8.09 (3 H, m), 7.82-7.85 (1 H, d, *J* = 8.4 Hz), 7.55-7.58 (2 H, m), 7.35-7.38 (1 H, d, *J* = 8.1 Hz), 6.83 (1 H, s), 5.64 (1 H, s), 2.82 (1 H, brs), 2.29 (3 H, s), 0.88-0.89 (2 H, m), 0.59 (2 H, brs). HRMS (ESI-TOF⁺): m/z [M+H]⁺ calcd for C₂₇H₂₁ClF₃N₆O: 537.1412; found: 537.1417.

### Example 30

### 3-(2-(Imidazo[1,2-b]pyridazin-3-yl)ethynyl)-4-methyl-N-(4-(4-methyl-1H-imidazol-1-yl)-3-(trifluoromethyl)-phenyl)benzamide

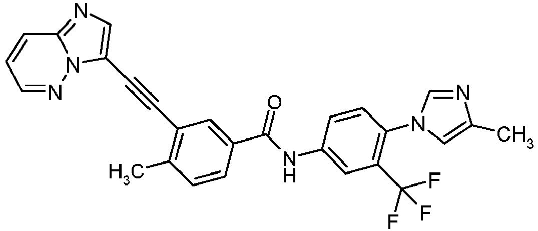

The title compound was synthesized from 3-ethynylimidazo[1,2-b]pyridazine and 3-iodo-4-methyl-N-(4-(4-methyl-1H-imidazol-1-yl)-3(trifluoromethyl)phenyl)benzamide in a manner similar to that described for in **Example 1.** The title compound was obtained as a light yellow solid. Mp: 124-126°C; ¹H NMR (300 MHz, CDCl₃) δ: 9.00 (1 H, s), 8.48 (1 H, d, *J* = 4.8 Hz), 8.17 (1 H, s), 8.09 (3 H, m), 7.93-7.95 (1 H, d, *J* = 8.7 Hz), 7.86-7.89 (1 H, d, *J* = 8.1 Hz), 7.55 (1 H, s), 7.39-7.42 (1 H, d, *J* = 8.1 Hz), 7.34-7.36 (1 H, d, *J* = 8.4 Hz), 7.11-7.16 (1 H, dd, *J* = 4.2 and 8.4 Hz), 6.82 (1 H, s), 2.64 (3 H, s), 2.29 (3 H, s). HRMS (ESI-TOF⁺): m/z [M+H]⁺ calcd for C₂₇H₂₀F₃N₆O: 501.1645; found: 501.1626.

### Example 31

### 4-Chloro-3-(2-(imidazo[1,2-b]pyridazin-3-yl)ethynyl)-N-(4-(4-methyl-1H-imidazol-1-yl)-3-(trifluoromethyl)phenyl)benzamide

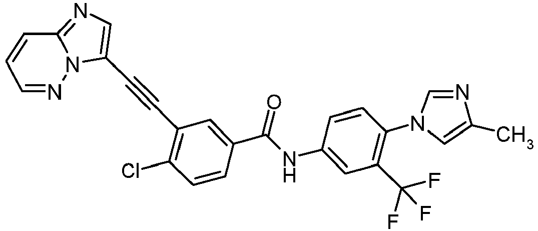

The title compound was synthesized from 3-ethynylimidazo[1,2-b]pyridazine and 4-chloro-3-iodo-N-(4-(4-methyl-1H-imidazol-1-yl)-3-(trifluoromethyl)phenyl)benzamide in a manner similar to that described for in **Example 1.** The title compound was obtained as a light yellow solid. Mp: 153-155°C; ¹H NMR (300 MHz, CDCl₃) δ: 9.39 (1 H, s), 8.49 (1 H, d, *J* = 3.3 Hz), 8.08-8.18 (4 H, m), 7.91-7.94 (2 H, m), 7.57-7.60 (2 H, m), 7.34 (1 H, brs), 7.14-7.16 (1 H, m), 6.85 (1 H, brs), 2.28 (3 H, s). HRMS (ESI-TOF⁺): m/z [M+H]⁺ calcd for C₂₆H₁₇ClF₃N₆O: 521.1099; found: 521.1092.

### Example 32

### 4-Chloro-3-(2-(imidazo[1,2-b]pyridazin-3-yl)ethynyl)-N-(3-(4-methyl-1H-imidazol-1-yl)-5-(trifluoromethyl)phenyl)benzamide

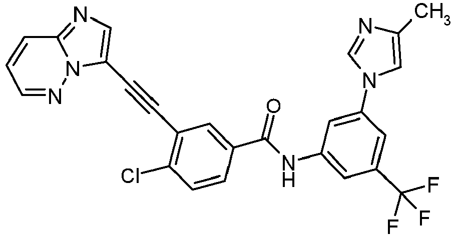

The title compound was synthesized from 3-ethynylimidazo[1,2-b]pyridazine and 4-chloro-3-iodo-N-(3-(4-methyl-1H-imidazol-1-yl)-5-(trifluoromethyl)phenyl)benzamide in a manner similar to that described for in **Example 1.** The title compound was obtained as a light yellow solid. Mp: 153-154°C; ¹H NMR (300 MHz, CDCl₃) δ: 9.35 (1 H, s), 8.48-8.49 (1 H, d, *J* = 3.6 Hz), 8.25 (1 H, s), 8.15 (2 H, brs), 7.89-7.92 (4 H, m), 7.57-7.60 (1 H, d, *J* = 8.4 Hz), 7.37 (1 H, s), 7.12-7.16 (1 H, dd, *J* = 4.2 and 9.3 Hz), 2.27 (3 H, s). HRMS (ESI-TOF⁺): m/z [M+2H]⁺² calcd for C₂₆H₁₈ClF₃N₆O: 261.0586; found: 261.0584.

### Example 33

### 4-Chloro-3-(2-(2-(cyclopropylamino)pyrimidin-5-yl)ethynyl)-N-(3-(4-methyl-1H-imidazol-1-yl)-5-(trifluoromethyl)phenyl)benzamide

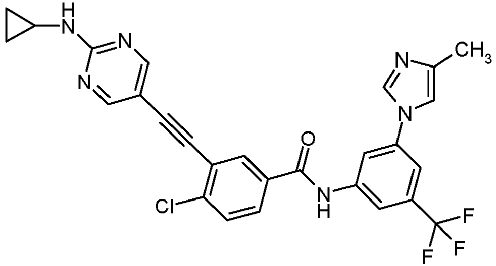

The title compound was synthesized from N-cyclopropyl-5-ethynylpyrimidin-3-amine and 4-chloro-3-iodo-N-(3-(4-methyl-1H-imidazol-1-yl)-5-(trifluoromethyl)phenyl) benzamide in a manner similar to that described for in **Example 1.** The product was obtained as a colorless solid. Mp: 163-165°C; ¹H NMR (300 MHz, DMSO-*d*₆) δ: 10.79 (1 H, s), 8.53 (3 H, brs), 8.19-8.25 (2 H, m), 8.12 (1 H, s), 7.95-8.00 (2 H, m), 7.75-7.79 (2 H, m), 2.75 (1 H, brs), 2.16 (3 H, s), 0.68-0.79(2 H, m), 0.51 (2 H, brs). HRMS (ESI-TOF⁺): m/z [M+H]⁺ calcd for C₂₇H₂₁ClF₃N₆O: 537.1412; found: 537.1399.

### Example 34

### 3-(2-(Imidazo[1,2-b]pyridazin-3-yl)ethynyl)piperidine-N-(4-((4-methylpiperazin-1-yl)methyl)-3-(trifluoromethyl)phenyl)-1-carboxamide

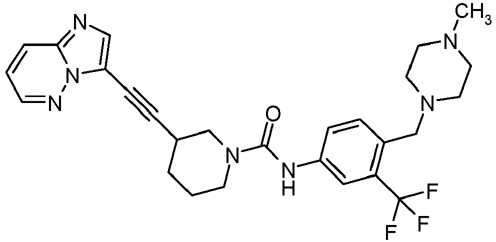

### Step 1. 1-BOC-3-formylpiperidine

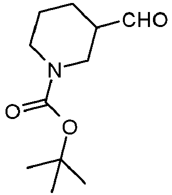

1-BOC-3-hydroxymethylpiperidine (2.15 g, 10 mmol) and triethylamine (3.03 g, 30 mmol) were dissolved in 10 mL DMSO, 15 mL DMSO solution of SO₃Py (4.77g, 30 mmol) was added to the above mixture in dropwise and the result mixture was stirred at rt for 2 hrs. The mixture was poured into 100 mL ice-water, extracted with EtOAc (100 mL×3), organic layer was washed with brine, dried with Na₂SO₄, filtered, and the filtrate was evaporated in vacuo. The crude product was purified by chromatography on silica gel (PE/EtOAc 5:1) to give 1.46 g product as colorless oil (68.5%). ¹H NMR (300 MHz, CDCl₃) δ: 9.70 (1 H, s), 3.91-3.93 (1 H, m), 3.62-3.67 (1 H, m), 3.29-3.36 (1 H, m), 3.05-3.13 (1 H, m), 2.40-2.45 (1 H, m), 1.95-1.97 (1 H, m), 1.63-1.72 (1 H, m), 1.49-1.59 (1 H, m), 1.46 (9 H, s).

### Step 2. 1-BOC-3-ethynylpiperidine

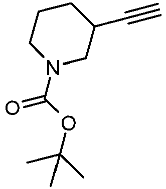

A solution of 1-BOC-3-formylpiperidine (1.46 g, 6.85 mmol) and (diazomethyl) phosponic acid dimethyl ester (1.79 g, 11.94 mmol) in 50 mL methanol was stirred at ice-bath for 10 min, K₂CO₃ (1.96 g, 14.2 mmol) was added to the above mixture and stirred at ice-bath for 2 hrs, then stirred at rt overnight. The mixture was evaporated in vacuo, to the residue was added EtOAc and water, the organic layer was separated and water layer was extracted with EtOAc (100 mL×3). The combined organic layers were washed with water and brine successively, dried with Na₂SO₄, filtered, and the filtrate was evaporated in vacuo to give 1.44 g product as colorless oil (100.0%). ¹H NMR (300 MHz, CDCl₃) δ: 3.90 (1 H, brs), 3.70-3.75 (1 H, m), 2.95-3.02 (2 H, m), 2.40-2.47 (1 H, m), 2.05 (1 H, d, *J* = 2.1 Hz), 1.94-1.99 (1 H, m), 1.69-1.73 (1 H, m), 1.50-1.63 (2 H, m), 1.46 (9 H, s).

### Step 3. 1-BOC-3-(2-(imidazo[1,2-b]pyridazin-3-yl)ethynyl)piperidine

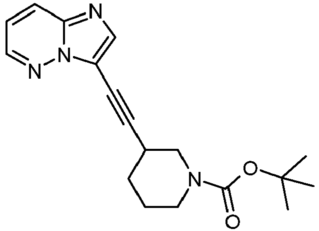

A solution of 3-bromoimidazo[1,2-b]pyridazine (0.40 g, 2 mmol), 1-BOC-3-ethynylpiperidine (0.55 g, 2.6 mmol), Pd(PPh₃)₂Cl₂ (70 mg, 0.1 mmol), CuI (29 mg, 0.15 mmol), and DIPEA (0.39 g, 3 mmol) in DMF (20 mL) was stirred at 80°C under Ar₂ for 6 hrs. The mixture was poured into 100 mL water, extracted with EtOAc (60 mL×3), organic layer was washed with brine, dried with Na₂SO₄, filtered, and the filtrate was evaporated in vacuo. The crude product was purified by chromatography on silica gel (PE/EtOAc 7:3 to 3:2) to give 0.39 g product as yellow oil (60.0%). ¹H NMR (300 MHz, CDCl₃) δ: 8.50 (1 H, d, *J* = 4.2 Hz), 7.98-8.08 (2 H, m), 7.13-7.17 (1 H, dd, *J* = 4.5 and 8.7 Hz), 4.09 (1 H, brs), 3.76-3.82 (1 H, m), 3.19 (1 H, brs), 3.02-3.09 (1 H, m), 2.81-2.88 (1 H, m), 2.32 (1 H, brs), 2.11-2.15 (1 H, m), 1.67-1.81 (2 H, m), 1.46 (9 H, s). LCMS: m/z [M+H]⁺ 327.2112.

### Step 4. 3-(2-(Imidazo[1,2-b]pyridazin-3-yl)ethynyl)piperidine

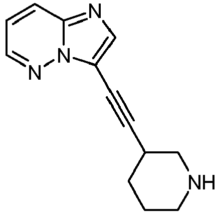

To a solution of 1-BOC-3-(2-(imidazo[1,2-b]pyridazin-3-yl)ethynyl)piperidine (0.39 g, 1.2 mmol) in CH₂Cl₂ (10 mL) was added TFA (1.23 g, 10.8 mmol), the mixture was stirred at rt overnight and evaporated in vacuo. To the residue was added 10 mL 10% K₂CO₃ solution, extracted with EtOAc (20 mL×3), dried with Na₂SO₄, after filtration, and the filtrate was evaporated under reduced pressure to give 0.33 g product as yellow oil (107.0%). ¹H NMR (300 MHz, CDCl₃) δ: 8.43 (1 H, d, *J* = 4.5 Hz), 7.93-7.97 (1 H, dd, *J* = 1.8 and 9.0 Hz), 7.91 (1 H, s), 7.05-7.09 (1 H, dd, *J* = 4.5 and 9.0 Hz), 2.82-2.98 (4 H, m), 2.08-2.11 (1 H, m), 1.75-1.84 (2 H, m), 1.53-1.58 (2 H, m).

### Step 5.

### 3-(2-(Imidazo[1,2-b]pyridazin-3-yl)ethynyl)piperidine-N-(4-((4-methylpiperazin-1-yl)methyl)-3-(trifluoromethyl)phenyl)-1-carboxamide

To a solution of 4-((4-methylpiperazin-1-yl)methyl)-3-(trifluoromethyl)aniline (as prepared in **Example 12 step 1** to **3**) (0.1 g, 0.37 mmol) in 15 mL anhydrous dioxane was added pyridine (0.036 mL, 0.45 mmol) and 4-nitrophenyl chloroformate (90 mg, 0.45 mmol), the mixture was stirred at 60°C for 2 hrs and then cooled to rt, and 3-(2-(imidazo[1,2-b]pyridazin-3-yl)ethynyl)piperidine (0.1 g, 0.44 mmol) was added to the above mixture. Then the mixture was stirred at 60°C for 9 h and evaporated in vacuo. The residue was purified by chromatography on silica gel (CH₂Cl₂/CH₃OH 25:1) to give 140 mg crude product, continued to purify via preparation TLC (CH₂Cl₂/CH₃OH 120:15) to give 60 mg product as a pale yellow solid. Mp: 65-70°C. ¹H NMR (300 MHz, CDCl₃) δ: 8.38 (1 H, s), 7.97 (1 H, d, *J* = 9.0 Hz), 7.88 (1 H, s), 7.58-7.61 (2 H, m), 7.42-7.44 (1 H, d, *J* = 7.8 Hz), 7.06-7.10 (1 H, m), 7.03 (1 H, s), 3.95 (1 H, m), 3.65 (4 H, m), 3.49-3.53 (2 H, m), 3.02 (4 H, m), 2.79 (4 H, brs), 2.69 (3 H, s), 2.13 (1 H, brs), 1.93 (2 H, m), 1.63 (1 H, brs). HRMS (ESI-TOF⁺): m/z [M+H]⁺ calcd for C₂₇H₃₁F₃N₇O: 526.2537; found: 526.2536.

### Example 35

### 3-(2-(Imidazo[1,2-b]pyridazin-3-yl)ethynyl)pyrrolidine-N-(4-((4-methylpiperazin-1-yl)methyl)-3-(trifluoromethyl)phenyl)-1-carboxamide

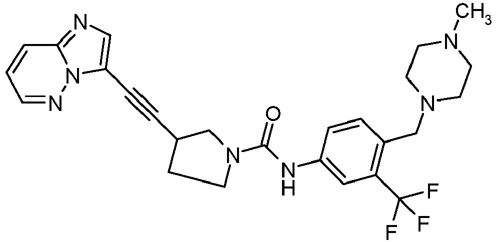

### Step 1. 1-BOC-3-formylpyrrolidine

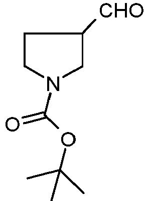

The title compound was synthesized using 1-BOC-3-hydroxymethylpyrrolidine as the material in a manner similar to that described for in **Example 30 step 1.** The product was obtained as colorless oil. ¹H NMR (300 MHz, CDCl₃) δ: 9.68 (1 H, s), 3.70 (1 H, m), 3.48-3.51 (1 H, m), 3.37 (2 H, brs), 2.99-3.03 (1 H, m), 2.05-2.24 (2 H, m), 1.45 (9 H, s).

### Step 2. 1-BOC-3-ethylnylpyrrolidine

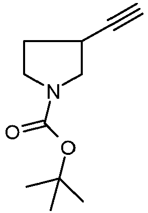

The title compound was synthesized using 1-BOC-3-formylpyrrolidine (as prepared above) as the material in a manner similar to that described for in **Example 30 step 2.** The product was obtained as colorless oil. ¹H NMR (300 MHz, CDCl₃) δ: 3.47-3.60 (2 H, m), 3.30 (2 H, brs), 2.90-2.95 (1 H, m), 2.12-2.18 (1 H, m), 2.10 (1 H, d, *J* = 1.8 Hz), 1.90-1.96 (1 H, m), 1.45 (9 H, s).

### Step 3. 1-BOC-3-(2-(imidazo[1,2-b]pyridazin-3-yl)ethynyl)pyrrolidine

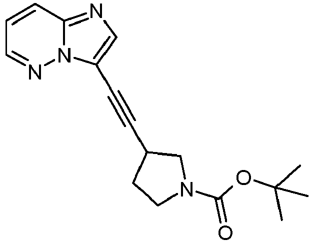

The title compound was synthesized using 1-BOC-3-ethynylpyrrolidine (as prepared above) as the material in a manner similar to that described for in **Example 30 step 3.** The product was obtained as yellow oil. ¹H NMR (300 MHz, CDCl₃) δ: 8.43 (1 H, d, *J* = 3.6 Hz), 7.92-7.99 (2 H, m), 7.07-7.11 (1 H, dd, *J* = 4.2 and 9.0 Hz), 3.76 (1 H, m), 3.60 (1 H, m), 3.32-3.45 (2 H, m), 2.25-2.32 (1 H, m), 2.12 (1 H, m), 1.71 (1 H, m), 1.47 (9 H, s).

### Step 4. 3-(2-(Imidazo[1,2-b]pyridazin-3-yl)ethynyl)pyrrolidine

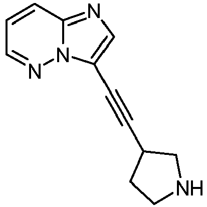

The title compound was synthesized using 1-BOC-3-(2-(imidazo[1,2-b]pyridazin-3-yl) ethynyl)pyrrolidine (as prepared above) as the material in a manner similar to that described for in **Example 30 step 4.** The product was obtained as yellow oil. ¹H NMR (300 MHz, CDCl₃) δ: 8.43 (1 H, d, *J* = 4.2 Hz), 7.94-7.97 (1 H, d, *J* = 9.0 Hz), 7.91 (1 H, s), 7.06-7.10 (1 H, dd, *J* = 4.2 and 9.0 Hz), 3.10-3.35 (5 H, m), 2.24-2.31 (1 H, m), 2.05-2.09 (1 H, m).

### Step 5.

### 3-(2-(Imidazo[1,2-b]pyridazin-3-yl)ethynyl)pyrrolidine-N-(4-((4-methylpiperazin-1-yl)methyl)-3-(trifluoromethyl)phenyl)-1-carboxamide

The title compound was synthesized from 3-(2-(imidazo[1,2-b]pyridazin-3-yl) ethynyl) pyrrolidine (as prepared above) and 4-((4-methylpiperazin-1-yl)-methyl)-3-(trifluoromethyl) aniline (as prepared in **Example 12 step 1** to **3**) in a manner similar to that described for in **Example 30.** The product was obtained as as a pale yellow solid. Mp: 83-85°C. ¹H NMR (300 MHz, CDCl₃) δ: 8.44 (1 H, s), 7.92-7.98 (2 H, m), 7.58-7.64 (3 H, m), 7.07-7.12 (1 H, dd, *J* = 4.5 and 9.0 Hz), 6.37 (1 H, s), 3.88-3.93 (1 H, m), 3.76 (1 H, m), 3.49-3.70 (4 H, m), 2.62 (8 H, brs), 2.44 (4 H, brs), 2.29-2.31 (2 H, m). HRMS (ESI-TOF⁺): m/z [M+H]⁺ calcd for C₂₆H₂₉F₃N₇O: 512.2380; found: 512.2375.

### Example 36

### N-(1,1-difluoro-2,3-dihydro-3-(4-methylpiperazin-1-yl)-1H-indan-6-yl)-3-(2-(imidazo[1,2-b]pyridazin-3-yl)ethynyl)-4-methylbenzamide

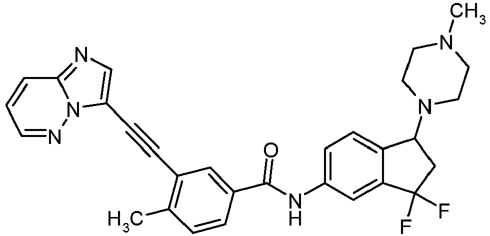

### Step 1. 6-Nitro-1-indatone

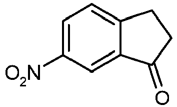

1-Indanone (13.2 g, 0.1 mol) was added in one portion to concentrated sulfuric acid (80 mL) at 0 °C. A solution of potassium nitrate (10.1 g, 0.1 mol) in concentrated sulfuric acid (30 mL) was added in small portions over a 40 min period. The mixture was stirred for 1 h at 0 °C, then poured over 500 g of ice. The mixture was filtered, washed with water, and air-dried. The crude product was purified by chromatography on silica gel (PE/EtOAc 2:1) to give 11.58 g product as colorless solid (65.4%). ¹H NMR (300 MHz, CDCl₃) δ: 2.81-2.85 (2 H, t, *J* = 6.3 Hz), 3.26-3.29 (2 H, t, *J* = 6.3 Hz), 7.65-7.68 (1 H, d, *J* = 8.4 Hz), 8.43-8.47 (1 H, dd, *J* = 8.4 and 2.1 Hz), 8.57 (1 H, s).

### Step 2. 6-Nitro-1H-inden-1-one

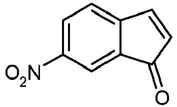

To a solution of the 6-nitro-1-indatone (2.66 g, 15 mmol) in dry toluene (100 mL) at 0°C under Ar was added successively Et₃N (2.55 mL, 18 mmol) and TMSOTf (2.85 mL, 15 mmol). The mixture was warmed to room temperature and stirred for 1h. The reaction mixture was again cooled to 0°C and diluted with ether (100 mL) and NaHCO₃ solution (150 mL). The layers were separated and the aqueous phase was extracted with ether (3 × 50 mL). The combined organic extracts were washed with brine then dried (Na₂SO₄), filtered, and evaporated under reduced pressure to afford the silyl enol ether as a light yellow oil.

The silyl enol ether was dissolved in CH₂Cl₂ (30 mL) and added dropwise to an aluminum foil-wrapped flask containing a suspension of Pd(OAc)₂ (3.3 g, 15 mmol) in dry CH₃CN (80 mL) under Ar. The mixture was stirred at room temperature for 2 h, then filtered through a short column of silica gel. The filtrate was evaporated under reduced pressure, and the residue was purified by chromatography on silica gel (9%-21% EtOAc / PE) to afford 0.69 g product as yellow solid (26.3%). ¹H NMR (300 MHz, CDCl₃) δ: 6.23-6.25 (1 H, d, *J* = 6.3 Hz), 7.27-7.30 (1 H, d, *J* = 8.1 Hz), 7.71-7.73 (1 H, d, *J* = 6.0 Hz), 8.26 (1 H, s), 8.32-8.35 (1 H, dd, *J* = 7.8 and 2.4 Hz).

### Step 3. 2,3-Dihydro-3-(4-methylpiperazin-1-yl)-6-nitroindan-1-one

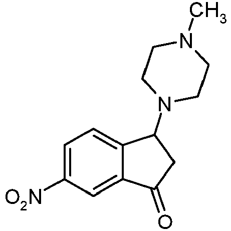

To a solution of 6-nitro-1H-inden-1-one (690 mg, 3.9 mmol) in THF (10 mL) was added N-methylpiperizine (0.87 mL, 7.8 mmol), and the resulting reaction mixture was stirred at room temperature for 12 h. THF was removed in vacuo, and the residue was purified by chromatography on silica gel (DCM/Methanol = 10:1) to afford 0.86 g product as black oil (80.3%). ¹H NMR (300 MHz, CDCl₃) δ: 2.31 (3 H, s), 2.43-2.48 (6 H, m), 2.61 (2 H, brs), 2.71-2.80 (1 H, dd, *J* = 7.2 and 12.3 Hz), 2.88-2.94 (1 H, m), 4.66 (1 H, brs), 7.89-7.92 (1 H, d, *J* = 8.1 Hz), 8.48-8.50 (1 H, d, *J* = 8.1 Hz), 8.56 (1 H, s).

### Step 4. 1,1-Dithioacetal-2,3-dihydro-3-(4-methylpiperazin-1-yl)-6-nitroindan

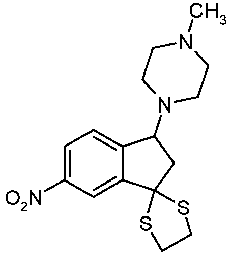

2,3-dihydro-3-(4-methylpiperazin-1-yl)-6-nitroindan-1-one (0.86 g, 3.1 mmol) and 1,2-ethanedithiol (0.62 mL,7.4 mmol) were dissolved in 25 mL dichloromethane and cooled to -15°C under Ar. Boron trifluoride-diethyl ether complex (2.2 mL, 8.4 mmol) was added at this temperature. The mixture was stirred for 3 h at -15°C and at room temperature over night. The solution was poured carefully into saturated NaHCO3 solution. The aqueous layer was extracted three times with dichloromethane. The organic layer was washed with brine, dried (Na2SO4) and evaporated. The residue was purified by column chromatography on silica gel (CH₂Cl₂/Methanol = 20:1 to 15:1) to afford 0.6 g product as a black solid (55.6%). LCMS: m/z [M+H]⁺ 352.1149.

### Step 5. 1-(1,1-Difluoro-2,3-dihydro-6-nitro-1H-indan-3-yl)-4-methylpiperazine

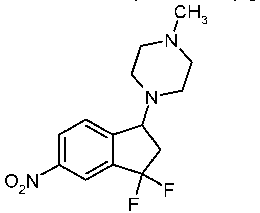

2.03 mL of a 70% solution of HF in pyridine was added to a suspension of 1,3-dibromo-dimethylhydantoin (2 g, 7 mmol) and 15 mL dichloromethane under Ar. The mixture was cooled to -74°C and a solution of 1,1-dithioacetal-2,3-dihydro-3-(4-methyl-piperazin-1-yl)-6-nitroindan (0.6 g, 1.7 mmol) in 5 mL CH₂Cl₂ was added below -65°C. After 5 h the cooling bath was removed. The reaction mixture was stirred over night at rt and was poured into 50 mL 2N NaOH containing 3 ml 39% NaHSO₃ solution. The aqueous layer was extracted three times with dichloromethane. The combined organic layers were washed with brine, dried (Na2SO4) and evaporated. The residue was purified by column chromatography on silica gel (DCM/Methanol = 30:1) to afford 90 mg product as a black solid (18.0%). LCMS: m/z [M+H]⁺ 298.1333.

### Step 6. 1-(1,1-Difluoro-2,3-dihydro-6-amino-1H-indan-3-yl)-4-methylpiperazine

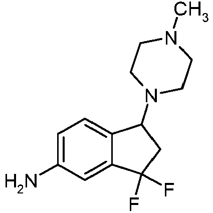

1-(1,1-difluoro-2,3-dihydro-6-nitro-1H-inden-3-yl)-4-methylpiperazine (90 mg, 0.3 mmol) was suspended in 10 ml of anhydrous ethanol. The mixture was hydrogenation with 50 mg of 10% Pd-C at ambient pressure for 5 h. Then the Pd-C was removed by filtration. The filtrate was evaporated to give the title compound 78 mg as a yellow oil (97.5%).

### Step 7. N-(1,1-difluoro-2,3-dihydro-3-(4-methylpiperazin-1-yl)-1H-indan-6-yl)-3-iodo-4-methylbenzamide

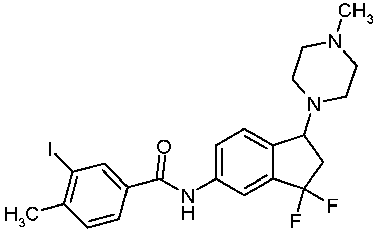

A solution of 3-iodo-4-methylbenzoic acid (79 mg, 0.3 mmol) in SOCl₂ (5 mL) was refluxed for 3 h, then evaporated in vacuum to remove residual SOCl₂. The residue was dissolved in 5 mL anhydrous THF and added to a solution of triethylamine (37 mg, 0.36 mmol), 1-(1,1-Difluoro-2,3-dihydro-6-amino-1H-indan-3-yl)-4-methylpiperazine (78 mg, 0.3 mmol) and DMAP (2 mg) in 5 mL anhydrous THF in dropwise. The result mixture was stirred at rt for 26 h, and evaporated in vacuum. The residue was purified by chromatography on silica gel (CH₂Cl₂/CH₃OH 97:3) to give 140 mg product as a pale yellow solid (91.5%). LCMS: m/z [M+H]⁺ 512.1049.

### Step 8. N-(1,1-difluoro-2,3-dihydro-3-(4-methylpiperazin-1-yl)-1H-indan-6-yl)-3-(2-(imidazo[1,2-b]pyridazin-3-yl)ethynyl)-4-methylbenzamide

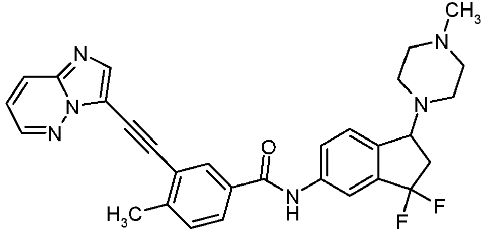

The title compound was synthesized from 3-Ethynylimidazo[1,2-b]pyridazine and N-(1,1-difluoro-2,3-dihydro-3-(4-methylpiperazin-1-yl)-1H-indan-6-yl)-3-iodo-4-methyl-benzamide in a manner similar to that described for in **Example 1.** The product was obtained as a pale yellow solid. Mp: 111-113°C. ¹H NMR (300 MHz, CDCl₃) δ: 0.83-0.87 (2 H, m), 2.31 (3 H, s), 2.49 (6 H, brs), 2.58-2.67 (5 H, m), 4.50-4.52 (1 H, m), 7.11-7.16 (1 H, dd, *J* = 9.0 and 4.5 Hz), 7.38-7.41 (1 H, d, *J* = 8.1 Hz), 7.46-7.49 (1 H, d, *J* = 8.1 Hz), 7.80-7.89 (3 H, m), 7.96-8.00 (1 H, dd, *J* = 9.6 and 1.5 Hz), 8.05 (1 H, d, *J* = 1.8 Hz), 8.09 (1 H, s), 8.18 (1 H, s), 8.48-8.49 (1 H, d, *J* = 4.2 Hz). HRMS (ESI-TOF⁺): m/z [M+2H]⁺² calcd for C₃₀H₃₀F₂N₆O: 264.1219; found: 264.1212.

### Example 37

### 3-(2-(2-(Cyclopropylamino)pyrimidin-5-yl)ethynyl)-N-(1,1-difluoro-2,3-dihydro-3-(4-methylpiperazin-1-yl)-1H-indan-6-yl)-4-methylbenzamide

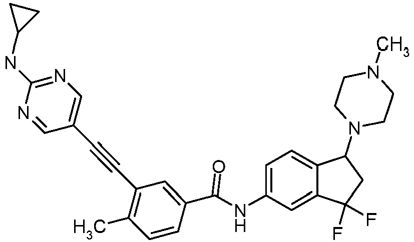

The title compound was synthesized from N-cyclopropyl-5-ethynylpyrimidin-3-amine and N-(1,1-difluoro-2,3-dihydro-3-(4-methylpiperazin-1-yl)-1H-indan-6-yl)-3-iodo-4-methyl benzamide (as prepared in **Example 36 step 1** to **7**) in a manner similar to that described for in **Example 1.** The product was obtained as a pale yellow solid. Mp: 95-97°C. ¹H NMR (300 MHz, CDCl₃) δ: 0.60-0.62 (2 H, m), 0.85-0.94 (4 H, m), 2.30 (3 H, s), 2.47 (5 H, brs), 2.56-2.67 (6 H, m), 2.82 (1 H, m), 4.49-4.51 (1 H, m), 7.35-7.38 (1 H, d, *J* = 8.1 Hz), 7.46-7.49 (1 H, d, *J* = 7.8 Hz), 7.74-7.79 (2 H, m), 7.83-7.86 (1 H, d, *J* = 8.1 Hz), 7.91 (1 H, s), 7.95 (1 H, s), 8.50 (2 H, s). HRMS (ESI-TOF⁺): m/z [M+2H]⁺² calcd for C₃₁H₃₄F₂N₆O: 272.1376; found: 272.1370.

### Example 38

### N-(1,1-difluoro-2,3-dihydro-3-(4-methylpiperazin-1-yl)-1H-indan-6-yl)-3-(2-(imidazo[1,2-b]pyridazin-3-yl)ethynyl)-4-chlorobenzamide

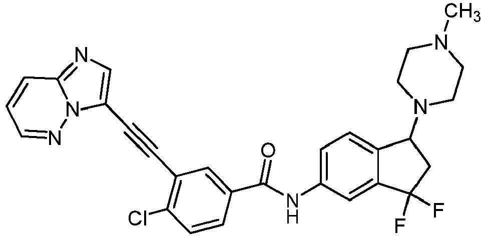

### Step 1. N-(1,1-difluoro-2,3-dihydro-3-(4-methylpiperazin-1-yl)-1H-indan-6-yl)-3-iodo-4-chlorobenzamide

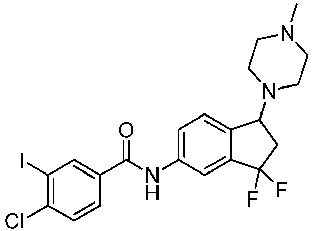

The title compound was synthesized from 4-chloro-3-iodobenzoic acid and 1-(1,1-difluoro-2,3-dihydro-6-amino-1H-indan-3-yl)-4-methylpiperazine (as prepared in Example 36 step 6) in a manner similar to that described in Example 36 step 7. The product was obtained as a pale yellow solid. LCMS: m/z [M+H]⁺ 532.0435.

### Step 2. N-(1,1-difluoro-2,3-dihydro-3-(4-methylpiperazin-1-yl)-1H-indan-6-yl)-3-(2-(imidazo[1,2-b]pyridazin-3-yl)ethynyl)-4-chlorobenzamide

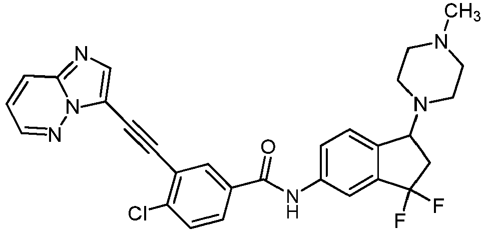

The title compound was synthesized from 3-Ethynylimidazo[1,2-b]pyridazine and 4-chloro-N-(3,3-difluoro-1-(4-methylpiperazin-1-yl)-2,3-dihydro-1H-indan-5-yl)-3-iodobenzamide in a manner similar to that described in **Example 1.** The product was obtained as a pale yellow solid. Mp: 140-142°C. ¹H NMR (300 MHz, CDCl₃) δ: 2.33 (3 H, s), 2.50-2.64 (10 H, m), 4.50 (1 H, m), 7.11-7.16 (1 H, dd, *J* = 9.0 and 4.5 Hz), 7.46-7.48 (1 H, d, *J* = 7.8 Hz), 7.55-7.58 (1 H, d, *J* = 8.4 Hz), 7.84-7.94 (4 H, m), 8.11 (1 H, s), 8.15 (1 H, s), 8.49-8.50 (1 H, d, *J* = 3.6 Hz), 8.57 (1 H, s). HRMS (ESI-TOF⁺): m/z [M+2H]⁺² calcd for C₂₉H₂₇ClF₂N₆O: 274.0951; found: 274.0962.

### Example 39

### N-(1,1-difluoro-2,3-dihydro-3-(4-methylpiperazin-1-yl)-1H-indan-6-yl)-3-(2-(imidazo[1,2-b]pyridazin-3-yl)ethynyl)-4-fluorobenzamide

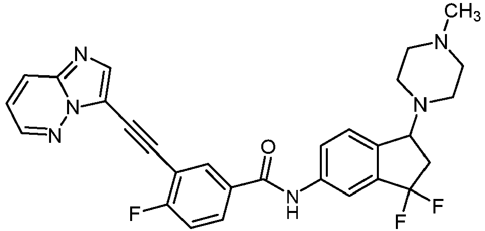

### Step 1. N-(1,1-difluoro-2,3-dihydro-3-(4-methylpiperazin-1-yl)-1H-indan-6-yl)-3-iodo-4-fluorobenzamide

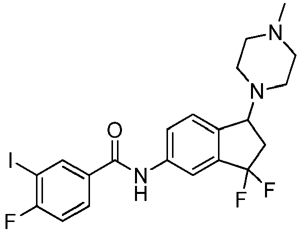

The title compound was synthesized from 4-fluoro-3-iodobenzoic acid and 1-(1,1-difluoro-2,3-dihydro-6-amino-1H-indan-3-yl)-4-methylpiperazine (as prepared in Example 36 step 6) in a manner similar to that described in Example 36 step 7. The product was obtained as a pale yellow solid. LCMS: m/z [M+H]⁺ 516.1312.

### Step 2. N-(1,1-difluoro-2,3-dihydro-3-(4-methylpiperazin-1-yl)-1H-indan-6-yl)-3-(2-(imidazo[1,2-b]pyridazin-3-yl)ethynyl)-4-fluorobenzamide

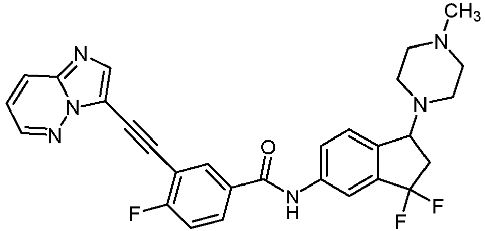

The title compound was synthesized from 3-Ethynylimidazo[1,2-b]pyridazine and 4-fluoro-N-(1,1-difluoro-2,3-dihydro-3-(4-methylpiperazin-1-yl)-1H-indan-6-yl)-3-iodobenzamide in a manner similar to that described in **Example 1.** The product was obtained as a pale yellow solid. Mp: 118-121°C. ¹H NMR (400 MHz, CDCl₃) δ: 2.38 (3 H, s), 2.53-2.57 (2 H, m), 2.59-2.66 (8 H, m), 4.49-4.51 (1 H, d, *J* = 6.4 Hz), 7.12-7.16 (1 H, m), 7.22-7.24 (1 H, d, *J* = 8.8 Hz), 7.86-7.89 (2 H, m), 7.94-7.96(2 H, m), 8.11-8.12(1 H, d, *J* = 4 Hz), 8.13 (1 H, s), 8.48-8.50 (1 H, dd, *J* = 4.4 and 1.6 Hz), 8.56 (1 H, s). HRMS (ESI-TOF⁺): m/z [M+H]⁺¹ calcd for C₂₉H₂₆F₃N₆O: 531.2115; found: 531.2120.

### Example 40

### Separation of the optical isomers of Compound 36 obtained in Example 36 and determination of the absolute configurations thereof

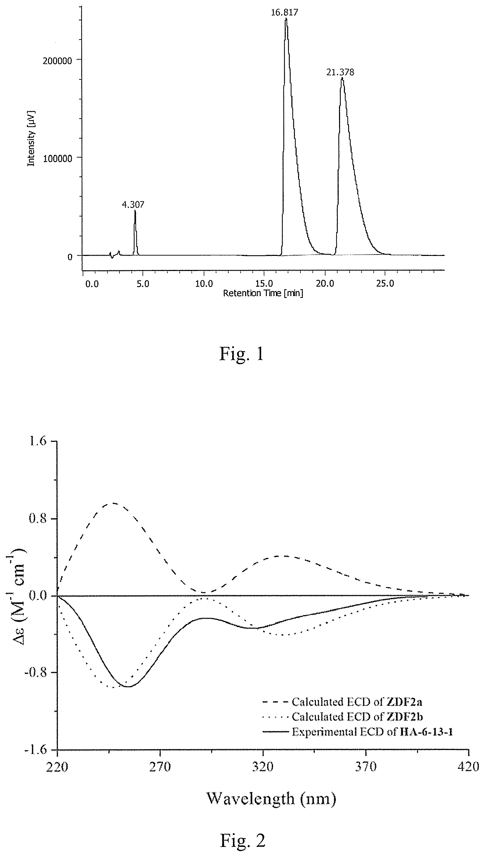

The racemate product (5.0 g) obtained in Example 36 was subjected to a separation with SFC (Supercritical Fluid Chromatography) method by the chiral column (CHIRALCEL AS-H, 0.46 cm I.D. × 25 cm L) (see Fig. 1). The products were respectively collected at Peak 1 with retention time of 16.817 min (hereinafter also refered to as HAP-71-P1, 2.8257 g, [α]²⁰_{,D} = +15.96° (c = 1.0, CHCl₃)) and Peak 2 with retention time of 21.378 min (hereinafter also refered to as HAP-71-P2, 2.5913 g, [α]²⁰_{,D} = -16.19° (c = 1.0, CHCl₃)).

The absolute configuration determination of the above separated products was performed according to the method described as follows.

### Step 1. Preparation of (S)-1-(1,1-Difluoro-2,3-dihydro-6-nitro-1H-indan-3-yl)-4-methylpiperazine (hereinafter refered to as HA-6-13-1)

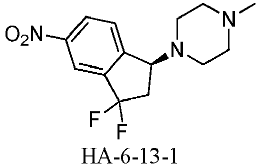

To a racemic mixture of 1-(1,1-Difluoro-2,3-dihydro-6-nitro-1H-indan-3-yl)-4-methylpiperazine (1 g, 4 mmol) in 10 mL methanol was added D-camphor-10-sulfonic acid (1.84 g, 8 mmol) at room temperature, then 5 mL isopropanol was added. The mixtrue was refluxed and cooled to room temperature. The solution was stayed at -20°C for 5 days, the result solid was collected by filtration. The solid was recrystalized in a solution of methanol:isopropanol=1:2 for four times to give 220 mg colorless solid. The solid was dissovled in 10 mL CH₂Cl₂ and 5 mL 1M NaOH solution was added therein, the mixture was stirred for 0.5 h, and the aqueous layer was seperated, which was further extracted with CH₂Cl₂ (2×10mL). The organic layers were combined and washed with brine, dried over Na₂SO₄, evaporated to give product 55 mg (hereinafter referred to as HA-6-13-1) . [α]²⁰_{,D} = +18.51° (c = 1.1, CHCl₃).

The compound 1-(1,1-Difluoro-2,3-dihydro-6-nitro-1H-indan-3-yl)-4-methylpiperazine theoretically theoretically has two optical isomers, hereinafter respectively refered to as ZDF2a and ZDF2b, as shown in below table.

| | |
|---|---|
| ZDF2a | 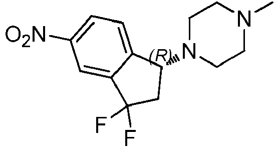 |
| ZDF2b | 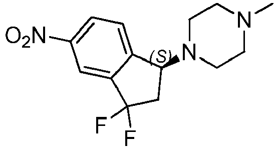 |

The absolute configuration of the above prepared Compound HA-6-13-1 was determined by comparison of the experimental ECD spectrum of Compound HA-6-13-1 and the corresponding calculated ECD spectra of the optical isomers ZDF2a and ZDF2b. The MMFF94 (Merck Molecular Force Field 94) conformational search followed by reoptimization using density functional theory with B3LYP/6-31G(d) (the Becke 3-Lee-Yang-Parr exchange-correlation functional) method afforded eleven lowest energy conformers. The overall ECD spectra of enantiomers ZDF2a and ZDF2b were then generated by Boltzmann weighting of the ECD spectra of eleven conformers. The ECD spectrum recorded for HA-6-13-1 matches the calculated ECD curve of ZDF2b, but is opposite to that of ZDF2a (see Fig. 2). Therefore, Compound HA-6-13-1 was deduced to have the S absolute configuration.

### Step 2. Preparation of (S)-N-(1,1-difluoro-2,3-dihydro-3-(4-methylpiperazin-1-yl)-1H-indan-6-yl)-3-iodo-4-methylbenzamide

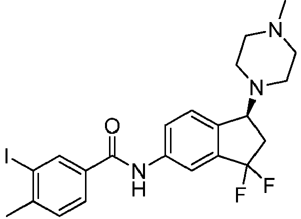

The title compound was synthesized from 3-iodo-4-methylbenzoic acid and (*S*)-1-(1,1-difluoro-2,3-dihydro-6-amino-1H-indan-3-yl)-4-methylpiperazine (prepared from HA-6-13-1) in a manner similar to that described in **Example 36 step 7.** The product was obtained as a pale yellow solid. LCMS: m/z [M+H]⁺ 512.1088.

### Step 3. Preparation of (S)-N-(1,1-difluoro-2,3-dihydro-3-(4-methylpiperazin-1-yl)-1H-indan-6-yl)-3-(2-(imidazo[1,2-b]pyridazin-3-yl)ethynyl)-4-methylbenzamide (corresponding to Compound 36-(S))

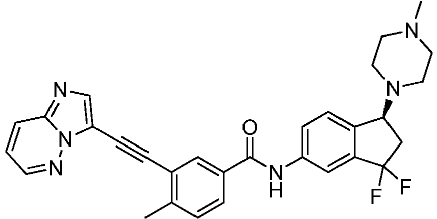

The title compound was synthesized from 3-Ethynylimidazo[1,2-b]pyridazine and (*S*)-N-(1,1-difluoro-2,3-dihydro-3-(4-methylpiperazin-1-yl)-1H-indan-6-yl)-3-iodo-4-methyl-benzamide in a manner similar to that described in **Example 1.** The product was obtained as a pale yellow solid. HRMS (ESI-TOF⁺): m/z [M+2H]⁺² calcd for C₃₀H₃₀F₂N₆O: 264.1219; found: 264.1216.

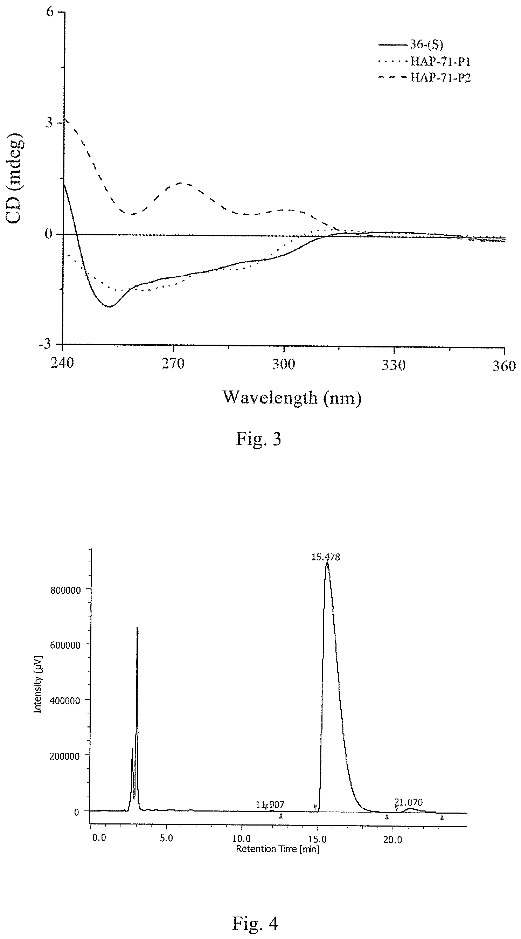

The ECD spectrum recorded for the above prepared compound matches the curve of the above separated product HAP-71-p1 (collected at peak 1), but is opposite to that of product HAP-71-p2 (collected at peak 2) (see Fig. 3). Therefore, HAP-71-p1 was deduced to have the S absolute configuration.

For further confirmation that the product HAP-71-p1 (collected at peak 1) has the same absolute configuration as the above prepared (*S*)-N-(1,1-difluoro-2,3-dihydro-3-(4-methylpiperazin-1-yl)-1H-indan-6-yl)-3-(2-(imidazo[1,2-b]pyridazin-3-yl)ethynyl)-4-methylbenzamide (Compound 36-(S)), the prepared Compound 36-(S) was subjected to passing through the chiral column (CHIRALCEL AS-H, 0.46 cm I.D. × 25 cm L), and the result of the chiral column analysis was consistent with ECD spectrum (please see Fig. 4).

So the absolute configuration of HAP-71-P2 was R, as shown in below.

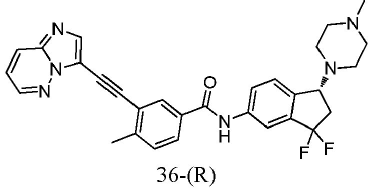

### Example 41

### Separation of the optical isomers of Compound 37 obtained in Example 37

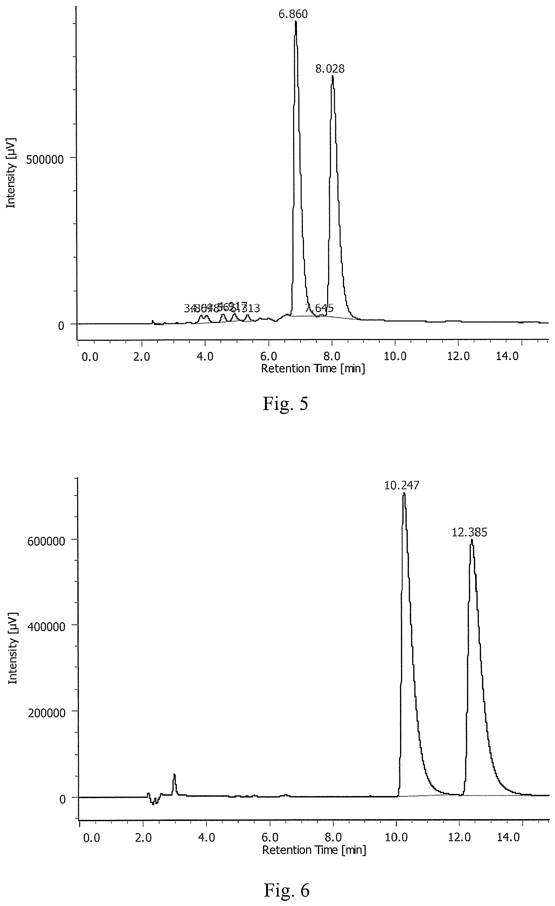

The racemate product (0.1631 g) obtained in Example 37 was subjected to a separation with SFC (Supercritical Fluid Chromatography) method by the chiral column (CHIRALCEL OJ-H, 0.46 cm I.D. × 25 cm L). The products collected at Peak 1 (0.0566 g, [α]²⁰_{,D} = +14.79° (c = 1.0, CHCl₃)) and Peak 2 (0.0694 g, [α]²⁰_{,D} = -12.69° (c = 1.0, CHCl₃))(see Fig. 5) were proved to be isomers having the absolute configurations of S and R, respectively (the determination was based on ECD study in a manner similar to that described in Example 40).

### Example 42

### Separation of the optical isomers of Compound 38 obtained in Example 38

The racemate product (0.2882 g) obtained in Example 36 was subjected to a separation with SFC (Supercritical Fluid Chromatography) method by the chiral column (CHIRALCEL OJ-H, 0.46 cm I.D. × 25 cm L). The products collected at Peak 1 (0.1228 g, [α]²⁰_{,D} = +14.53° (c = 1.0, CHCl₃)) and Peak 2 (0.1472 g, [α]²⁰_{,D} = -14.21° (c = 1.0, CHCl₃)) (see Fig. 6) were proved to be isomers having the absolute configurations of S and R, respectively (the determination was based on ECD study in a manner similar to that described in example 40).

### Example 43

### Separation of the optical isomers of Compound 39 obtained in Example 39

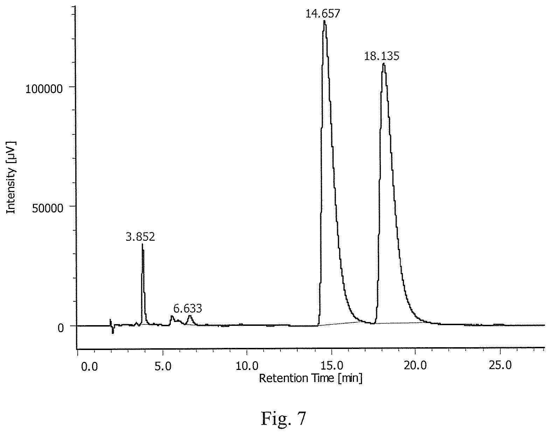

The racemate product (100 mg) obtained in Example 40 was subjected to a separation with SFC (Supercritical Fluid Chromatography)method by the chiral column (CHIRALCEL AS-H, 0.46 cm I.D. × 25 cm L). The products collected at Peak 1 (40 mg, [α]²⁰_{,D} = +15.26° (c = 1.0, CHCl₃)) and Peak 2 (43 mg, [α]²⁰_{,D} = -15.53° (c = 1.0, CHCl₃)) (see Fig. 7) were proved to be isomers having the absolute configurations of S and R, respectively (the determination was based on ECD study in a manner similar to that described in example 40).

### BIOLOGICAL ASSAY

The activity of the compounds of the present, compounds 36(S) to 39(S), can be evaluated using the following assays.

### ASSESSMENT OF CELL DEATH ASSAY

Method of cell death assay:

### Experiment 1:

K562 cells were seeded in 24-well plates with 1.5x10⁵ cells/ml/well, then cells were treated AP-245534 and test compounds dosed as 100nM 48 hours. Then cells were harvested at 4000rpm x 4 min, and resuspended with 150 ul PBS, live cells were counted with TC10 via trypan blue methods.

### Results:

**Table 1 Compound activities in killing K562 cells at 100 nM (% live cells vs control)**

| **Compound** | **% live cells** | **Compound** | **% live cells** |
|---|---|---|---|
| **3** | 11.3 | **28** | 12.7 |
| **5** | 10.8 | **29** | 5.87 |
| **6** | 10.1 | **30** | 12.6 |
| **10** | 11 | **31** | 14.4 |
| **12** | 10.1 | **32** | 9.57 |
| **13** | 8.9 | **33** | 7.97 |
| **14** | 8.37 | **34** | 12 |
| **15** | 17 | **35** | 11.3 |
| **16** | 8.46 | **36** | 9.48 |
| **17** | 18.3 | **37** | 4.32 |
| **23** | 8.6 | **negative control** | 100 |
| **24** | 16.4 | **AP-24534*** | 10.1 |
| **26** | 18.3 | | |

| | | | |
|---|---|---|---|
| * The structure of AP-24534 is below (J. Med. Chem., 2010, 53, 4701-4719), | | | |

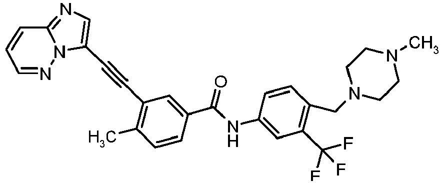

### Experiment 2:

K562 cells (2x10⁵ cells/ml) were seeded in 24-well cell culture plate and treated with DMSO or agents. After treatment in the presence of DMSO or agents for 48 h, cells were collected by centrifuging at 500g for 5 minutes, and then cells were resuspended in appropriate volume of PBS. 10 uL of cell suspension was mixed with 10uL of trypan blue solution and live cells were counted by TC10 (Bio-Rad, Richmond, CA).

### Results:

**Table 2 Some representative compounds IC₅₀ values**

| **Compound** | **IC₅₀ (nM)** |
|---|---|
| **3** | 3 |
| **12** | 4 |
| **13** | 11 |
| **23** | 16 |
| **30** | 3.6 |
| **36** | 0.87 |
| **37** | 3.4 |

As seen in the **Experiment 2** above, compounds of the instant invention that had an IC₅₀ value greater than 0 nM but less than 20 nM were given an "A" rating. Compounds of the instant invention that had an IC₅₀ value equal to, or greater than, 20 nM, but less than 1000 nM, were given a "B" rating.

### Experiment 3:

### Methods:

This study evaluated the effect of the pure optical isomers on human leukemia cell viability in vitro. Ponatinib was used as the positive control. Two BCR-ABL-positive human leukemia cell lines, K562 and KU812, were used in this study. Besides, Ba/F3 cell transfectants expressing native or T315I BCR-ABL, whose viability is dependent on ectopically expressed BCR-ABL, were also used in this work. Ba/F3 cells expressing BCR-ABL^{T315I} were used for testing the efficiency of tested compounds on point mutation BCR-ABL^{T315I}, which cause drug resistance to Imatinib (Gleevec). U937 and JURKAT, two BCR-ABL-negative human leukemia cell lines, were employed to test the selectivity of tested compounds.

Cells were seeded in 24-well plates, and treated with drugs at corresponding concentrations. Cell viability was measured using a MTT (3-(4,5-dimethylthiazol-2-yl)-2,5-diphenyltetrazolium bromide)-based colorimetric assay with a 96-well plate reader. IC50 values were calculated using the mean of two independent experiments.

**Results:** Effect of BCR-ABL inhibitors on cell viability of BCR-ABL-positive and -negative human leukemia cell lines and a panel of Ba/F3 cell transfectants (IC50 as Mean±S.E.M, nM).

The results showed that no matter the racemic compounds or the pure isomers with absolute configuration R or S show effect on leukemia cells or BaF3 cells expressing native BCR-ABL. Particularly, compounds 36-(S) and 38-(S) perform similarly on leukemia cells or BaF3 cells expressing native BCR-ABL, compared to reference compound Ponatinib. The compounds 36-(S) and 38-(S) work better on BaF3 cells expressing BCR-ABL^{T315I} than reference compound Ponatinib, implying that the two compounds could be more potent in treating the patients developing drug resistance to imatinib, the first generation tyrosine kinase inhibitor. Besides, 36-(S) and 38-(S) are less potent on BCR-ABL-negative human leukemia cell lines U937 and JURKAT. It means that 36-(S) and 38-(S) may have better selectivity to BCR-ABL and less side-effect in clinical use.

**Table 3 Effect of BCR-ABL inhibitors on cell viability of BCR-ABL-positive and -negative human leukemia cell lines and Ba/F3 cell transfected cells (IC50 as Mean±S.E.M, nM)**

| **Compound** | **Ba/F3 cells** | | **CML leukemia cells** | | **Non-CML leukemia cells** | |
|---|---|---|---|---|---|---|
| | Native BCR-ABL | T315I BCR-ABL | K562 | KU812 | U937 | JURKAT |
| **Ponatinib** | 0.9±0.1 | 9.6±1.7 | 0.3±0.1 | 0.1±0.1 | 245.8±12.5 | 295.0±5.0 |
| Reference compound **36** | 2.7±0.2 | 11.0±0.2 | 0.5±0.1 | 0.2±0.1 | 607.8±42.2 | 672.4±77.6 |
| **36-(S)** | 2.1±0.2 | 4.7±1.3 | 0.4±0.1 | 0.1±0.1 | 867.6±88.7 | 850.7±25.7 |
| Reference compound **36-(R)** | 31.2±3.8 | >100 | 3.6±0.1 | 2.7±0.1 | >1000 | >1000 |
| Reference compound **37** | 9.3±2.5 | 40.0±9.6 | 1.0±0.2 | 0.4±0.1 | 190.8±3.3 | 292.0±8.0 |
| **37-(S)** | 3.5±0.9 | 12.4±0.4 | 0.6±0.1 | 0.2±0.1 | 165.0±15.0 | 280.0±20.0 |
| Reference compound **37-(R)** | 62.5±6.9 | >100 | 10.4±1.5 | 5.2±0.2 | 465.3±9.7 | 399.9±64.9 |
| Reference compound **38** | 4.9±2.1 | 10.1±0.7 | 0.8±0.1 | 0.4±0.1 | >1000 | >1000 |
| **38-(S)** | 2.2±0.3 | 3.8±0.2 | 0.6±0.1 | 0.2±0.1 | >1000 | >1000 |
| Reference compound **38-(R)** | 78.2±2.3 | >100 | 24.9±9.9 | 11.9±0.3 | >1000 | >1000 |
| Reference compound **39** | 3.6±0.7 | 13.0±0.9 | 0.8±0.2 | 0.5±0.3 | 809.8±39.2 | >1000 |
| **39 (S)** | 2.3±0.5 | 6.5±1.2 | 0.3±0.2 | 0.2±0.1 | >1000 | >1000 |
| Reference compound **39 (R)** | 47.6±4.5 | >100 | 6.4±1.3 | 3.6±0.8 | >1000 | >1000 |

### Experiment 4:

### Methods

The murine pro-B cell line Ba/F3 harboring the T315I mutant isoform of BCR-ABL was subcutaneously injected into NOD SCID mice (NOD.CB17-Prkdc^{scid}/J). Mice were dosed orally with tested compounds for 14 days: Ponatinib used as the positive drug at doses of 10 or 20 mg/kg/day; Compound 36-(S) at doses of 10 or 20 mg/kg/day; Compound 38-(S) at dose of 5 mg/kg/day. The tumor volumes in two dimensions and the body weights of the mice were measured three times a week (volume= L × W² × 0.5).

### Results:

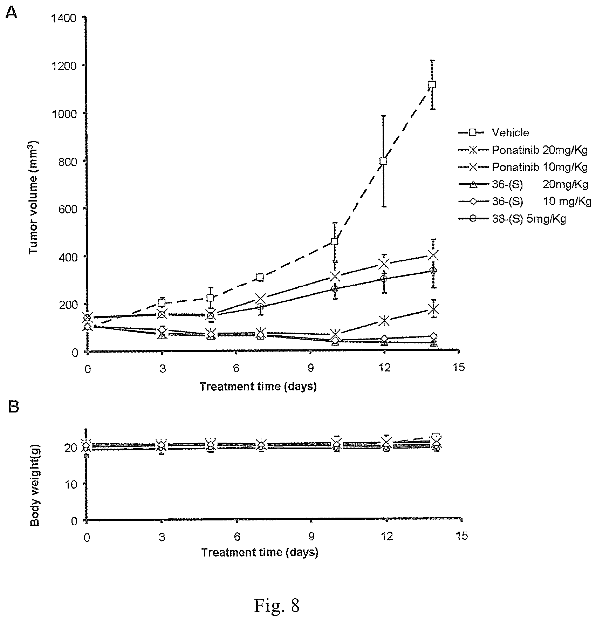

The TGI (Tumor growth inhibition) for the drugs are: Ponatinib 20 mg/kg/day, 94%; Ponatinib 10 mg/kg/day, 75%; Compound 36-(S) 20 mg/kg/day, 108%; Compound 36-(S) 10 mg/kg/day, 105%; Compound 38-(S) 5 mg/kg/day, 81%. The TGI greater than 100% means that tumors treated with drugs over 14 days are smaller than their original sizes. The mouse body weight didn't change obviously over the treatment period at the described doses. The results are also shown in Fig. 8, A and B.

From the animal experiment results, we can conclude that 36-(S) at the dose of 10 mg/kg/day works better than Ponatinib at the dose of 20 mg/kg/day. 38-(S) at the dose of 5 mg/kg/day works better than Ponatinib at the dose of 10 mg/kg/day. Overall, 36-(S) and 38-(S) are more potent than Ponatinib in BaF3/BCR-ABL^{T315I} mouse xenograft model.

The above results suggest the compounds of the present invention, compounds 36(S) to 39(S), compounds according to Formula VIII with absolute configuration S have good effects on inhibiting protein kinase, such as Bcr-Abl, c-Kit, c-Src, FGFR1, FLT3, LYN, PDGFR, particularly Bcr-Able (T315I); inhibiting or curing cancers, such as Chronic Myelogenous Leukemia (CML), Acute Lymphocytic Leukemia (ALL), Non-Small Cell Lung Cancer (NSCLC), Gastrointestinal stromal tumor (GIST), Acute Myelogenous Leukemia (AML), particularly Chronic Myelogenous Leukemia (CML); and inhibiting and/or treating inflammation such as Asthma, Rheumatoid Arthritis.

## Claims

1. A compound with absolute configuration S, or a pharmaceutically acceptable salt, hydrate, or solvate thereof, wherein the compound is selected from the group consisting of:
(S)-N-(1,1-difluoro-2,3-dihydro-3-(4-methylpiperazin-1-yl)-1H-indan-6-yl)-3-(2-(imidazo[1,2-b]pyridazin-3-yl)ethynyl)-4-methylbenzamide;
(S)-3-(2-(2-(Cyclopropylamino)pyrimidin-5-yl)ethynyl)-N-(1,1-difluoro-2,3-dihydro-3-(4-methylpiperazin-1-yl)-1H-indan-6-yl)-4-methylbenzamide;
(S)-N-(1,1-difluoro-2,3-dihydro-3-(4-methylpiperazin-1-yl)-1H-indan-6-yl)-3-(2-(imidazo[1,2-b]pyridazin-3-yl)ethynyl)-4-chlorobenzamide; and
(S)-N-(1,1-difluoro-2,3-dihydro-3-(4-methylpiperazin-1-yl)-1H-indan-6-yl)-3-(2-(imidazo[1,2-b]pyridazin-3-yl)ethynyl)-4-fluorobenzamide.

2. A pharmaceutical composition comprising at least one compound of claim 1 or a salt, hydrate, or solvate thereof, and one or more pharmaceutically acceptable carriers and/or additives.

3. A compound of claim 1, or a salt, hydrate, or solvate thereof for use in the treatment of cancer or inflammation, wherein protein kinase is inhibited.

4. The compound for use according to claim 3, wherein said protein kinase is selected from the group consisting of Bcr-Abl, c-Kit, c-Src, FGFR1, FLT3, LYN and PDGFR.

5. A compound of claim 1, or a salt, hydrate, or solvate thereof for use in the treatment of cancer.

6. The compound for use according to claim 5, wherein said cancer is selected from Chronic Myelogenous Leukemia (CML), Acute Lymphocytic Leukemia (ALL), Non-Small Cell Lung Cancer (NSCLC), Gastrointestinal stromal tumor (GIST) and Acute Myelogenous Leukemia (AML).

7. A compound of claim 1, or a salt, hydrate, or solvate thereof for use in the treatment of inflammation.

8. The compound for use according to claim 7, wherein said inflammation is selected from Asthma and Rheumatoid Arthritis.

## Patentansprüche

1. Eine Verbindung mit der absoluten Konfiguration S, oder ein pharmazeutisch verträgliches Salz, Hydrat oder Solvat davon, wobei die Verbindung ausgewählt ist aus der Gruppe bestehend aus:
(S)-N-(1,1-Difluor-2,3-dihydro-3-(4-methylpiperazin-1-yl)-1H-indan-6-yl)-3-(2-(imidazo[1,2-b]pyridazin-3-yl)ethynyl)-4-methylbenzamid;
(S)-3-(2-(2-(Cyclopropylamino)pyrimidin-5-yl)ethynyl)-N-(1,1-difluor-2,3-dihydro-3-(4-methylpiperazin-1-yl)-1H-indan-6-yl)-4-methylbenzamid;
(S)-N-(1,1-Difluor-2,3-dihydro-3-(4-methylpiperazin-1-yl)-1H-indan-6-yl)-3-(2-(imidazo[1,2-b]pyridazin-3-yl)ethynyl)-4-chlorobenzamid und
(S)-N-(1,1-Difluor-2,3-dihydro-3-(4-methylpiperazin-1-yl)-1H-indan-6-yl)-3-(2-(imidazo[1,2-b]pyridazin-3-yl)ethynyl)-4-fluorobenzamid.

2. Eine pharmazeutische Zusammensetzung, die mindestens eine Verbindung nach Anspruch 1 oder ein Salz, Hydrat oder Solvat davon, und einen oder mehrere pharmazeutisch verträgliche Träger und/oder Zusätze umfasst.

3. Eine Verbindung nach Anspruch 1 oder ein Salz, Hydrat oder Solvat davon zur Verwendung bei der Behandlung von Krebs oder einer Entzündung, wobei eine Proteinkinase gehemmt wird.

4. Die Verbindung zur Verwendung nach Anspruch 3, wobei die Proteinkinase ausgewählt ist aus der Gruppe bestehend aus Bcr-Abl, c-Kit, c-Src, FGFR1, FLT3, LYN und PDGFR.

5. Eine Verbindung nach Anspruch 1 oder ein Salz, Hydrat oder Solvat davon zur Verwendung bei der Behandlung von Krebs.

6. Die Verbindung zur Verwendung nach Anspruch 5, wobei der Krebs ausgewählt ist aus chronischer myeloischer Leukämie (CML), akuter lymphozytischer Leukämie (ALL), nicht kleinzelligem Lungenkrebs (NSCLC), gastrointestinalem Stromatumor (GIST) und akuter myeloischer Leukämie (AML).

7. Eine Verbindung nach Anspruch 1 oder ein Salz, Hydrat oder Solvat davon zur Verwendung bei der Behandlung einer Entzündung.

8. Die Verbindung zur Verwendung nach Anspruch 7, wobei die Entzündung ausgewählt ist aus Asthma und rheumatoider Arthritis.

## Revendications

1. Composé présentant une configuration absolue S, ou sel, hydrate ou solvate pharmaceutiquement acceptable de celui-ci, le composé étant choisi dans le groupe constitué par :
le (S)-N-(1,1-difluoro-2,3-dihydro-3-(4-méthylpipérazin-1-yl)-1H-indan-6-yl)-3-(2-imidazo[1,2-b]pyridazin-3-yl)éthynyl)-4-méthylbenzamide ;
le (S)-3-(2-(2-(cyclopropylamino)pyrimidin-5-yl)éthynyl)-N-(1,1-difluoro-2,3-dihydro-3-(4-méthylpipérazin-1-yl)-1H-indan-6-yl)-4-méthylbenzamide ;
le (S)-N-(1,1-difluoro-2,3-dihydro-3-(4-méthylpipérazin-1-yl)-1H-indan-6-yl)-3-(2-imidazo[1,2-b]pyridazin-3-yl)éthynyl)-4-chlorobenzamide ; et
le (S)-N-(1,1-difluoro-2,3-dihydro-3-(4-méthylpipérazin-1-yl)-1H-indan-6-yl)-3-(2-imidazo[1,2-b]pyridazin-3-yl)éthynyl)-4-fluorobenzamide.

2. Composition pharmaceutique comprenant au moins un composé selon la revendication 1 ou un sel, hydrate ou solvate de celui-ci, et un ou plusieurs véhicules et/ou additifs pharmaceutiquement acceptables.

3. Composé selon la revendication 1, ou sel, hydrate ou solvate de celui-ci destiné à être utilisé dans le traitement d'un cancer ou d'une inflammation, dans lequel une protéine kinase est inhibée.

4. Composé destiné à être utilisé selon la revendication 3, ladite protéine kinase étant choisie dans le groupe constitué par Bcr-Abl, c-Kit, c-Src, FGFR1, FLT3, LYN et PDGFR.

5. Composé selon la revendication 1, ou sel, hydrate ou solvate de celui-ci destiné à être utilisé dans le traitement d'un cancer.

6. Composé destiné à être utilisé selon la revendication 5, ledit cancer étant choisi parmi une leucémie myéloïde chronique (LMC), une leucémie lymphoïde aigüe (LLA), un cancer du poumon non à petites cellules (CPNPC), une tumeur stromale gastro-intestinale (TSGI) et une leucémie myléoïde aiguë (LMA).

7. Composé selon la revendication 1, ou sel, hydrate ou solvate de celui-ci destiné à être utilisé dans le traitement d'une inflammation.

8. Composé destiné à être utilisé selon la revendication 7, ladite inflammation étant choisie parmi l'asthme et la polyarthrite rhumatoïde.
